# EUROPEAN PATENT SPECIFICATION

(11) **EP 2 115 148 B1**
(45) Date of publication and mention of the grant of the patent: **14.09.2011**
(21) Application number: 08708767.2
(22) Date of filing: 07.02.2008
(51) Int. Cl.: C12N 15/82

(54) **COMPOSITIONS AND METHODS USING RNA INTERFERENCE OF CDPK-LIKE FOR CONTROL OF NEMATODES**
ZUSAMMENSETZUNGEN UND VERFAHREN, DIE RNA-INTERFERENZEN VON CDPK-LIKE ZUR KONTROLLE VON NEMATODEN VERWENDEN
COMPOSITIONS ET PROCÉDÉS FAISANT APPEL À L'INTERFÉRENCE DE L'ARN DE TYPE CDPK DANS LA LUTTE CONTRE LES NÉMATODES

(30) Priority: 09.02.2007 US 900466 P
(43) Date of publication of application: 11.11.2009
(73) Proprietor: BASF Plant Science GmbH, 67056 Ludwigshafen (DE)
(72) Inventor: TALTON, Lawrence Winfield, Sanford, NC 27330 (US); REN, Peifeng, Cary, NC 27519 (US); ASCENZI, Robert A., Cary, NC 27511 (US)
(74) Representative: Popp, Andreas
(86) International application number: PCT/EP2008/051482
(87) International publication number: WO 2008/095970

(56) References cited:
- WO-A-2004/005485
- WO-A-2006/020821
- US-A1- 2004 031 072
- IVASHUTA S. ET AL.: "RNA interference identifies a calcium-dependent protein kinase involved in medicago truncatula root development" THE PLANT CELL, vol. 17, November 2005 (2005-11), pages 2911-2921, XP002478172
- GARGANTINI P. ET AL.: "A CDPK isoform participates in the regulation of nodule number in Medicago truncatula" THE PLANT JOURNAL, vol. 48, 2006, pages 843-856, XP002478173
- LUDWIG A A ET AL: "CDPK-mediated signalling pathways: Specificity and cross-talk" JOURNAL OF EXPERIMENTAL BOTANY, OXFORD UNIVERSITY PRESS, GB, vol. 55, no. 395, January 2004 (2004-01), pages 181-188, XP002343024 ISSN: 0022-0957
- GHEYSEN ET AL: "RNAi from plants to nematodes" TRENDS IN BIOTECHNOLOGY, ELSEVIER PUBLICATIONS, CAMBRIDGE, GB, vol. 25, no. 3, 24 January 2007 (2007-01-24), pages 89-92, XP005896599 ISSN: 0167-7799

### SEQUENCE LISTING

<110> BASF Plant Science GmbH
   Ascenzi, Robert
<120> Compositions and Methods Using RNA Interference Of CDPK-Like For Control of Nematodes
<130> PF 58856-PCT
<160> 32
<170> PatentIn version 3.4
<210> 1
   <211> 1045
   <212> DNA
   <213> Glycine max
<220>
   <221> 3'UTR
   <222> (663)..(1045)
   <223> Predicted poly-A tail nucleotide residues 1031 to 1045
<400> 1
   aagatcctcg tgcgagatat actgctgctc aggctctttc acatccatgg gttagagaag     60
   gaggagaggc attagagatt cctattgata tatctgtcct gaacaacatg cgacagtttg    120
   tgaaatatag tcggttgaaa caatttgcac taagggcatt ggctagcaca cttaatgaag    180
   gagagttgtc tgatctaaaa gatcagtttg atgcaataga tgtggacaaa aatggttcta    240
   ttagtcttga ggagatgaga caggctcttg ctaaagatca accttggaag ttgaaagaat    300
   cacgtgtgct agagatattg caagcgatag acagcaacac agatgggcta gtggatttca    360
   ccgagtttgt ggcagctact ttacatgtac atcaattgga ggaacatgat tctgacaagt    420
   ggcagcaacg gtcacaggct gcttttgaga aatttgactt ggataaggat ggctatatta    480
   ctccagatga acttagaatg catacgggtt tgagaggctc cattgatcca ttgcttgagg    540
   aagccgatat tgataaagat gggaaaatca gcttaccaga atttcgtaga cttctaagaa    600
   ctgcaagcat gggttctcga acagtaatga gcccaagtca ccgtcatcat cgaaagattt    660
   agattagata tgtttcggag acagggataa ttcaggagat gagagtgatg gatttccttt    720
   taagatgatg atctcttatg cagattgtgc tgaagccctc cttgtgatat ctattctgct    780
   gcacattttg cactctgacc atcattatat ggaactctga aactagagtt aatattgact    840
   atggcatacc acacaggatt gtgtgttaag cttagtctag acgaccatgt caagaggaat    900
   ttaaatagtg tgtggattga cttgaggtat cagagtttca tcggtgtaca tcattggctt    960
   gtttgtgcca tacatgtaat gtgtaatact gtaactataa ataaaaaact tcttactccc   1020
   cttcccctgc aaaaaaaaaa aaaaa                                          1045
<210> 2
   <211> 320
   <212> DNA
   <213> Glycine max
<400> 2
   gttagagaag gaggagaggc attagagatt cctattgata tatctgtcct gaacaacatg     60
   cgacagtttg tgaaatatag tcggttgaaa caatttgcac taagggcatt ggctagcaca    120
   cttaatgaag gagagttgtc tgatctaaaa gatcagtttg atgcaataga tgtggacaaa    180
   aatggttcta ttagtcttga ggagatgaga caggctcttg ctaaagatca accttggaag    240
   ttgaaagaat cacgtgtgct agagatattg caagcgatag acagcaacac agatgggcta    300
   gtggatttca ccgagtttgt                                                320
<210> 3
   <211> 219
   <212> PRT
   <213> Glycine max
<400> 3
   Asp Pro Arg Ala Arg Tyr Thr Ala Ala Gln Ala Leu Ser His Pro Trp
   1               5                   10                  15
   Val Arg Glu Gly Gly Glu Ala Leu Glu Ile Pro Ile Asp Ile Ser Val
               20                  25                  30
   Leu Asn Asn Met Arg Gln Phe Val Lys Tyr Ser Arg Leu Lys Gln Phe
           35                  40                  45
   Ala Leu Arg Ala Leu Ala Ser Thr Leu Asn Glu Gly Glu Leu Ser Asp
       50                  55                  60
   Leu Lys Asp Gln Phe Asp Ala Ile Asp Val Asp Lys Asn Gly Ser Ile
   65                  70                  75                  80
   Ser Leu Glu Glu Met Arg Gln Ala Leu Ala Lys Asp Gln Pro Trp Lys
                   85                  90                  95
   Leu Lys Glu Ser Arg Val Leu Glu Ile Leu Gln Ala Ile Asp Ser Asn
               100                 105                 110
   Thr Asp Gly Leu Val Asp Phe Thr Glu Phe Val Ala Ala Thr Leu His
           115                 120                 125
   Val His Gln Leu Glu Glu His Asp Ser Asp Lys Trp Gln Gln Arg Ser
       130                 135                 140
   Gln Ala Ala Phe Glu Lys Phe Asp Leu Asp Lys Asp Gly Tyr Ile Thr
   145                 150                 155                 160
   Pro Asp Glu Leu Arg Met His Thr Gly Leu Arg Gly Ser Ile Asp Pro
                   165                 170                 175
   Leu Leu Glu Glu Ala Asp Ile Asp Lys Asp Gly Lys Ile Ser Leu Pro
               180                 185                 190
   Glu Phe Arg Arg Leu Leu Arg Thr Ala Ser Met Gly Ser Arg Thr Val
           195                 200                 205
   Met Ser Pro Ser His Arg His His Arg Lys Ile
       210                 215
<210> 4
   <211> 2211
   <212> DNA
   <213> Medicago truncatula
<220>
   <221> 5'UTR
   <222> (1)..(146)
<220>
   <221> 3'UTR
   <222> (1830)..(2211)
<400> 4
   ttctccttct tcccttcttc gtcgttcctg ccacattcct tccttcttct caatctcaat      60
   catctcacac gctttccaat ttctaaatca atgatcattt ttttcaaatt ttcttaatta     120
   tcatttttca aacaaaacaa aacatcatgg gtttatgttt ttcttctaca aaggttgtta     180
   gtggttctaa cagcaacacc acaaacaatg ataaccgtaa acggaatcag tcaacaacca     240
   cggataccac cgtcacggta acaacagcaa caacggcggc gcagaaacaa acggcacaga     300
   gacgtaaagg agggtcaaat gaaacagccc agaagaagaa tcatcatcaa catcataggt     360
   taaaagagaa aacaggttct aaacatgttc cttgtggaaa aagaacggat tttgggtatg     420
   agaaagattt tgataaaaga ttttctcttg ggaaattgtt gggtcatgga caatttggtt     480
   atacttatgt tggtgttgat aaatccaatg gagatcgtgt cgccgttaag cgactcgaga     540
   aagctaagat ggttctccct atagcagttg aggatgtaaa aagagaggtc aagatattga     600
   aagaacttac aggccatgaa aatgtggttc agttttataa tgcttttgac gatgattcat     660
   atgtgtacat agttatggag ttatgcgaag gtggagaact actagaccgg atactaaaca     720
   aaaaggacag ccgttatact gaaaaagatg ccgccgttgt tgtaaggcag atgctgaagg 780
   ttgcagctca gtgtcattta catggtttgg tacatcgcga catgaagcca gagaattttc 840
   ttttcaaatc aaacaaagaa gattcagctt tgaaggccac tgattttggt ttgtctgatt 900
   tcataaaacc tggaaagagg tttcaagata ttgttggaag tgcttactat gtcgcaccag 960
   aagtgttgaa acggaagtca ggccctgaat cagatgtatg gagtattggt gtgattacat 1020
   acatattgct ttgtgggaga cgtccatttt gggacaagac tgaagatggt atcttcaagg 1080
   aggtcttacg aaacaagcct gatttccggc ggaaaccatg gccaaccata agtaatgctg 1140
   caaaagattt tgtgaagaaa ttgttggtaa aagatcctcg ggcaagatta acggctgctc 1200
   aggctctatc acatccatgg gttagagaag gaggagaggc atccgagatt ccaattgata 1260
   tatctgtcct aaacaacatg cggcagtttg tgaagtatag tcgattgaaa caatttgcat 1320
   tgagggcatt ggctagcaca cttaacgaag gtgagttgtc tgatctaaaa gatcagtttg 1380
   atgcaataga tgtggacaaa aatggtgcta ttagtcttga agagatgaga caggctcttg 1440
   ctaaagatct cccgtggaag ttgaaagaat cccgtgtatt ggagatattg caagcgattg 1500
   acagcaacac ggatggatta gtagatttca ccgagtttgt tgcagctact ttacatgtgc 1560
   atcaattgga ggaacatgac tccgacaagt ggcagcaacg gtcacaggct gcttttgaga 1620
   aatttgacat agacaaggat ggctatatta ctccagagga actcagaatg cacactggta 1680
   tgagaggttc catcgatcct ctgctcgagg aagccgatat tgacaaagat ggaaagatca 1740
   gcttaccaga attcaggaga cttttaagaa ctgcaagcat tggttctcga aacgtaacaa 1800
   gcccaactct acgtcatcga aggatctagc ttagatgtgt ttcagaggcg gagatgattc 1860
   aaatgagagt gttggagttc ccttttaaga tgatctcctg tgcaggttgt gttgaagcca 1920
   ttgtgatata tgctctgcag attttgcaat ctcttgccat tccatggaac tctgaaactt 1980
   aaattatatt gaatatggcg tcgtcttgca aaggattgtg ttaaaagctt agtctagagg 2040
   actgtatcaa gaagtgtttc atagagtgta tagaccctgc ttggattggc ttcaggtatc 2100
   aaagttgaga gtttccatca gtgtacatca tttttaagtg tcagggtttt ctggatttac 2160
   ttgtgccata acttgtaatg tgtaactata aataaaaatc ttctattcta c 2211
<210> 5
   <211> 740
   <212> DNA
   <213> Unknown
<220>
   <223> Synthesized hairpin targeting sequence from 49806575
<400> 5
   acaaactcgg tgaaatccac tagcccatct gtgttgctgt ctatcgcttg caatatctct 60
   agcacacgtg attctttcaa cttccaaggt tgatctttag caagagcctg tctcatctcc 120
   tcaagactaa tagaaccatt tttgtccaca tctattgcat caaactgatc ttttagatca 180
   gacaactctc cttcattaag tgtgctagcc aatgccctta gtgcaaattg tttcaaccga 240
   ctatatttca caaactgtcg catgttgttc aggacagata tatcaatagg aatctctaat 300
   gcctctcctc cttctctaac cgtcgtgaaa gctctctgga agaagaagat tctcatcagg 360
   atccccgaga aactcggata agagactcac agagtagcca tgctcggtca caacgctaca 420
   gttagagaag gaggagaggc attagagatt cctattgata tatctgtcct gaacaacatg 480
   cgacagtttg tgaaatatag tcggttgaaa caatttgcac taagggcatt ggctagcaca 540
   cttaatgaag gagagttgtc tgatctaaaa gatcagtttg atgcaataga tgtggacaaa 600
   aatggttcta ttagtcttga ggagatgaga caggctcttg ctaaagatca accttggaag 660
   ttgaaagaat cacgtgtgct agagatattg caagcgatag acagcaacac agatgggcta 720
   gtggatttca ccgagtttgt 740

<210> 6
   <211> 1999
   <212> DNA
   <213> Arabidopsis thaliana
<400> 6
   gtagtgccct tcatggatac caaaagagaa aatttgattt agtgcataca tataacaata 60
   taacgccgca taataatact gtataaaaca gtcatgtaac gatatgacag cagtaataca 120
   gttccaagag acgttataat cgtatgcaat catatgcttg cgtagatttt ccaacagttt 180
   tgtttcgttg ataggaggaa ctcaacactc tagggtagtg attggtagac actattagca 240
   caaaaaatat taattttact ctgatgttta ccaaaaaagt taccaatcaa atatttaaga 300
   gatcgtactc ttccacggcg actctaaaaa ccaaagatat aggttagact cataactact 360
   ttataaagaa aatgtttaac gataactacc gagatctaat aaataaacct tcattttcaa 420
   gtatattata tttgcttctt ttgtttatat atcaaaccaa gttctggttt ataaaaatat 480
   tagataaaac tcgtctaaat aggtaggtgt aaaataaaat tttaaatttt tatcgataat 540
   atttaaaatt tgaaaagtta ataatgatcc acacattttt tctaatattt aatttagtaa 600
   tttttgtatt aaataaaatt tcaatcatat acattcgatt tttctataca ttttaactat 660
   ctatttctgc ataataaact gtattttcat tttatacgct tcatcttatg gatgatattt 720
   aaattttaaa tagtaattca tacacttttt aatatttaat ttagtatttt cttaaatcca 780
   aattttaatc ttacaattta aatatctact ttaacataat acaaatacaa tttaatttca 840
   ttgtattaaa ttcaaatata atttgattat aataaaatac aatttaattc taaaaagtcc    900
   atcttagatt ttaattttcc tttttagttt tgaaaattaa aaatttaaat ttattagata    960
   tatatgttac tttttcagtt ttcctattta tttaagaaaa aaatattttt taacacatgt   1020
   caacttgtaa acaatagact gaacacgtca ttttatatta tgtttagttt tgaaaattaa   1080
   agttaattaa atatttatat ttcttttttt tagcttttct aattattttt aaaatagtaa   1140
   atatttttaa tacaaatcaa tatctgaaca atagatttga tacataacat aatcctataa   1200
   attattaact tggaaaacga tagtttatat aataaaatta ttttcttaag ttctctaacc   1260
   ataacaatta aactatattt tagcgaagaa aagaagagaa taccgagaga acgcaacttg   1320
   cactaaaagc taccactttg gcaaatcact catttatatt attatatact atcacctcaa   1380
   ttcaatcgaa acctcaaaat aacactaata tatacacaaa gaaacaacag aataacaccg   1440
   aagaatatag gtttaggaaa atccagaatt tgttgagact aaagagatca aattttcgat   1500
   acaaggtttt gctcaatttg tattttcata ataaaattct ttatttcacc atagacttac   1560
   atgattagtt tttcttttaa taaaaaaaaa cacgcgacat gaaaattata ttatctcagt   1620
   gttgtcgaat ttgaatttga attttgagtt aaatactaca catttgttga caacttatta   1680
   aactttacaa gtctgctaca aatattgtca aatatttact aattaatgga ccaaaatcct   1740
   ctaacttgca aatttgtatc tacatcaact taaaaattag gaatatgcga cccaaaaaaa   1800
   aaaaaactag gaataataat aaaaaaatgg aatgatgtgg aggaagctct ttactctttg   1860
   agaggaagtt tataaattga ccacacattt agtctattat catcacatgt attaagactt   1920
   gacaacttgt ctttctcaca ccaaacccct ctcctctgtt tcataacatc tgctctttct   1980
   tttttttcct aagccccta                                                  1999
<210> 7
   <211> 560
   <212> PRT
   <213> Medicago truncatula
<400> 7
   Met Gly Leu Cys Phe Ser Ser Thr Lys Val Val Ser Gly Ser Asn Ser
   1               5                   10                  15
   Asn Thr Thr Asn Asn Asp Asn Arg Lys Arg Asn Gln Ser Thr Thr Thr
               20                  25                  30
   Asp Thr Thr Val Thr Val Thr Thr Ala Thr Thr Ala Ala Gln Lys Gln
           35                  40                  45
   Thr Ala Gln Arg Arg Lys Gly Gly Ser Asn Glu Thr Ala Gln Lys Lys
   50 55 60
   Asn His His Gln His His Arg Leu Lys Glu Lys Thr Gly Ser Lys His
   65 70 75 80
   Val Pro Cys Gly Lys Arg Thr Asp Phe Gly Tyr Glu Lys Asp Phe Asp
   85 90 95
   Lys Arg Phe Ser Leu Gly Lys Leu Leu Gly His Gly Gln Phe Gly Tyr
   100 105 110
   Thr Tyr Val Gly Val Asp Lys Ser Asn Gly Asp Arg Val Ala Val Lys
   115 120 125
   Arg Leu Glu Lys Ala Lys Met Val Leu Pro Ile Ala Val Glu Asp Val
   130 135 140
   Lys Arg Glu Val Lys Ile Leu Lys Glu Leu Thr Gly His Glu Asn Val
   145 150 155 160
   Val Gln Phe Tyr Asn Ala Phe Asp Asp Asp Ser Tyr Val Tyr Ile Val
   165 170 175
   Met Glu Leu Cys Glu Gly Gly Glu Leu Leu Asp Arg Ile Leu Asn Lys
   180 185 190
   Lys Asp Ser Arg Tyr Thr Glu Lys Asp Ala Ala Val Val Val Arg Gln
   195 200 205
   Met Leu Lys Val Ala Ala Gln Cys His Leu His Gly Leu Val His Arg
   210 215 220
   Asp Met Lys Pro Glu Asn Phe Leu Phe Lys Ser Asn Lys Glu Asp Ser
   225 230 235 240
   Ala Leu Lys Ala Thr Asp Phe Gly Leu Ser Asp Phe Ile Lys Pro Gly
   245 250 255
   Lys Arg Phe Gln Asp Ile Val Gly Ser Ala Tyr Tyr Val Ala Pro Glu
   260 265 270
   Val Leu Lys Arg Lys Ser Gly Pro Glu Ser Asp Val Trp Ser Ile Gly
   275 280 285
   Val Ile Thr Tyr Ile Leu Leu Cys Gly Arg Arg Pro Phe Trp Asp Lys
   290 295 300
   Thr Glu Asp Gly Ile Phe Lys Glu Val Leu Arg Asn Lys Pro Asp Phe
   305                 310                 315                 320
   Arg Arg Lys Pro Trp Pro Thr Ile Ser Asn Ala Ala Lys Asp Phe Val
                   325                 330                 335
   Lys Lys Leu Leu Val Lys Asp Pro Arg Ala Arg Leu Thr Ala Ala Gln
               340                 345                 350
   Ala Leu Ser His Pro Trp Val Arg Glu Gly Gly Glu Ala Ser Glu Ile
           355                 360                 365
   Pro Ile Asp Ile Ser Val Leu Asn Asn Met Arg Gln Phe Val Lys Tyr
       370                 375                 380
   Ser Arg Leu Lys Gln Phe Ala Leu Arg Ala Leu Ala Ser Thr Leu Asn
   385                 390                 395                 400
   Glu Gly Glu Leu Ser Asp Leu Lys Asp Gln Phe Asp Ala Ile Asp Val
                   405                 410                 415
   Asp Lys Asn Gly Ala Ile Ser Leu Glu Glu Met Arg Gln Ala Leu Ala
               420                 425                 430
   Lys Asp Leu Pro Trp Lys Leu Lys Glu Ser Arg Val Leu Glu Ile Leu
           435                 440                 445
   Gln Ala Ile Asp Ser Asn Thr Asp Gly Leu Val Asp Phe Thr Glu Phe
       450                 455                 460
   Val Ala Ala Thr Leu His Val His Gln Leu Glu Glu His Asp Ser Asp
   465                 470                 475                 480
   Lys Trp Gln Gln Arg Ser Gln Ala Ala Phe Glu Lys Phe Asp Ile Asp
                   485                 490                 495
   Lys Asp Gly Tyr Ile Thr Pro Glu Glu Leu Arg Met His Thr Gly Met
               500                 505                 510
   Arg Gly Ser Ile Asp Pro Leu Leu Glu Glu Ala Asp Ile Asp Lys Asp
           515                 520                 525
   Gly Lys Ile Ser Leu Pro Glu Phe Arg Arg Leu Leu Arg Thr Ala Ser
       530                 535                 540
   Ile Gly Ser Arg Asn Val Thr Ser Pro Thr Leu Arg His Arg Arg Ile
   545 550 555 560
<210> 8 <211> 1683
   <212> DNA
   <213> Medicago truncatula
<400> 8
   atgggtttat gtttttcttc tacaaaggtt gttagtggtt ctaacagcaa caccacaaac      60
   aatgataacc gtaaacggaa tcagtcaaca accacggata ccaccgtcac ggtaacaaca     120
   gcaacaacgg cggcgcagaa acaaacggca cagagacgta aaggagggtc aaatgaaaca     180
   gcccagaaga agaatcatca tcaacatcat aggttaaaag agaaaacagg ttctaaacat     240
   gttccttgtg gaaaaagaac ggattttggg tatgagaaag attttgataa aagattttct     300
   cttgggaaat tgttgggtca tggacaattt ggttatactt atgttggtgt tgataaatcc     360
   aatggagatc gtgtcgccgt taagcgactc gagaaagcta agatggttct ccctatagca     420
   gttgaggatg taaaaagaga ggtcaagata ttgaaagaac ttacaggcca tgaaaatgtg     480
   gttcagtttt ataatgcttt tgacgatgat tcatatgtgt acatagttat ggagttatgc     540
   gaaggtggag aactactaga ccggatacta aacaaaaagg acagccgtta tactgaaaaa     600
   gatgccgccg ttgttgtaag gcagatgctg aaggttgcag ctcagtgtca tttacatggt     660
   ttggtacatc gcgacatgaa gccagagaat tttcttttca aatcaaacaa agaagattca      720
   gctttgaagg ccactgattt tggtttgtct gatttcataa aacctggaaa gaggtttcaa     780
   gatattgttg gaagtgctta ctatgtcgca ccagaagtgt tgaaacggaa gtcaggccct     840
   gaatcagatg tatggagtat tggtgtgatt acatacatat tgctttgtgg gagacgtcca     900
   ttttgggaca agactgaaga tggtatcttc aaggaggtct tacgaaacaa gcctgatttc     960
   cggcggaaac catggccaac cataagtaat gctgcaaaag attttgtgaa gaaattgttg    1020
   gtaaaagatc ctcgggcaag attaacggct gctcaggctc tatcacatcc atgggttaga    1080
   gaaggaggag aggcatccga gattccaatt gatatatctg tcctaaacaa catgcggcag    1140
   tttgtgaagt atagtcgatt gaaacaattt gcattgaggg cattggctag cacacttaac    1200
   gaaggtgagt tgtctgatct aaaagatcag tttgatgcaa tagatgtgga caaaaatggt    1260
   gctattagtc ttgaagagat gagacaggct cttgctaaag atctcccgtg gaagttgaaa    1320
   gaatcccgtg tattggagat attgcaagcg attgacagca acacggatgg attagtagat    1380
   ttcaccgagt ttgttgcagc tactttacat gtgcatcaat tggaggaaca tgactccgac    1440
   aagtggcagc aacggtcaca ggctgctttt gagaaatttg acatagacaa ggatggctat    1500
   attactccag aggaactcag aatgcacact ggtatgagag gttccatcga tcctctgctc    1560
   gaggaagccg atattgacaa agatggaaag atcagcttac cagaattcag gagactttta    1620
   agaactgcaa gcattggttc tcgaaacgta acaagcccaa ctctacgtca tcgaaggatc    1680
   tag                                                                  1683
<210> 9
   <211> 560
   <212> PRT
   <213> Medicago truncatula
<400> 9
   Met Gly Leu Cys Phe Ser Ser Thr Lys Val Val Ser Gly Ser Asn Ser
   1               5                   10                  15
   Asn Thr Thr Asn Asn Asp Asn Arg Lys Arg Asn Gln Ser Thr Thr Thr
               20                  25                  30
   Asp Thr Thr Val Thr Val Thr Thr Ala Thr Thr Ala Ala Gln Lys Gln
           35                  40                  45
   Thr Ala Gln Arg Arg Lys Gly Gly Ser Asn Glu Thr Ala Gln Lys Lys
       50                  55                  60
   Asn His His Gln His His Arg Leu Lys Glu Lys Thr Gly Ser Lys His
   65                  70                  75                  80
   Val Pro Cys Gly Lys Arg Thr Asp Phe Gly Tyr Glu Lys Asp Phe Asp
                   85                  90                  95
   Lys Arg Phe Ser Leu Gly Lys Leu Leu Gly His Gly Gln Phe Gly Tyr
               100                 105                 110
   Thr Tyr Val Gly Val Asp Lys Ser Asn Gly Asp Arg Val Ala Val Lys
           115                 120                 125
   Arg Leu Glu Lys Ala Lys Met Val Leu Pro Ile Ala Val Glu Asp Val
       130                 135                 140
   Lys Arg Glu Val Lys Ile Leu Lys Glu Leu Thr Gly His Glu Asn Val
   145                 150                 155                 160
   Val Gln Phe Tyr Asn Ala Phe Asp Asp Asp Ser Tyr Val Tyr Ile Val
                   165                 170                 175
   Met Glu Leu Cys Glu Gly Gly Glu Leu Leu Asp Arg Ile Leu Asn Lys
   180 185 190
   Lys Asp Ser Arg Tyr Thr Glu Lys Asp Ala Ala Val Val Val Arg Gln
   195 200 205
   Met Leu Lys Val Ala Ala Gln Cys His Leu His Gly Leu Val His Arg
   210 215 220
   Asp Met Lys Pro Glu Asn Phe Leu Phe Lys Ser Asn Lys Glu Asp Ser
   225 230 235 240
   Ala Leu Lys Ala Thr Asp Phe Gly Leu Ser Asp Phe Ile Lys Pro Gly
   245 250 255
   Lys Arg Phe Gln Asp Ile Val Gly Ser Ala Tyr Tyr Val Ala Pro Glu
   260 265 270
   Val Leu Lys Arg Lys Ser Gly Pro Glu Ser Asp Val Trp Ser Ile Gly
   275 280 285
   Val Ile Thr Tyr Ile Leu Leu Cys Gly Arg Arg Pro Phe Trp Asp Lys
   290 295 300
   Thr Glu Asp Gly Ile Phe Lys Glu Val Leu Arg Asn Lys Pro Asp Phe
   305 310 315 320
   Arg Arg Lys Pro Trp Pro Thr Ile Ser Asn Ala Ala Lys Asp Phe Val
   325 330 335
   Lys Lys Leu Leu Val Lys Asp Pro Arg Ala Arg Leu Thr Ala Ala Gln
   340 345 350
   Ala Leu Ser His Pro Trp Val Arg Glu Gly Gly Glu Ala Ser Glu Ile
   355 360 365
   Pro Ile Asp Ile Ser Val Leu Asn Asn Met Arg Gln Phe Val Lys Tyr
   370 375 380
   Ser Arg Leu Lys Gln Phe Ala Leu Arg Ala Leu Ala Ser Thr Leu Asn
   385 390 395 400
   Glu Gly Glu Leu Ser Asp Leu Lys Asp Gln Phe Asp Ala Ile Asp Val
   405 410 415
   Asp Lys Asn Gly Ala Ile Ser Leu Glu Glu Met Arg Gln Ala Leu Ala
   420 425 430
   Lys Asp Leu Pro Trp Lys Leu Lys Glu Ser Arg Val Leu Glu Ile Leu
           435                 440                 445
   Gln Ala Ile Asp Ser Asn Thr Asp Gly Leu Val Asp Phe Thr Glu Phe
       450                 455                 460
   Val Ala Ala Thr Leu His Val His Gln Leu Glu Glu His Asp Ser Asp
   465                 470                 475                 480
   Lys Trp Gln Gln Arg Ser Gln Ala Ala Phe Glu Lys Phe Asp Ile Asp
                   485                 490                 495
   Lys Asp Gly Tyr Ile Thr Pro Glu Glu Leu Arg Met His Thr Gly Met
               500                 505                 510
   Arg Gly Ser Ile Asp Pro Leu Leu Glu Glu Ala Asp Ile Asp Lys Asp
           515                 520                 525
   Gly Lys Ile Ser Leu Pro Glu Phe Arg Arg Leu Leu Arg Thr Ala Ser
       530                 535                 540
   Ile Gly Ser Arg Asn Val Thr Ser Pro Thr Leu Arg His Arg Arg Ile
   545                 550                 555                 560
<210> 10
   <211> 1719
   <212> DNA
   <213> Nicotiana tabacum
<400> 10
   atgggtaata actgtttttc tagctcaaaa gttagtggtt ctaacagcaa caccccctcc 60
   accaccgcca cagccaccac cgtgaatgtc cggaggaaca aagcaaatcc accttctaca 120
   tccacaatta catcaacaaa acaagaaggg tctcattgca ataaacagaa agttaaagat 180
   aaccacaaaa gccaacacca aaaacaacaa cctagaaatt ctcagcaaaa tgttaagaag 240
   cataataatg ggaggagaca gaagagtggg gttattgctt gtgggaaaag aactgatttt 300
   gggtatgata aagattttga taagaggttt accattggga agttgttggg tcatggccaa 360
   tttggttata cctacgttgc cacccacaag tctaatggag atcgcgtcgc tgtcaagaga 420
   attgagaaga acaagatggt tcttccgatt gcagttgagg atgtaaaacg agaagtcaag 480
   atattgaagg ccttatccgg tcatgagaat gtggttcaat tcaataatgc atttgaggat 540
   gataactacg tctacatagt aatggagtta tgtgagggtg gagaactctt ggaccgcatt 600
   ttggccaaaa aggacagccg ttatgccgag aaagatgcag caatagttgt acgtcagatg 660
   ctaaaagaag ccgctcaatg tcatttacat ggtttggtgc atcgtgatat gaaacctgag 720
   aattttctct ttaaatcttc aaaggaggat tcaccattga aggccacaga ttttggtctt 780
   tcagacttca taagaccagg gaagaagttc caagatattg ttggtagtgc atattacgta 840
   gcgccagagg tattaaagcg tagatcagga cccgaatcag atgagtggag tattggtgtt 900
   attacataca ttttgctctg tggccgtcgc cgcttctggg ataaaacaga ggatggcata 960
   ttcaaggagg tactaagaaa caagcctgat tttcgtcgca agccgtggcc aactatcagc 1020
   aacagtgcta aagattttgt taagaaatta ttggtgaagg atcctcgtgc tagacttact 1080
   gctgcccagg ccctatcgca tccatgggtc cgcgaaggag gtgatgcatc tgagattcca 1140
   ctggacattt ctgtcttatc aaacatgcga caatttgtca agtacagtcg attaaaacag 1200
   tttgctttac gggcattggc tagcacagtt gatgaggagg agctggcaga tgtgcgggat 1260
   cagttttctg caattgatgt ggataaaaat ggtgtcatta gccttgaaga aatgagacag 1320
   gcccttgcta aggatcttcc ctggaagatg aaagagtcac gggttcttga gattcttcaa 1380
   gcgattgata gtaactctga tgggctactt gatttcccag agtttgtcgc agccactcta 1440
   catgtccatc agttggagga gcataattct ataaaatggc aggaaagatc gcaagctgct 1500
   tttgaggaat ttgatgttga tagagatgga ttcataactc cagaagaact tagaatgcat 1560
   actggattaa agggctccat agacccactt ctagaagaag cagatatcga caaagatggg 1620
   aagataagct tgtcggaatt tcgtaggctt ctaagaactg caagtataag ctcgcggatg 1680
   gtgactagtc caactgttag aggctctcgg aaaagttag 1719
<210> 11
   <211> 572
   <212> PRT
   <213> Nicotiana tabacum
<400> 11
   Met Gly Asn Asn Cys Phe Ser Ser Ser Lys Val Ser Gly Ser Asn Ser
   1               5                   10                  15
   Asn Thr Pro Ser Thr Thr Ala Thr Ala Thr Thr Val Asn Val Arg Arg
               20                  25                  30
   Asn Lys Ala Asn Pro Pro Ser Thr Ser Thr Ile Thr Ser Thr Lys Gln
           35                  40                  45
   Glu Gly Ser His Cys Asn Lys Gln Lys Val Lys Asp Asn His Lys Ser
       50                  55                  60
   Gln His Gln Lys Gln Gln Pro Arg Asn Ser Gln Gln Asn Val Lys Lys
   65                  70                  75                  80
   His Asn Asn Gly Arg Arg Gln Lys Ser Gly Val Ile Ala Cys Gly Lys
                   85                  90                  95
   Arg Thr Asp Phe Gly Tyr Asp Lys Asp Phe Asp Lys Arg Phe Thr Ile
               100                 105                 110
   Gly Lys Leu Leu Gly His Gly Gln Phe Gly Tyr Thr Tyr Val Ala Thr
           115                 120                 125
   His Lys Ser Asn Gly Asp Arg Val Ala Val Lys Arg Ile Glu Lys Asn
       130                 135                 140
   Lys Met Val Leu Pro Ile Ala Val Glu Asp Val Lys Arg Glu Val Lys
   145                 150                 155                 160
   Ile Leu Lys Ala Leu Ser Gly His Glu Asn Val Val Gln Phe Asn Asn
                   165                 170                 175
   Ala Phe Glu Asp Asp Asn Tyr Val Tyr Ile Val Met Glu Leu Cys Glu
               180                 185                 190
   Gly Gly Glu Leu Leu Asp Arg Ile Leu Ala Lys Lys Asp Ser Arg Tyr
           195                 200                 205
   Ala Glu Lys Asp Ala Ala Ile Val Val Arg Gln Met Leu Lys Glu Ala
       210                 215                 220
   Ala Gln Cys His Leu His Gly Leu Val His Arg Asp Met Lys Pro Glu
   225                 230                 235                 240
   Asn Phe Leu Phe Lys Ser Ser Lys Glu Asp Ser Pro Leu Lys Ala Thr
                   245                 250                 255
   Asp Phe Gly Leu Ser Asp Phe Ile Arg Pro Gly Lys Lys Phe Gln Asp
               260                 265                 270
   Ile Val Gly Ser Ala Tyr Tyr Val Ala Pro Glu Val Leu Lys Arg Arg
           275                 280                 285
   Ser Gly Pro Glu Ser Asp Glu Trp Ser Ile Gly Val Ile Thr Tyr Ile
       290                 295                 300
   Leu Leu Cys Gly Arg Arg Arg Phe Trp Asp Lys Thr Glu Asp Gly Ile
   305 310 315 320
   Phe Lys Glu Val Leu Arg Asn Lys Pro Asp Phe Arg Arg Lys Pro Trp
   325 330 335
   Pro Thr Ile Ser Asn Ser Ala Lys Asp Phe Val Lys Lys Leu Leu Val
   340 345 350
   Lys Asp Pro Arg Ala Arg Leu Thr Ala Ala Gln Ala Leu Ser His Pro
   355 360 365
   Trp Val Arg Glu Gly Gly Asp Ala Ser Glu Ile Pro Leu Asp Ile Ser
   370 375 380
   Val Leu Ser Asn Met Arg Gln Phe Val Lys Tyr Ser Arg Leu Lys Gln
   385 390 395 400
   Phe Ala Leu Arg Ala Leu Ala Ser Thr Val Asp Glu Glu Glu Leu Ala
   405 410 415
   Asp Val Arg Asp Gln Phe Ser Ala Ile Asp Val Asp Lys Asn Gly Val
   420 425 430
   Ile Ser Leu Glu Glu Met Arg Gln Ala Leu Ala Lys Asp Leu Pro Trp
   435 440 445
   Lys Met Lys Glu Ser Arg Val Leu Glu Ile Leu Gln Ala Ile Asp Ser
   450 455 460
   Asn Ser Asp Gly Leu Leu Asp Phe Pro Glu Phe Val Ala Ala Thr Leu
   465 470 475 480
   His Val His Gln Leu Glu Glu His Asn Ser Ile Lys Trp Gln Glu Arg
   485 490 495
   Ser Gln Ala Ala Phe Glu Glu Phe Asp Val Asp Arg Asp Gly Phe Ile
   500 505 510
   Thr Pro Glu Glu Leu Arg Met His Thr Gly Leu Lys Gly Ser Ile Asp
   515 520 525
   Pro Leu Leu Glu Glu Ala Asp Ile Asp Lys Asp Gly Lys Ile Ser Leu
   530 535 540
   Ser Glu Phe Arg Arg Leu Leu Arg Thr Ala Ser Ile Ser Ser Arg Met
   545 550 555 560
   Val Thr Ser Pro Thr Val Arg Gly Ser Arg Lys Ser
   565 570
<210> 12
   <211> 1704
   <212> DNA
   <213> Nicotiana tabacum
<400> 12
   atgggcagct gtttttctag ctccaaagtt agtggctcaa atagcaatac cccttctaca      60
   actactacaa atgtaaatgt tcatcacaac cgtccttcaa caacaacaac aacaactgtt     120
   acatcaagaa aacaagaggg gtcaaattat aatagagata aaggtaatat taatacaaaa     180
   aacagccacc aaaaacaaca acctaggagt tctcagcaga atgttgttgt taagccaagt     240
   tcaagaagac aaagtggagg ggttattcct tgtgggaaaa gaacagattt tgggtatgat     300
   aaagattttg ataagaggta tactattggt aaattgttgg gtcatggcca atttgggtat     360
   acatatgttg ctacagatag atcttctgga gatcgtgttg ctgttaagaa aattgagaaa     420
   aacaagatgg ttcttccaat tgcggttgag gacgtgaaac gagaagtcaa gatattgaag     480
   gccttagctg gtcacgagaa tgtggttcaa ttctataatt catttgagga tgataattat     540
   gtctacatcg taatggagtt atgtgagggt ggagaactat tggaccgaat cttgtccaaa     600
   aaagatagtc gatatactga gaaagatgcg gcgatagttg tacgccagat gctaaaagtg     660
   gctgctgagt gtcatttaca tggtctggtg catcgagata tgaaacctga gaattttctc     720
   tttaagctct tcaaaggtgg attcgccatt aaaagcacag attttggtct ttcagacttc     780
   ataagaccag ggaaaaagtt ccaagacatt gttggcagtg catattatgt agccccagag     840
   gtgttaaagc gtagatcagg acctgaatca gatgtatgga gtataggtgt aattacatac     900
   attttgctat gcggccgtcg ccgcttctgg gacaaaactg aggatggtat attcaaggag     960
   gtcctacgaa acaagcctga ttttcgtcgc aagccatggc caaacataag caacagtgct    1020
   aaagattttg taaagaaatt actggtgaag gatccgcgcg ctagacttac tgccgctcag    1080
   gccctatcgc atccatgggt ccgagaagga ggtatcgcat ctgagatccc actcgacatt    1140
   tctgttttat ccaacatgcg gcaatttgtc agatatagtc gcctaaaaca gttcgcttta    1200
   cgggcgttag ctagcacgct tgatgaggag gagctctctg atctgaagga tcaattttct    1260
   gcaattgatg tggataagaa tggtgtcatc agtctcgaag aaatgagaca ggcccttgcc    1320
   aaggatctcc catggaaaat gaaagagtca cgagttcttg agattcttca agcgattgat    1380
   agtaacacag acgggcttgt tgatttcccg gagtttgtgg ccgccactct acatgtccat    1440
   cagttggagg agcataattc tacaaaatgg cagcaaagat cgcaagctgc ttttgagaaa    1500
   tttgatgttg atagagatgg attcataacc ccggaagaac ttaaaatgca cacgggtttg    1560
   aaaggatcga tagacccact tttagaggaa gcggacattg acaaagacgg gaagataagc    1620
   ctgtcagaat tccgtaggct tttaagaact gctagtatga gttcaccaac ggtgagagat    1680
   tcacggagaa atgtagcttt gtaa                                          1704
<210> 13
   <211> 567
   <212> PRT
   <213> Nicotiana tabacum
<400> 13
   Met Gly Ser Cys Phe Ser Ser Ser Lys Val Ser Gly Ser Asn Ser Asn
   1 5 10 15
   Thr Pro Ser Thr Thr Thr Thr Asn Val Asn Val His His Asn Arg Pro
   20 25 30
   Ser Thr Thr Thr Thr Thr Thr Val Thr Ser Arg Lys Gln Glu Gly Ser
   35 40 45
   Asn Tyr Asn Arg Asp Lys Gly Asn Ile Asn Thr Lys Asn Ser His Gln
   50 55 60
   Lys Gln Gln Pro Arg Ser Ser Gln Gln Asn Val Val Val Lys Pro Ser
   65 70 75 80
   Ser Arg Arg Gln Ser Gly Gly Val Ile Pro Cys Gly Lys Arg Thr Asp
   85 90 95
   Phe Gly Tyr Asp Lys Asp Phe Asp Lys Arg Tyr Thr Ile Gly Lys Leu
   100 105 110
   Leu Gly His Gly Gln Phe Gly Tyr Thr Tyr Val Ala Thr Asp Arg Ser
   115 120 125
   Ser Gly Asp Arg Val Ala Val Lys Lys Ile Glu Lys Asn Lys Met Val
   130 135 140
   Leu Pro Ile Ala Val Glu Asp Val Lys Arg Glu Val Lys Ile Leu Lys
   145 150 155 160
   Ala Leu Ala Gly His Glu Asn Val Val Gln Phe Tyr Asn Ser Phe Glu
   165 170 175
   Asp Asp Asn Tyr Val Tyr Ile Val Met Glu Leu Cys Glu Gly Gly Glu
   180 185 190
   Leu Leu Asp Arg Ile Leu Ser Lys Lys Asp Ser Arg Tyr Thr Glu Lys
   195 200 205
   Asp Ala Ala Ile Val Val Arg Gln Met Leu Lys Val Ala Ala Glu Cys
   210 215 220
   His Leu His Gly Leu Val His Arg Asp Met Lys Pro Glu Asn Phe Leu
   225 230 235 240
   Phe Lys Leu Phe Lys Gly Gly Phe Ala Ile Lys Ser Thr Asp Phe Gly
   245 250 255
   Leu Ser Asp Phe Ile Arg Pro Gly Lys Lys Phe Gln Asp Ile Val Gly
   260 265 270
   Ser Ala Tyr Tyr Val Ala Pro Glu Val Leu Lys Arg Arg Ser Gly Pro
   275 280 285
   Glu Ser Asp Val Trp Ser Ile Gly Val Ile Thr Tyr Ile Leu Leu Cys
   290 295 300
   Gly Arg Arg Arg Phe Trp Asp Lys Thr Glu Asp Gly Ile Phe Lys Glu
   305 310 315 320
   Val Leu Arg Asn Lys Pro Asp Phe Arg Arg Lys Pro Trp Pro Asn Ile
   325 330 335
   Ser Asn Ser Ala Lys Asp Phe Val Lys Lys Leu Leu Val Lys Asp Pro
   340 345 350
   Arg Ala Arg Leu Thr Ala Ala Gln Ala Leu Ser His Pro Trp Val Arg
   355 360 365
   Glu Gly Gly Ile Ala Ser Glu Ile Pro Leu Asp Ile Ser Val Leu Ser
   370 375 380
   Asn Met Arg Gln Phe Val Arg Tyr Ser Arg Leu Lys Gln Phe Ala Leu
   385 390 395 400
   Arg Ala Leu Ala Ser Thr Leu Asp Glu Glu Glu Leu Ser Asp Leu Lys
   405 410 415
   Asp Gln Phe Ser Ala Ile Asp Val Asp Lys Asn Gly Val Ile Ser Leu
   420 425 430
   Glu Glu Met Arg Gln Ala Leu Ala Lys Asp Leu Pro Trp Lys Met Lys
   435 440 445
   Glu Ser Arg Val Leu Glu Ile Leu Gln Ala Ile Asp Ser Asn Thr Asp
   450 455 460
   Gly Leu Val Asp Phe Pro Glu Phe Val Ala Ala Thr Leu His Val His
   465 470 475 480
   Gln Leu Glu Glu His Asn Ser Thr Lys Trp Gln Gln Arg Ser Gln Ala
   485 490 495
   Ala Phe Glu Lys Phe Asp Val Asp Arg Asp Gly Phe Ile Thr Pro Glu
   500 505 510
   Glu Leu Lys Met His Thr Gly Leu Lys Gly Ser Ile Asp Pro Leu Leu
   515 520 525
   Glu Glu Ala Asp Ile Asp Lys Asp Gly Lys Ile Ser Leu Ser Glu Phe
   530 535 540
   Arg Arg Leu Leu Arg Thr Ala Ser Met Ser Ser Pro Thr Val Arg Asp
   545 550 555 560
   Ser Arg Arg Asn Val Ala Leu
   565
<210> 14
   <211> 1695
   <212> DNA
   <213> Solanum tuberosum
<400> 14
   atgggtagtt gtttttcaag ctccaaagtt agtggctcaa atagcaatac cccttcaacc 60
   aacaataccg ccacaaacac aaacacaacg gtaaatgttc atccaaaccg cagggaaacc 120
   tcaaaagcac cttcaacaac ggttgttaat tcaagaaatc aagaagggtc gaattataat 180
   cgaggtaaag gtaatattaa ccagaaaaac caacaaaaac aacctaggaa ttcacagcag 240
   aatgttaagc caagttcaag aagacaaggt ggggttattc cttgtgggaa aagaacagat 300
   tttgggtatg ataaagattt tgaaaagaga tatacaattg ggaaattgtt gggtcatggt 360
   caatttggtt atacatatgt tgctacagat aaatcttcag gagatcgtgt ggctgtcaaa 420
   agaattgaga aaaacaagat ggttcttccc attgcggttg aggatgtgaa acgagaagtc 480
   aagatattga aggccttagg tcgtcatgag aatgtggttc aattctataa ttcattcgag 540
   gatcataatt atgtctacat cgtaatggag ttatgtgaag gtggagaact attggacgat 600
   tgtcaaaaga cagtcggtat acgagaagat gcagcaatag tcgtacccca gatgctaaaa 660
   gtggcagctg agtgtcattt acatggtttg gtgcatcgtg atatgaaacc tgagaatttt 720
   ctctttaagt ctacaaagga ggattcacca ttaaaagcca cagattttgg atcttcagac 780
   ttcatcagac caggaaaagt ccaagacatt gtcggtagtg catattatgt agctccagag 840
   gtattaaagc gtagatcagg acctgaatca gatgtgtgga gtattggcgt aattacatac 900
   attttgctat gtggtcgtcg gcctttctgg gacaaaactg aggatggtat attcaaggag 960
   gtcctacgga acaaacctga ttttcgtcgc aagccatggt ctaacataag caacagtgct 1020
   aaagattttg taaagaaatt actggtgaag gatcctcgcg ctagacttac tgctgctcag 1080
   gccctatcac atccttgggt ccgagaggga ggggatgcat ctgagattcc actggacatt 1140
   tctgttttat ccaacatgcg gcaatttgtc agatacagtc atctaaaaca gtttgcttta 1200
   cgggcgttac gtagcacact tgatgaggag gagatcgctg atctgcggga tcaattttct 1260
   gcaattgatg tggataagaa tggtgtcatc agtctcgaag aaatgagaca ggcccttgcg 1320
   aaggatctcc catggaaaat gaaagaatca cgagttcttg agattcttca agcgattgac 1380
   agtaacacag atgggcttgt tgattttccg gagtttgtgg ctgccactct acatgtccat 1440
   cagttggagg agcataattc tgcaaagtgg cagcaaagat cacaagctgc ttttgagaaa 1500
   tttgatgttg atagagatgg attcattact ccagaagaac ttaaaatgca cacgggcttg 1560
   agaggctcca tagatccact tctagaggaa gcagatattg acaaagacgg aaagataagc 1620
   atatcagaat ttcgtagact tttaagaact gctagtatga cttcaccaac ggtgagagat 1680
   tcacggggta tgtag 1695
<210> 15
   <211> 564
   <212> PRT
   <213> Solanum tuberosum
<400> 15
   Met Gly Ser Cys Phe Ser Ser Ser Lys Val Ser Gly Ser Asn Ser Asn
   1               5                   10                  15
   Thr Pro Ser Thr Asn Asn Thr Ala Thr Asn Thr Asn Thr Thr Val Asn
               20                  25                  30
   Val His Pro Asn Arg Arg Glu Thr Ser Lys Ala Pro Ser Thr Thr Val
           35                  40                  45
   Val Asn Ser Arg Asn Gln Glu Gly Ser Asn Tyr Asn Arg Gly Lys Gly
       50                  55                  60
   Asn Ile Asn Gln Lys Asn Gln Gln Lys Gln Pro Arg Asn Ser Gln Gln
   65                  70                  75                  80
   Asn Val Lys Pro Ser Ser Arg Arg Gln Gly Gly Val Ile Pro Cys Gly
                   85                  90                  95
   Lys Arg Thr Asp Phe Gly Tyr Asp Lys Asp Phe Glu Lys Arg Tyr Thr
               100                 105                 110
   Ile Gly Lys Leu Leu Gly His Gly Gln Phe Gly Tyr Thr Tyr Val Ala
           115                 120                 125
   Thr Asp Lys Ser Ser Gly Asp Arg Val Ala Val Lys Arg Ile Glu Lys
       130                 135                 140
   Asn Lys Met Val Leu Pro Ile Ala Val Glu Asp Val Lys Arg Glu Val
   145                 150                 155                 160
   Lys Ile Leu Lys Ala Leu Gly Arg His Glu Asn Val Val Gln Phe Tyr
                   165                 170                 175
   Asn Ser Phe Glu Asp His Asn Tyr Val Tyr Ile Val Met Glu Leu Cys
               180                 185                 190
   Glu Gly Gly Glu Leu Leu Asp Asp Cys Gln Lys Thr Val Gly Ile Arg
           195                 200                 205
   Glu Asp Ala Ala Ile Val Val Pro Gln Met Leu Lys Val Ala Ala Glu
       210                 215                 220
   Cys His Leu His Gly Leu Val His Arg Asp Met Lys Pro Glu Asn Phe
   225                 230                 235                 240
   Leu Phe Lys Ser Thr Lys Glu Asp Ser Pro Leu Lys Ala Thr Asp Phe
                   245                 250                 255
   Gly Ser Ser Asp Phe Ile Arg Pro Gly Lys Val Gln Asp Ile Val Gly
               260                 265                 270
   Ser Ala Tyr Tyr Val Ala Pro Glu Val Leu Lys Arg Arg Ser Gly Pro
           275                 280                 285
   Glu Ser Asp Val Trp Ser Ile Gly Val Ile Thr Tyr Ile Leu Leu Cys
   290 295 300
   Gly Arg Arg Pro Phe Trp Asp Lys Thr Glu Asp Gly Ile Phe Lys Glu
   305 310 315 320
   Val Leu Arg Asn Lys Pro Asp Phe Arg Arg Lys Pro Trp Ser Asn Ile
   325 330 335
   Ser Asn Ser Ala Lys Asp Phe Val Lys Lys Leu Leu Val Lys Asp Pro
   340 345 350
   Arg Ala Arg Leu Thr Ala Ala Gln Ala Leu Ser His Pro Trp Val Arg
   355 360 365
   Glu Gly Gly Asp Ala Ser Glu Ile Pro Leu Asp Ile Ser Val Leu Ser
   370 375 380
   Asn Met Arg Gln Phe Val Arg Tyr Ser His Leu Lys Gln Phe Ala Leu
   385 390 395 400
   Arg Ala Leu Arg Ser Thr Leu Asp Glu Glu Glu Ile Ala Asp Leu Arg
   405 410 415
   Asp Gln Phe Ser Ala Ile Asp Val Asp Lys Asn Gly Val Ile Ser Leu
   420 425 430
   Glu Glu Met Arg Gln Ala Leu Ala Lys Asp Leu Pro Trp Lys Met Lys
   435 440 445
   Glu Ser Arg Val Leu Glu Ile Leu Gln Ala Ile Asp Ser Asn Thr Asp
   450 455 460
   Gly Leu Val Asp Phe Pro Glu Phe Val Ala Ala Thr Leu His Val His
   465 470 475 480
   Gln Leu Glu Glu His Asn Ser Ala Lys Trp Gln Gln Arg Ser Gln Ala
   485 490 495
   Ala Phe Glu Lys Phe Asp Val Asp Arg Asp Gly Phe Ile Thr Pro Glu
   500 505 510
   Glu Leu Lys Met His Thr Gly Leu Arg Gly Ser Ile Asp Pro Leu Leu
   515 520 525
   Glu Glu Ala Asp Ile Asp Lys Asp Gly Lys Ile Ser Ile Ser Glu Phe
   530 535 540
   Arg Arg Leu Leu Arg Thr Ala Ser Met Thr Ser Pro Thr Val Arg Asp
   545 550 555 560
   Ser Arg Gly Met
<210> 16
   <211> 1716
   <212> DNA
   <213> Arabidopsis thaliana
<400> 16
   atgggtctct gtttctcctc cgccgccaaa tcctccggcc acaaccgcag tagccggaat     60
   ccccacccac atcctcctct cacggttgtt aaatccagac caccgcgatc tccatgttcc    120
   ttcatggccg ttacgatcca aaaggatcat agaacgcaac cgcgacgcaa tgcaacggct    180
   aagaaaacgc cgacgcggca tacgccaccg cacgggaaag tgagagagaa agttataagc    240
   aataacggta ggagacatgg agaaacgatc ccctacggta agcgtgtaga ttttgggtac    300
   gctaaagatt ttgatcaccg ttacaccatt ggaaaattgc ttggacatgg tcaatttggt    360
   tatacatatg tcgctaccga taaaaaaacc ggtgatcgtg tcgccgtcaa gaagatcgat    420
   aaggccaaga tgacaattcc gatagctgtg gaagatgtaa agagagaggt gaagatatta    480
   caagctttaa ctggtcatga aaatgtggtt cggttttata atgccttcga ggacaagaac    540
   tctgtttata tagttatgga gttatgcgag ggtggtgaat tacttgatcg tattttagcc    600
   aggaaggata gccgttatag cgagagagac gcggccgtgg tggtgagaca aatgctgaaa    660
   gttgcggctg agtgtcattt acgcggtttg gttcaccgag atatgaaacc agagaatttt    720
   ctgttcaaat caaccgaaga agattcgcct ctaaaagcta ctgatttcgg tttatctgac    780
   ttcataaagc caggcaaaaa gtttcatgat attgttggga gtgcatacta tgtagcacct    840
   gaagtgttaa aacgtaggtc gggacctgaa tcagatgtgt ggagtattgg tgtaatcagt    900
   tacattcttc tctgcgggag acgaccattc tgggataaga ctgaagacgg tatcttcaag    960
   gaggttttga agaacaaacc tgatttcaga agaaaaccgt ggccaaccat tagcaacagc   1020
   gccaaagatt ttgtcaagaa gttgttagta aaagacccga gagcgcgatt aacagctgca   1080
   caagcactat cacatccatg ggttagagaa ggaggagatg catctgagat tcccatagac   1140
   atatctgttc tgaacaacat gcgtcagttt gtgaaattta gccgccttaa gcaattcgct   1200
   ttaagggctc ttgcaacgac acttgatgag gaagagttgg ctgatcttcg agaccagttt   1260
   gatgcgattg atgttgacaa gaatggtgtc attagccttg aagagatgag gcaggctcta   1320
   gcgaaagatc atccttggaa gcttaaggat gcaagagttg ccgagattct tcaagcgatt   1380
   gatagcaaca cagatggatt cgtggacttt ggcgagtttg tcgccgctgc gctacacgta   1440
   aaccaattag aggaacacga ttccgagaag tggcaacaga gatcaagagc agcatttgaa   1500
   aaattcgaca tagatggaga tggatttata acagcagagg aacttcgaat gcatactggc   1560
   ttgaaagggt ccattgagcc acttcttgaa gaagcagaca ttgacaatga tggtaaaatc   1620
   agtctccaag agtttcgtag acttttgaga actgcaagta tcaaatcaag aaatgttaga   1680
   agccctcctg gttatcttat ttctcgcaag gtctaa                             1716
<210> 17
   <211> 571
   <212> PRT
   <213> Arabidopsis thaliana
<400> 17
   Met Gly Leu Cys Phe Ser Ser Ala Ala Lys Ser Ser Gly His Asn Arg
   1               5                   10                  15
   Ser Ser Arg Asn Pro His Pro His Pro Pro Leu Thr Val Val Lys Ser
               20                  25                  30
   Arg Pro Pro Arg Ser Pro Cys Ser Phe Met Ala Val Thr Ile Gln Lys
           35                  40                  45
   Asp His Arg Thr Gln Pro Arg Arg Asn Ala Thr Ala Lys Lys Thr Pro
       50                  55                  60
   Thr Arg His Thr Pro Pro His Gly Lys Val Arg Glu Lys Val Ile Ser
   65                  70                  75                  80
   Asn Asn Gly Arg Arg His Gly Glu Thr Ile Pro Tyr Gly Lys Arg Val
                   85                  90                  95
   Asp Phe Gly Tyr Ala Lys Asp Phe Asp His Arg Tyr Thr Ile Gly Lys
               100                 105                 110
   Leu Leu Gly His Gly Gln Phe Gly Tyr Thr Tyr Val Ala Thr Asp Lys
           115                 120                 125
   Lys Thr Gly Asp Arg Val Ala Val Lys Lys Ile Asp Lys Ala Lys Met
       130                 135                 140
   Thr Ile Pro Ile Ala Val Glu Asp Val Lys Arg Glu Val Lys Ile Leu
   145                 150                 155                 160
   Gln Ala Leu Thr Gly His Glu Asn Val Val Arg Phe Tyr Asn Ala Phe
                   165                 170                 175
   Glu Asp Lys Asn Ser Val Tyr Ile Val Met Glu Leu Cys Glu Gly Gly
               180                 185                 190
   Glu Leu Leu Asp Arg Ile Leu Ala Arg Lys Asp Ser Arg Tyr Ser Glu
           195                 200                 205
   Arg Asp Ala Ala Val Val Val Arg Gln Met Leu Lys Val Ala Ala Glu
       210                 215                 220
   Cys His Leu Arg Gly Leu Val His Arg Asp Met Lys Pro Glu Asn Phe
   225                 230                 235                 240
   Leu Phe Lys Ser Thr Glu Glu Asp Ser Pro Leu Lys Ala Thr Asp Phe
                   245                 250                 255
   Gly Leu Ser Asp Phe Ile Lys Pro Gly Lys Lys Phe His Asp Ile Val
               260                 265                 270
   Gly Ser Ala Tyr Tyr Val Ala Pro Glu Val Leu Lys Arg Arg Ser Gly
           275                 280                 285
   Pro Glu Ser Asp Val Trp Ser Ile Gly Val Ile Ser Tyr Ile Leu Leu
       290                 295                 300
   Cys Gly Arg Arg Pro Phe Trp Asp Lys Thr Glu Asp Gly Ile Phe Lys
   305                 310                 315                 320
   Glu Val Leu Lys Asn Lys Pro Asp Phe Arg Arg Lys Pro Trp Pro Thr
                   325                 330                 335
   Ile Ser Asn Ser Ala Lys Asp Phe Val Lys Lys Leu Leu Val Lys Asp
               340                 345                 350
   Pro Arg Ala Arg Leu Thr Ala Ala Gln Ala Leu Ser His Pro Trp Val
           355                 360                 365
   Arg Glu Gly Gly Asp Ala Ser Glu Ile Pro Ile Asp Ile Ser Val Leu
       370                 375                 380
   Asn Asn Met Arg Gln Phe Val Lys Phe Ser Arg Leu Lys Gln Phe Ala
   385                 390                 395                 400
   Leu Arg Ala Leu Ala Thr Thr Leu Asp Glu Glu Glu Leu Ala Asp Leu
                   405                 410                 415
   Arg Asp Gln Phe Asp Ala Ile Asp Val Asp Lys Asn Gly Val Ile Ser
               420                 425                 430
   Leu Glu Glu Met Arg Gln Ala Leu Ala Lys Asp His Pro Trp Lys Leu
           435                 440                 445
   Lys Asp Ala Arg Val Ala Glu Ile Leu Gln Ala Ile Asp Ser Asn Thr
       450                 455                 460
   Asp Gly Phe Val Asp Phe Gly Glu Phe Val Ala Ala Ala Leu His Val
   465                 470                 475                 480
   Asn Gln Leu Glu Glu His Asp Ser Glu Lys Trp Gln Gln Arg Ser Arg
                   485                 490                 495
   Ala Ala Phe Glu Lys Phe Asp Ile Asp Gly Asp Gly Phe Ile Thr Ala
               500                 505                 510
   Glu Glu Leu Arg Met His Thr Gly Leu Lys Gly Ser Ile Glu Pro Leu
           515                 520                 525
   Leu Glu Glu Ala Asp Ile Asp Asn Asp Gly Lys Ile Ser Leu Gln Glu
       530                 535                 540
   Phe Arg Arg Leu Leu Arg Thr Ala Ser Ile Lys Ser Arg Asn Val Arg
   545                 550                 555                 560
   Ser Pro Pro Gly Tyr Leu Ile Ser Arg Lys Val
                   565                 570
<210> 18
   <211> 1605
   <212> DNA
   <213> Arabidopsis thaliana
<400> 18
   atgggtctct gtttctcgtc tcctaaagcc accagacgtg gcaccggtag tcggaaccct     60
   aatcctgatt ctccgacgca ggggaaggcg agtgagaagg ttagtaataa aaacaagaag    120
   aatacgaaga agatccaatt gaggcaccaa ggagggatcc cgtacggtaa acgcatcgat    180
   tttgggtacg ctaaggattt cgataaccga tacaccattg ggaaattgct cggacatggc    240
   cagtttggct ttacctacgt cgccaccgat aacaacaatg gcaatcgcgt tgcagtcaag    300
   agaatcgaca aggccaagat gactcaaccg attgaagtgg aagatgtgaa gcgagaggtt    360
   aagatactac aagctttagg tgggcatgag aatgtggtag gctttcacaa tgcatttgag    420
   gacaagacct atatttacat tgtcatggag ttatgtgacg gtggtgaatt gctagatcga    480
   atactagcta agaaagatag ccgttacacc gagaaagatg cagcggttgt ggtgagacaa    540
   atgctgaaag ttgcagctga atgtcattta cgaggtctag ttcaccgcga catgaagcca    600
   gagaattttc tgttcaaatc aaccgaagaa ggttcatctc taaaggctac agattttggt    660
   ctgtcggact tcataaagcc aggagtgaaa tttcaggata tagtaggaag cgcatactac    720
   gttgcacctg aagtgttgaa acgtaggtca ggacctgaat cagacgtttg gagtattggt    780
   gtcatcactt acattctgct ctgcgggaga agacccttct gggataagac tcaagacgga    840
   atattcaatg aggtcatgag gaaaaaacct gatttcagag aagtcccatg gccaaccatt    900
   agcaacggtg ccaaagattt cgtgaagaag ttgttagtta aggagcctcg ggcacggtta    960
   acagcagctc aagcgctatc acattcatgg gtgaaagaag gaggtgaggc ctcagaggtt   1020
   ccaatagaca tatctgttct taacaatatg cgtcagtttg tgaaattcag ccgtctgaag   1080
   cagattgcac ttagggctct ggcgaaaaca attaatgagg atgagctgga tgatctcaga   1140
   gaccagtttg atgcgattga cattgacaag aacggttcta taagccttga ggaaatgagg   1200
   caggctcttg cgaaagatgt tccttggaaa ctcaaagatg caagagttgc agagattctc   1260
   caagcgaatg atagcaacac tgatggtttg gtggatttca ctgagtttgt ggtggctgct   1320
   ttgcacgtga accaattgga ggagcatgac tctgagaagt ggcaacagag gtcaagagca   1380
   gcatttgaca agtttgacat agacggagat gggtttataa ctcccgaaga acttagattg   1440
   caaacgggtt taaaaggctc catcgaacca cttcttgaag aggccgacgt agatgaagat   1500
   gggagaatca gcatcaatga gtttcgcaga cttttgagaa gtgcaagcct caagtcaaaa   1560
   aatgttaaaa gccctcctgg ttaccagctt tcacaaaaga tgtaa                   1605
<210> 19
   <211> 534
   <212> PRT
   <213> Arabidopsis thaliana
<400> 19
   Met Gly Leu Cys Phe Ser Ser Pro Lys Ala Thr Arg Arg Gly Thr Gly
   1               5                   10                  15
   Ser Arg Asn Pro Asn Pro Asp Ser Pro Thr Gln Gly Lys Ala Ser Glu
               20                  25                  30
   Lys Val Ser Asn Lys Asn Lys Lys Asn Thr Lys Lys Ile Gln Leu Arg
           35                  40                  45
   His Gln Gly Gly Ile Pro Tyr Gly Lys Arg Ile Asp Phe Gly Tyr Ala
       50                  55                  60
   Lys Asp Phe Asp Asn Arg Tyr Thr Ile Gly Lys Leu Leu Gly His Gly
   65                  70                  75                  80
   Gln Phe Gly Phe Thr Tyr Val Ala Thr Asp Asn Asn Asn Gly Asn Arg
                   85                  90                  95
   Val Ala Val Lys Arg Ile Asp Lys Ala Lys Met Thr Gln Pro Ile Glu
               100                 105                 110
   Val Glu Asp Val Lys Arg Glu Val Lys Ile Leu Gln Ala Leu Gly Gly
           115                 120                 125
   His Glu Asn Val Val Gly Phe His Asn Ala Phe Glu Asp Lys Thr Tyr
       130                 135                 140
   Ile Tyr Ile Val Met Glu Leu Cys Asp Gly Gly Glu Leu Leu Asp Arg
   145                 150                 155                 160
   Ile Leu Ala Lys Lys Asp Ser Arg Tyr Thr Glu Lys Asp Ala Ala Val
                   165                 170                 175
   Val Val Arg Gln Met Leu Lys Val Ala Ala Glu Cys His Leu Arg Gly
               180                 185                 190
   Leu Val His Arg Asp Met Lys Pro Glu Asn Phe Leu Phe Lys Ser Thr
           195                 200                 205
   Glu Glu Gly Ser Ser Leu Lys Ala Thr Asp Phe Gly Leu Ser Asp Phe
       210                 215                 220
   Ile Lys Pro Gly Val Lys Phe Gln Asp Ile Val Gly Ser Ala Tyr Tyr
   225                 230                 235                 240
   Val Ala Pro Glu Val Leu Lys Arg Arg Ser Gly Pro Glu Ser Asp Val
                   245                 250                 255
   Trp Ser Ile Gly Val Ile Thr Tyr Ile Leu Leu Cys Gly Arg Arg Pro
               260                 265                 270
   Phe Trp Asp Lys Thr Gln Asp Gly Ile Phe Asn Glu Val Met Arg Lys
           275                 280                 285
   Lys Pro Asp Phe Arg Glu Val Pro Trp Pro Thr Ile Ser Asn Gly Ala
       290                 295                 300
   Lys Asp Phe Val Lys Lys Leu Leu Val Lys Glu Pro Arg Ala Arg Leu
   305                 310                 315                 320
   Thr Ala Ala Gln Ala Leu Ser His Ser Trp Val Lys Glu Gly Gly Glu
                   325                 330                 335
   Ala Ser Glu Val Pro Ile Asp Ile Ser Val Leu Asn Asn Met Arg Gln
               340                 345                 350
   Phe Val Lys Phe Ser Arg Leu Lys Gln Ile Ala Leu Arg Ala Leu Ala
           355                 360                 365
   Lys Thr Ile Asn Glu Asp Glu Leu Asp Asp Leu Arg Asp Gln Phe Asp
       370                 375                 380
   Ala Ile Asp Ile Asp Lys Asn Gly Ser Ile Ser Leu Glu Glu Met Arg
   385                 390                 395                 400
   Gln Ala Leu Ala Lys Asp Val Pro Trp Lys Leu Lys Asp Ala Arg Val
                   405                 410                 415
   Ala Glu Ile Leu Gln Ala Asn Asp Ser Asn Thr Asp Gly Leu Val Asp
               420                 425                 430
   Phe Thr Glu Phe Val Val Ala Ala Leu His Val Asn Gln Leu Glu Glu
           435                 440                 445
   His Asp Ser Glu Lys Trp Gln Gln Arg Ser Arg Ala Ala Phe Asp Lys
       450                 455                 460
   Phe Asp Ile Asp Gly Asp Gly Phe Ile Thr Pro Glu Glu Leu Arg Leu
   465                 470                 475                 480
   Gln Thr Gly Leu Lys Gly Ser Ile Glu Pro Leu Leu Glu Glu Ala Asp
                   485                 490                 495
   Val Asp Glu Asp Gly Arg Ile Ser Ile Asn Glu Phe Arg Arg Leu Leu
               500                 505                 510
   Arg Ser Ala Ser Leu Lys Ser Lys Asn Val Lys Ser Pro Pro Gly Tyr
           515                 520                 525
   Gln Leu Ser Gln Lys Met
   530
<210> 20
   <211> 1572
   <212> DNA
   <213> Arabidopsis thaliana
<400> 20
   atgggtgtct gtttctccgc cattagagtc actggtgcta gcagcagtag acgaagcagt 60
   cagaccaaat ccaaggctgc tcctactccc atcgatacca aggcctctac caaacgccga 120
   accggctcca tcccctgcgg caagcgtacc gattttggct actccaaaga cttccacgat 180
   cactacacca tcggcaagtt gctcggccat ggtcaattcg gctacaccta cgtcgccatc 240
   cacagaccca atggagatcg cgtcgccgtc aaaagactcg ataagtctaa gatggttctt 300
   cctattgctg ttgaggatgt caagcgtgag gttcagattc ttattgctct ctctggccac 360
   gagaatgttg ttcagtttca caatgccttt gaggatgacg attacgtcta tattgttatg 420
   gagttgtgcg aaggaggcga attgctggat aggatattat ccaagaaagg taatcggtac 480
   tccgagaaag atgcagccgt tgtcgttagg cagatgctca aagttgcagg agaatgtcat 540
   ctacacggtc ttgtacatag agatatgaaa ccagagaact ttttgttcaa atcagctcaa 600
   ctagattcgc ctctaaaggc tacggatttt ggtttatcgg attttatcaa accagggaaa 660
   aggttccatg acattgttgg tagcgcctat tatgtggctc ctgaggtatt aaagcgcaga 720
   tcagggcctg aatcagatgt atggagcatt ggtgtgatta cgtatatatt actttgtggg 780
   aggcggcctt tttgggatag aactgaagat ggtatattta aagaggtttt aagaaataaa 840
   cctgacttca gccgtaaacc ttgggcaact ataagtgaca gcgccaaaga ttttgtgaaa 900
   aagttacttg taaaagaccc acgagcacgg ctaactgctg cacaagcact atcacatgcg 960
   tgggttagag aaggcgggaa tgctactgat atccctgtcg acatttcagt tctgaacaac 1020
   ttaagacaat ttgtgagata cagccgtcta aagcaatttg ctttaagggc gcttgctagc 1080
   acacttgacg aggcagagat ctctgacctc agagatcaat ttgatgcgat tgatgtggat 1140
   aaaaatggcg tcattagtct tgaagagatg agacaggcac ttgccaaaga tcttccttgg 1200
   aaactgaaag actcacgagt tgctgagatc cttgaagcga ttgatagcaa cactgatggg 1260
   ttagtggact tcacagagtt tgtagcagca gctctacatg ttcatcaact agaagaacat 1320
   gattcagaga aatggcagct aaggtcaaga gcagcttttg agaaattcga cctagacaaa 1380
   gacgggtaca taacgcctga ggaacttcga atgcacacgg ggttaagagg atcaatagat 1440
   ccactgctgg atgaagcaga catagacaga gatgggaaaa taagcctgca tgagttcagg 1500
   agacttctaa gaacagcgag cataagttca cagagagcac caagccctgc aggtcacagg 1560
   aatcttcgat ag 1572
<210> 21
   <211> 523
   <212> PRT
   <213> Arabidopsis thaliana
<400> 21
   Met Gly Val Cys Phe Ser Ala Ile Arg Val Thr Gly Ala Ser Ser Ser
   1               5                   10                  15
   Arg Arg Ser Ser Gln Thr Lys Ser Lys Ala Ala Pro Thr Pro Ile Asp
               20                  25                  30
   Thr Lys Ala Ser Thr Lys Arg Arg Thr Gly Ser Ile Pro Cys Gly Lys
           35                  40                  45
   Arg Thr Asp Phe Gly Tyr Ser Lys Asp Phe His Asp His Tyr Thr Ile
       50                  55                  60
   Gly Lys Leu Leu Gly His Gly Gln Phe Gly Tyr Thr Tyr Val Ala Ile
   65                  70                  75                  80
   His Arg Pro Asn Gly Asp Arg Val Ala Val Lys Arg Leu Asp Lys Ser
                   85                  90                  95
   Lys Met Val Leu Pro Ile Ala Val Glu Asp Val Lys Arg Glu Val Gln
               100                 105                 110
   Ile Leu Ile Ala Leu Ser Gly His Glu Asn Val Val Gln Phe His Asn
           115                 120                 125
   Ala Phe Glu Asp Asp Asp Tyr Val Tyr Ile Val Met Glu Leu Cys Glu
       130                 135                 140
   Gly Gly Glu Leu Leu Asp Arg Ile Leu Ser Lys Lys Gly Asn Arg Tyr
   145                 150                 155                 160
   Ser Glu Lys Asp Ala Ala Val Val Val Arg Gln Met Leu Lys Val Ala
                   165                 170                 175
   Gly Glu Cys His Leu His Gly Leu Val His Arg Asp Met Lys Pro Glu
               180                 185                 190
   Asn Phe Leu Phe Lys Ser Ala Gln Leu Asp Ser Pro Leu Lys Ala Thr
           195                 200                 205
   Asp Phe Gly Leu Ser Asp Phe Ile Lys Pro Gly Lys Arg Phe His Asp
       210                 215                 220
   Ile Val Gly Ser Ala Tyr Tyr Val Ala Pro Glu Val Leu Lys Arg Arg
   225                 230                 235                 240
   Ser Gly Pro Glu Ser Asp Val Trp Ser Ile Gly Val Ile Thr Tyr Ile
                   245                 250                 255
   Leu Leu Cys Gly Arg Arg Pro Phe Trp Asp Arg Thr Glu Asp Gly Ile
               260                 265                 270
   Phe Lys Glu Val Leu Arg Asn Lys Pro Asp Phe Ser Arg Lys Pro Trp
           275                 280                 285
   Ala Thr Ile Ser Asp Ser Ala Lys Asp Phe Val Lys Lys Leu Leu Val
       290                 295                 300
   Lys Asp Pro Arg Ala Arg Leu Thr Ala Ala Gln Ala Leu Ser His Ala
   305                 310                 315                 320
   Trp Val Arg Glu Gly Gly Asn Ala Thr Asp Ile Pro Val Asp Ile Ser
                   325                 330                 335
   Val Leu Asn Asn Leu Arg Gln Phe Val Arg Tyr Ser Arg Leu Lys Gln
               340                 345                 350
   Phe Ala Leu Arg Ala Leu Ala Ser Thr Leu Asp Glu Ala Glu Ile Ser
           355                 360                 365
   Asp Leu Arg Asp Gln Phe Asp Ala Ile Asp Val Asp Lys Asn Gly Val
       370                 375                 380
   Ile Ser Leu Glu Glu Met Arg Gln Ala Leu Ala Lys Asp Leu Pro Trp
   385                 390                 395                 400
   Lys Leu Lys Asp Ser Arg Val Ala Glu Ile Leu Glu Ala Ile Asp Ser
                   405                 410                 415
   Asn Thr Asp Gly Leu Val Asp Phe Thr Glu Phe Val Ala Ala Ala Leu
               420                 425                 430
   His Val His Gln Leu Glu Glu His Asp Ser Glu Lys Trp Gln Leu Arg
           435                 440                 445
   Ser Arg Ala Ala Phe Glu Lys Phe Asp Leu Asp Lys Asp Gly Tyr Ile
       450                 455                 460
   Thr Pro Glu Glu Leu Arg Met His Thr Gly Leu Arg Gly Ser Ile Asp
   465                 470                 475                 480
   Pro Leu Leu Asp Glu Ala Asp Ile Asp Arg Asp Gly Lys Ile Ser Leu
                   485                 490                 495
   His Glu Phe Arg Arg Leu Leu Arg Thr Ala Ser Ile Ser Ser Gln Arg
               500                 505                 510
   Ala Pro Ser Pro Ala Gly His Arg Asn Leu Arg
           515                 520
<210> 22
   <211> 1569
   <212> DNA
   <213> Oryza sativa
   <400> 22
   atgggcgcgt gcttctcatc ccacactgcg accgccgccg ccgatggcgg gagcgggaag      60
   cggcagcagc ggaaggggga tcacaagggg aagctccccg atggcggcgg cggcgagaag     120
   gagaaggagg cggcgcgggt ggagttcggg tacgagaggg acttcgaggg gaggtaccag     180
   gtcgggaggc tgctcggcca cggccagttc ggctacacct tcgccgccac cgaccgggcc     240
   tccggtgacc gcgtcgccgt caagcgcatc gacaaggcca agatggttcg ccctgttgct     300
   gtggaggatg taaagagaga agtgaagatt cttaaagaac ttaaaggcca tgagaatatt     360
   gttcacttct acaatgcgtt tgaagatgac tcatatgtat atattgtgat ggaactatgt     420
   gagggtggtg aactattgga ccggattttg gcaaaaaaga acagccgtta tagtgagaaa     480
   gatgctgcag tggtggtgcg gcagatgctc aaagtggcag ctgagtgcca tctgcatggg     540
   ctagttcacc gagatatgaa gcccgagaac ttccttttca aatcaaccaa ggaggactca     600
   cctttaaagg caacagattt tggtctgtca gacttcataa aaccagggaa aaagtttcac     660
   gatatagttg gcagtgccta ttatgtagca ccagaagttt taaaacgacg gtctggccct     720
   gagtcagatg tttggagcat aggagtcata acttatattt tgctctgtgg gagacgccct     780
   ttttggaata agacagagga tggcatattc agagaggtac taagaaacaa gcctgatttt     840
   cgtaagaagc cttggccagg catcagttca ggtgctaaag atttcgttaa aaagttactt     900
   gtaaagaacc caagggcaag attaaccgct gctcaagctc tctcgcatcc atgggtaaga     960
   gaaggaggag aagcatctga gatccctgtt gatatatctg tattgtccaa catgcgtcag    1020
   tttgtcaagt acagccgttt taagcaattt gctctgaggg ctttagcaag tacactaaaa    1080
   gaggaagaac tagcagatct gaaggaccag ttcgatgcaa ttgatgttga taaaagtgga    1140
   tcaattagta ttgaggaaat gcggcatgcc cttgcaaagg atcttccttg gagattgaag    1200
   ggcccccgtg ttctcgagat tatccaagca atcgacagca acactgatgg tcttgtggac    1260
   tttgaagagt ttgtagcagc aaccctccat atacatcaaa tggctgagct tgactctgaa    1320
   aggtggggcc tacgctgcca ggctgctttc agcaaatttg atctggatgg tgacggatac    1380
   atcactccag atgaactcag aatggtgcag cacactggct tgaagggttc catcgagcca    1440
   ttgctggagg aggccgacat cgacaaagac gggagaataa gcttgtcgga gttccgcaag    1500
   ctcctgcgga cagcgagcat gagcaacctt cccagtccaa gaggacctcc aaatccacaa    1560
   cccctgtga                                                            1569
<210> 23
   <211> 522
   <212> PRT
   <213> Oryza sativa
<400> 23
   Met Gly Ala Cys Phe Ser Ser His Thr Ala Thr Ala Ala Ala Asp Gly
   1               5                   10                  15
   Gly Ser Gly Lys Arg Gln Gln Arg Lys Gly Asp His Lys Gly Lys Leu
               20                  25                  30
   Pro Asp Gly Gly Gly Gly Glu Lys Glu Lys Glu Ala Ala Arg Val Glu
           35                  40                  45
   Phe Gly Tyr Glu Arg Asp Phe Glu Gly Arg Tyr Gln Val Gly Arg Leu
       50                  55                  60
   Leu Gly His Gly Gln Phe Gly Tyr Thr Phe Ala Ala Thr Asp Arg Ala
   65                  70                  75                  80
   Ser Gly Asp Arg Val Ala Val Lys Arg Ile Asp Lys Ala Lys Met Val
                   85                  90                  95
   Arg Pro Val Ala Val Glu Asp Val Lys Arg Glu Val Lys Ile Leu Lys
               100                 105                 110
   Glu Leu Lys Gly His Glu Asn Ile Val His Phe Tyr Asn Ala Phe Glu
           115                 120                 125
   Asp Asp Ser Tyr Val Tyr Ile Val Met Glu Leu Cys Glu Gly Gly Glu
   130 135 140
   Leu Leu Asp Arg Ile Leu Ala Lys Lys Asn Ser Arg Tyr Ser Glu Lys
   145 150 155 160
   Asp Ala Ala Val Val Val Arg Gln Met Leu Lys Val Ala Ala Glu Cys
   165 170 175
   His Leu His Gly Leu Val His Arg Asp Met Lys Pro Glu Asn Phe Leu
   180 185 190
   Phe Lys Ser Thr Lys Glu Asp Ser Pro Leu Lys Ala Thr Asp Phe Gly
   195 200 205
   Leu Ser Asp Phe Ile Lys Pro Gly Lys Lys Phe His Asp Ile Val Gly
   210 215 220
   Ser Ala Tyr Tyr Val Ala Pro Glu Val Leu Lys Arg Arg Ser Gly Pro
   225 230 235 240
   Glu Ser Asp Val Trp Ser Ile Gly Val Ile Thr Tyr Ile Leu Leu Cys
   245 250 255
   Gly Arg Arg Pro Phe Trp Asn Lys Thr Glu Asp Gly Ile Phe Arg Glu
   260 265 270
   Val Leu Arg Asn Lys Pro Asp Phe Arg Lys Lys Pro Trp Pro Gly Ile
   275 280 285
   Ser Ser Gly Ala Lys Asp Phe Val Lys Lys Leu Leu Val Lys Asn Pro
   290 295 300
   Arg Ala Arg Leu Thr Ala Ala Gln Ala Leu Ser His Pro Trp Val Arg
   305 310 315 320
   Glu Gly Gly Glu Ala Ser Glu Ile Pro Val Asp Ile Ser Val Leu Ser
   325 330 335
   Asn Met Arg Gln Phe Val Lys Tyr Ser Arg Phe Lys Gln Phe Ala Leu
   340 345 350
   Arg Ala Leu Ala Ser Thr Leu Lys Glu Glu Glu Leu Ala Asp Leu Lys
   355 360 365
   Asp Gln Phe Asp Ala Ile Asp Val Asp Lys Ser Gly Ser Ile Ser Ile
   370 375 380
   Glu Glu Met Arg His Ala Leu Ala Lys Asp Leu Pro Trp Arg Leu Lys
   385                 390                 395                 400
   Gly Pro Arg Val Leu Glu Ile Ile Gln Ala Ile Asp Ser Asn Thr Asp
                   405                 410                 415
   Gly Leu Val Asp Phe Glu Glu Phe Val Ala Ala Thr Leu His Ile His
               420                 425                 430
   Gln Met Ala Glu Leu Asp Ser Glu Arg Trp Gly Leu Arg Cys Gln Ala
           435                 440                 445
   Ala Phe Ser Lys Phe Asp Leu Asp Gly Asp Gly Tyr Ile Thr Pro Asp
       450                 455                 460
   Glu Leu Arg Met Val Gln His Thr Gly Leu Lys Gly Ser Ile Glu Pro
   465                 470                 475                 480
   Leu Leu Glu Glu Ala Asp Ile Asp Lys Asp Gly Arg Ile Ser Leu Ser
                   485                 490                 495
   Glu Phe Arg Lys Leu Leu Arg Thr Ala Ser Met Ser Asn Leu Pro Ser
               500                 505                 510
   Pro Arg Gly Pro Pro Asn Pro Gln Pro Leu
           515                 520
<210> 24
   <211> 1539
   <212> DNA
   <213> Oryza sativa
<400> 24
   atgggactct gctcctcctc cagcgcccgc cgggacgccg gcacacccgg cggcggtaac     60
   ggcgcgggga acaaggataa cgcggggagg aaggggatcg tggcgtgcgg gaagcggacg    120
   gacttcgggt acgacaagga cttcgaggcg cggtacgcgc tcgggaagct gctgggccac    180
   ggccagttcg gctacacctt cgccgccgtc gaccgccgct ccagcgagcg cgtcgccgtc    240
   aagcgcatcg acaagaacaa gatggttctt cctgttgccg ttgaagacgt aaagcgagaa    300
   gttaaaatac tgaaggcctt acaaggccat gaaaatgttg tacattttta caatgcattt    360
   gaagatgata attatgtgta tattgttatg gaattatgtg aaggcgggga gttacttgac    420
   cggatactag ccaagaaaga tagccgttat agcgagaaag atgctgcagt agttgtgcgg    480
   caaatgctca aggttgctgc tgagtgccat ttgcatggtt tggttcatcg ggacatgaag    540
   cctgagaact tcctcttcaa atcaaccaaa gaggactcat ccctcaaggc tacagatttt    600
   ggtctttcag attttataag accagggaaa cactttcgtg acattgttgg aagtgcctac    660
   tatgtagcac cagaagtgct taagcgtaag tcaggcccag aatctgacgt ttggagtatt    720
   ggcgtaataa cctatattct actgtgtgga agacgacctt tctgggacaa aactgaagat    780
   ggaatattta aagaggtgtt gaaaaacaag ccagattttc gtcgcaagcc ctggccaaat    840
   atcactcctt gtgctaaaga ctttgtacaa aagttgcttg ttaaggatcc ccgtgcaaga    900
   ttaactgctg ctcaggcatt atcacatgaa tgggtgagag aaggaggaca ggcatctgat    960
   atacctctag atatatctgt attacataat atgcgtcagt ttgtaaaata cagtcggttt   1020
   aagcaatttg ctttacgggc gttagcttct acactaaatg cagaagagtt gtctgatctt   1080
   cgtgaccagt tcaatgccat tgatgttgac aagaatggaa caattagtct ggaagaactg   1140
   aagcaggctc ttgcaaagga tgttccatgg agattaaagg gtccacgtgt tttagagatt   1200
   gttgaggcaa ttgacagtaa cacagatgga ttagttgatt tcgaagagtt tgttgctgca   1260
   acattacatg tgcatcagct agtggaacat gatactgaga agtggaaatc attgtctcaa   1320
   gctgcatttg ataaatttga tgttgacgga gatggctata tcacatctga tgaactgaga   1380
   atgcaaacag gactgaaagg ttctattgat cccctcctgg aggaggctga cattgacaga   1440
   gatggaaaaa taagcctaga tgaatttcgc aggctcctga aaactgcaag catgagttca   1500
   cgcaatgtac aaactccgag gagtgttcac agatcgtag                          1539
<210> 25
   <211> 512
   <212> PRT
   <213> Oryza sativa
<400> 25
   Met Gly Leu Cys Ser Ser Ser Ser Ala Arg Arg Asp Ala Gly Thr Pro
   1               5                   10                  15
   Gly Gly Gly Asn Gly Ala Gly Asn Lys Asp Asn Ala Gly Arg Lys Gly
               20                  25                  30
   Ile Val Ala Cys Gly Lys Arg Thr Asp Phe Gly Tyr Asp Lys Asp Phe
           35                  40                  45
   Glu Ala Arg Tyr Ala Leu Gly Lys Leu Leu Gly His Gly Gln Phe Gly
       50                  55                  60
   Tyr Thr Phe Ala Ala Val Asp Arg Arg Ser Ser Glu Arg Val Ala Val
   65                  70                  75                  80
   Lys Arg Ile Asp Lys Asn Lys Met Val Leu Pro Val Ala Val Glu Asp
                   85                  90                  95
   Val Lys Arg Glu Val Lys Ile Leu Lys Ala Leu Gln Gly His Glu Asn
               100                 105                 110
   Val Val His Phe Tyr Asn Ala Phe Glu Asp Asp Asn Tyr Val Tyr Ile
           115                 120                 125
   Val Met Glu Leu Cys Glu Gly Gly Glu Leu Leu Asp Arg Ile Leu Ala
       130                 135                 140
   Lys Lys Asp Ser Arg Tyr Ser Glu Lys Asp Ala Ala Val Val Val Arg
   145                 150                 155                 160
   Gln Met Leu Lys Val Ala Ala Glu Cys His Leu His Gly Leu Val His
                   165                 170                 175
   Arg Asp Met Lys Pro Glu Asn Phe Leu Phe Lys Ser Thr Lys Glu Asp
               180                 185                 190
   Ser Ser Leu Lys Ala Thr Asp Phe Gly Leu Ser Asp Phe Ile Arg Pro
           195                 200                 205
   Gly Lys His Phe Arg Asp Ile Val Gly Ser Ala Tyr Tyr Val Ala Pro
       210                 215                 220
   Glu Val Leu Lys Arg Lys Ser Gly Pro Glu Ser Asp Val Trp Ser Ile
   225                 230                 235                 240
   Gly Val Ile Thr Tyr Ile Leu Leu Cys Gly Arg Arg Pro Phe Trp Asp
                   245                 250                 255
   Lys Thr Glu Asp Gly Ile Phe Lys Glu Val Leu Lys Asn Lys Pro Asp
               260                 265                 270
   Phe Arg Arg Lys Pro Trp Pro Asn Ile Thr Pro Cys Ala Lys Asp Phe
           275                 280                 285
   Val Gln Lys Leu Leu Val Lys Asp Pro Arg Ala Arg Leu Thr Ala Ala
       290                 295                 300
   Gln Ala Leu Ser His Glu Trp Val Arg Glu Gly Gly Gln Ala Ser Asp
   305                 310                 315                 320
   Ile Pro Leu Asp Ile Ser Val Leu His Asn Met Arg Gln Phe Val Lys
                   325                 330                 335
   Tyr Ser Arg Phe Lys Gln Phe Ala Leu Arg Ala Leu Ala Ser Thr Leu
               340                 345                 350
   Asn Ala Glu Glu Leu Ser Asp Leu Arg Asp Gln Phe Asn Ala Ile Asp
           355                 360                 365
   Val Asp Lys Asn Gly Thr Ile Ser Leu Glu Glu Leu Lys Gln Ala Leu
       370                 375                 380
   Ala Lys Asp Val Pro Trp Arg Leu Lys Gly Pro Arg Val Leu Glu Ile
   385                 390                 395                 400
   Val Glu Ala Ile Asp Ser Asn Thr Asp Gly Leu Val Asp Phe Glu Glu
                   405                 410                 415
   Phe Val Ala Ala Thr Leu His Val His Gln Leu Val Glu His Asp Thr
               420                 425                 430
   Glu Lys Trp Lys Ser Leu Ser Gln Ala Ala Phe Asp Lys Phe Asp Val
           435                 440                 445
   Asp Gly Asp Gly Tyr Ile Thr Ser Asp Glu Leu Arg Met Gln Thr Gly
       450                 455                 460
   Leu Lys Gly Ser Ile Asp Pro Leu Leu Glu Glu Ala Asp Ile Asp Arg
   465                 470                 475                 480
   Asp Gly Lys Ile Ser Leu Asp Glu Phe Arg Arg Leu Leu Lys Thr Ala
                   485                 490                 495
   Ser Met Ser Ser Arg Asn Val Gln Thr Pro Arg Ser Val His Arg Ser
               500                 505                 510
<210> 26
   <211> 1689
   <212> DNA
   <213> Glycine max
<400> 26
   atgggcctct gtttctcctc caccaaggtc agcggctcca gcagcaacaa caacaacaac 60
   aacaacgcct catccaaccg taaccgcaaa tgttcggccg cgccggcggc tgcagcgccg 120
   ccggagccgg tgacgccgca gaagaaacaa ccgtcgcagg ctcaacggcg gcgagtgcca 180
   gaggagtcgc ggaagaaccc acgcgccaaa gacaaagcgg gtgcgcgtcg gcaagggaca 240
   cgtgttccgt gcggaaagcg aacggatttc gggtacgaga aagacttcga gaatagattc 300
   tcgctcggga aattgttggg acatggacaa ttcggttaca cctacgttgg aattgacaaa 360
   aaaaatgggg accgtgtcgc ggttaagaga ctagagaaga gcaagatggt tctccccatt 420
   gcggttgagg atgttaagcg agaagtcaag atattgaaag aacttacagg ccatgaaaat 480
   gtggttcagt tctttaatgc ttttgaggat gattcatatg tgtacatagt tatggagtta 540
   tgtgagggtg gagaactgct agatcggata ttggccaaga aggacagtcg ttatactgaa 600
   aaagatgcag ctgtggttgt aaggcagatg ctaaaggttg cggctgagtg tcatttacat 660
   gggttggtac accgggacat gaaaccagag aattttcttt tcaagtcaac caaagaagat 720
   tcacctttaa aggctaccga ttttggtttg tctgatttca taaaacctgg aaagaggttt 780
   caagatattg ttggcagtgc ttactatgtt gcaccagaag tgttaaaacg taagtcaggt 840
   cccgagtcgg atgtatggag tattggtgtg attacataca tattgctttg tgggagacgc 900
   ccattttggg ataagacaga ggatggtatc ttcaaggagg tcttacggaa caagccggat 960
   ttccggcgga aaccatggcc tactataagc aatgctgcaa aagattttat gaagaaattg 1020
   ttggtaaaag atcctcgtgc gagatatgct gctgctcagg ctctttcaca tccatgggtt 1080
   agagaaggag gagaggcatt agagattcct attgatatat ctgtcctgaa caacatgcga 1140
   cagtttgtga aatatagtcg gttgaaacaa tttgcactaa gggcattggc tagcacactt 1200
   aatgaaggag agttgtctga tctaaaagat cagtttgatg caatagatgt ggacaaaaat 1260
   ggttctatta gtcttgagga gatgagacag gctcttgcta aagatcaacc ttggaagttg 1320
   aaagaatcac gtgtgctaga gatattgcaa gcgatagaca gcaacacaga tgggctagtg 1380
   gatttcaccg agtttgtggc agctacttta catgtacatc aattggagga acatgattct 1440
   gacaagtggc agcaacggtc acaggctgct tttgagaaat ttgacttgga taaggatggc 1500
   tatattactc cagatgaact tagaatgcat acgggtttga gaggctccat tgatccattg 1560
   cttgaggaag ccgatactga taaagatggg aaaatcagct taccagaatt tcgtagactt 1620
   ctaagaactg caagcatggg ttctcgaaca gtaatgagcc caagtcaccg tcatcatcga 1680
   aagatttag 1689
<210> 27
   <211> 562
   <212> PRT
   <213> Glycine max
<400> 27
   Met Gly Leu Cys Phe Ser Ser Thr Lys Val Ser Gly Ser Ser Ser Asn
   1               5                   10                  15
   Asn Asn Asn Asn Asn Asn Ala Ser Ser Asn Arg Asn Arg Lys Cys Ser
               20                  25                  30
   Ala Ala Pro Ala Ala Ala Ala Pro Pro Glu Pro Val Thr Pro Gln Lys
           35                  40                  45
   Lys Gln Pro Ser Gln Ala Gln Arg Arg Arg Val Pro Glu Glu Ser Arg
       50                  55                  60
   Lys Asn Pro Arg Ala Lys Asp Lys Ala Gly Ala Arg Arg Gln Gly Thr
   65                  70                  75                  80
   Arg Val Pro Cys Gly Lys Arg Thr Asp Phe Gly Tyr Glu Lys Asp Phe
                   85                  90                  95
   Glu Asn Arg Phe Ser Leu Gly Lys Leu Leu Gly His Gly Gln Phe Gly
               100                 105                 110
   Tyr Thr Tyr Val Gly Ile Asp Lys Lys Asn Gly Asp Arg Val Ala Val
           115                 120                 125
   Lys Arg Leu Glu Lys Ser Lys Met Val Leu Pro Ile Ala Val Glu Asp
       130                 135                 140
   Val Lys Arg Glu Val Lys Ile Leu Lys Glu Leu Thr Gly His Glu Asn
   145                 150                 155                 160
   Val Val Gln Phe Phe Asn Ala Phe Glu Asp Asp Ser Tyr Val Tyr Ile
                   165                 170                 175
   Val Met Glu Leu Cys Glu Gly Gly Glu Leu Leu Asp Arg Ile Leu Ala
               180                 185                 190
   Lys Lys Asp Ser Arg Tyr Thr Glu Lys Asp Ala Ala Val Val Val Arg
           195                 200                 205
   Gln Met Leu Lys Val Ala Ala Glu Cys His Leu His Gly Leu Val His
       210                 215                 220
   Arg Asp Met Lys Pro Glu Asn Phe Leu Phe Lys Ser Thr Lys Glu Asp
   225                 230                 235                 240
   Ser Pro Leu Lys Ala Thr Asp Phe Gly Leu Ser Asp Phe Ile Lys Pro
                   245                 250                 255
   Gly Lys Arg Phe Gln Asp Ile Val Gly Ser Ala Tyr Tyr Val Ala Pro
               260                 265                 270
   Glu Val Leu Lys Arg Lys Ser Gly Pro Glu Ser Asp Val Trp Ser Ile
           275                 280                 285
   Gly Val Ile Thr Tyr Ile Leu Leu Cys Gly Arg Arg Pro Phe Trp Asp
       290                 295                 300
   Lys Thr Glu Asp Gly Ile Phe Lys Glu Val Leu Arg Asn Lys Pro Asp
   305                 310                 315                 320
   Phe Arg Arg Lys Pro Trp Pro Thr Ile Ser Asn Ala Ala Lys Asp Phe
                   325                 330                 335
   Met Lys Lys Leu Leu Val Lys Asp Pro Arg Ala Arg Tyr Ala Ala Ala
               340                 345                 350
   Gln Ala Leu Ser His Pro Trp Val Arg Glu Gly Gly Glu Ala Leu Glu
           355                 360                 365
   Ile Pro Ile Asp Ile Ser Val Leu Asn Asn Met Arg Gln Phe Val Lys
       370                 375                 380
   Tyr Ser Arg Leu Lys Gln Phe Ala Leu Arg Ala Leu Ala Ser Thr Leu
   385                 390                 395                 400
   Asn Glu Gly Glu Leu Ser Asp Leu Lys Asp Gln Phe Asp Ala Ile Asp
                   405                 410                 415
   Val Asp Lys Asn Gly Ser Ile Ser Leu Glu Glu Met Arg Gln Ala Leu
               420                 425                 430
   Ala Lys Asp Gln Pro Trp Lys Leu Lys Glu Ser Arg Val Leu Glu Ile
           435                 440                 445
   Leu Gln Ala Ile Asp Ser Asn Thr Asp Gly Leu Val Asp Phe Thr Glu
       450                 455                 460
   Phe Val Ala Ala Thr Leu His Val His Gln Leu Glu Glu His Asp Ser
   465                 470                 475                 480
   Asp Lys Trp Gln Gln Arg Ser Gln Ala Ala Phe Glu Lys Phe Asp Leu
                   485                 490                 495
   Asp Lys Asp Gly Tyr Ile Thr Pro Asp Glu Leu Arg Met His Thr Gly
   500 505 510
   Leu Arg Gly Ser Ile Asp Pro Leu Leu Glu Glu Ala Asp Thr Asp Lys
   515 520 525
   Asp Gly Lys Ile Ser Leu Pro Glu Phe Arg Arg Leu Leu Arg Thr Ala
   530 535 540
   Ser Met Gly Ser Arg Thr Val Met Ser Pro Ser His Arg His His Arg
   545 550 555 560
   Lys Ile
<210> 28
   <211> 44
   <212> RNA
   <213> Unknown
<220>
   <223> Primer
<400> 28
   cgacuggagc acgaggacac ugacauggac ugaaggagua gaaa 44
<210> 29
   <211> 23
   <212> DNA
   <213> Unknown
<220>
   <223> Primer
<400> 29
   cgactggagc acgaggacac tga 23
<210> 30
   <211> 26
   <212> DNA
   <213> Unknown
<220>
   <223> Primer
<400> 30
   ggacactgac atggactgaa ggagta 26
<210> 31
   <211> 28
   <212> DNA
   <213> Unknown
<220>
   <223> Primer
<400> 31
   tagcaagagc ctgtctcatc tcctcaag 28
<210> 32
   <211> 28
   <212> DNA
   <213> Unknown
<220>
   <223> Primer
<400> 32
   tgctagccaa tgcccttagt gcaaattg 28

## Description

The field of this invention is the control of nematodes, in particular the control of soybean cyst nematodes. The invention also relates to the introduction of genetic material into plants that are susceptible to nematodes in order to increase resistance to nematodes.

Nematodes are microscopic roundworms that feed on the roots, leaves and stems of more than 2,000 row crops, vegetables, fruits, and ornamental plants, causing an estimated $100 billion crop loss worldwide. A variety of parasitic nematode species infect crop plants, including root-knot nematodes (RKN), cyst- and lesion-forming nematodes. Root-knot nematodes, which are characterized by causing root gall formation at feeding sites, have a relatively broad host range and are therefore pathogenic on a large number of crop species. The cyst- and lesion-forming nematode species have a more limited host range, but still cause considerable losses in susceptible crops.

Pathogenic nematodes are present throughout the United States, with the greatest concentrations occurring in the warm, humid regions of the South and West and in sandy soils. Soybean cyst nematode (*Heterodera glycines*), the most serious pest of soybean plants, was first discovered in the United States in North Carolina in 1954. Some areas are so heavily infested by soybean cyst nematode (SCN) that soybean production is no longer economically possible without control measures. Although soybean is the major economic crop attacked by SCN, SCN parasitizes some fifty hosts in total, including field crops, vegetables, ornamentals, and weeds.

Signs of nematode damage include stunting and yellowing of leaves, and wilting of the plants during hot periods. However, nematode infestation can cause significant yield losses without any obvious above-ground disease symptoms. The primary causes of yield reduction are due to root damage underground. Roots infected by SCN are dwarfed or stunted. Nematode infestation also can decrease the number of nitrogen-fixing nodules on the roots, and may make the roots more susceptible to attacks by other soil-borne plant pathogens.

The nematode life cycle has three major stages: egg, juvenile, and adult. The life cycle varies between species of nematodes. For example, the SCN life cycle can usually be completed in 24 to 30 days under optimum conditions whereas other species can take as long as a year, or longer, to complete the life cycle. When temperature and moisture levels become favorable in the spring, worm-shaped juveniles hatch from eggs in the soil. Only nematodes in the juvenile developmental stage are capable of infecting soybean roots.

The life cycle of SCN has been the subject of many studies, and as such are a useful example for understanding the nematode life cycle. After penetrating soybean roots, SCN juveniles move through the root until they contact vascular tissue, at which time they stop migrating and begin to feed. With a stylet, the nematode injects secretions that modify certain root cells and transform them into specialized feeding sites. The root cells are morphologically transformed into large multinucleate syncytia (or giant cells in the case of RKN), which are used as a source of nutrients for the nematodes. The actively feeding nematodes thus steal essential nutrients from the plant resulting in yield loss. As female nematodes feed, they swell and eventually become so large that their bodies break through the root tissue and are exposed on the surface of the root.

After a period of feeding, male SCN nematodes, which are not swollen as adults, migrate out of the root into the soil and fertilize the enlarged adult females. The males then die, while the females remain attached to the root system and continue to feed. The eggs in the swollen females begin developing, initially in a mass or egg sac outside the body, and then later within the nematode body cavity. Eventually the entire adult female body cavity is filled with eggs, and the nematode dies. It is the egg-filled body of the dead female that is referred to as the cyst. Cysts eventually dislodge and are found free in the soil. The walls of the cyst become very tough, providing excellent protection for the approximately 200 to 400 eggs contained within. SCN eggs survive within the cyst until proper hatching conditions occur. Although many of the eggs may hatch within the first year, many also will survive within the protective cysts for several years.

A nematode can move through the soil only a few inches per year on its own power. However, nematode infestation can be spread substantial distances in a variety of ways. Anything that can move infested soil is capable of spreading the infestation, including farm machinery, vehicles and tools, wind, water, animals, and farm workers. Seed sized particles of soil often contaminate harvested seed. Consequently, nematode infestation can be spread when contaminated seed from infested fields is planted in non-infested fields. There is even evidence that certain nematode species can be spread by birds. Only some of these causes can be prevented.

Traditional practices for managing nematode infestation include: maintaining proper soil nutrients and soil pH levels in nematode-infested land; controlling other plant diseases, as well as insect and weed pests; using sanitation practices such as plowing, planting, and cultivating of nematode-infested fields only after working non-infested fields; cleaning equipment thoroughly with high pressure water or steam after working in infested fields; not using seed grown on infested land for planting non-infested fields unless the seed has been properly cleaned; rotating infested fields and alternating host crops with non-host crops; using nematicides; and planting resistant plant varieties.

Methods have been proposed for the genetic transformation of plants in order to confer increased resistance to plant parasitic nematodes. U.S. Patent Nos. 5,589,622 and 5,824,876 are directed to the identification of plant genes expressed specifically in or adjacent to the feeding site of the plant after attachment by the nematode. The promoters of these plant target genes can then be used to direct the specific expression of detrimental proteins or enzymes, or the expression of antisense RNA to the target gene or to general cellular genes. The plant promoters may also be used to confer nematode resistance specifically at the feeding site by transforming the plant with a construct comprising the promoter of the plant target gene linked to a gene whose product induces lethality in the nematode after ingestion.

Recently, RNA interference (RNAi), also referred to as gene silencing, has been proposed as a method for controlling nematodes. When double-stranded RNA (dsRNA) corresponding essentially to the sequence of a target gene or mRNA is introduced into a cell, expression from the target gene is inhibited (See e.g., U.S. Patent No. 6,506,559). U.S. Patent No. 6,506,559 demonstrates the effectiveness of RNAi against known genes in *Caenorhabditis elegans*, but does not demonstrate the usefulness of RNAi for controlling plant parasitic nematodes.

Use of RNAi to target essential nematode genes has been proposed, for example, in PCT Publication WO 01/96584, WO 01/17654, US 2004/0098761, US 2005/0091713, US 2005/0188438, US 2006/0037101, US 2006/0080749, US 2007/0199100, and US 2007/0250947.

A number of models have been proposed for the action of RNAi. In mammalian systems, dsRNAs larger than 30 nucleotides trigger induction of interferon synthesis and a global shut-down of protein syntheses, in a non-sequence-specific manner. However, U.S. Patent No. 6,506,559 discloses that in nematodes, the length of the dsRNA corresponding to the target gene sequence may be at least 25, 50, 100, 200, 300, or 400 bases, and that even larger dsRNAs were also effective at inducing RNAi in *C. elegans.* It is known that when hairpin RNA constructs comprising double stranded regions ranging from 98 to 854 nucleotides were transformed into a number of plant species, the target plant genes were efficiently silenced. There is general agreement that in many organisms, including nematodes and plants, large pieces of dsRNA are cleaved into about 19-24 nucleotide fragments (siRNA) within cells, and that these siRNAs are the actual mediators of the RNAi phenomenon.

The various calcium-dependent protein kinases (CDPKs) in plants mediate a variety of responses to the environment. A specific CDPK in *Medicago truncatula* (CDPK1) was demonstrated to be necessary for the formation of symbiotic interactions between plants and *Rhizobia* and mycorrhizal fungi (see Ivashuta et al., (2005) Plant Cell 17: 2911-2921). Ivashuta *et al*. suggest that the CDPK1 is involved in the cell wall expansion and/or synthesis.

Although there have been numerous efforts to use RNAi to control plant parasitic nematodes, to date no transgenic nematode-resistant plant has been deregulated in any country. Accordingly, there continues to be a need to identify safe and effective compositions and methods for the controlling plant parasitic nematodes using RNAi, and for the production of plants having increased resistance to plant parasitic nematodes.

The present inventors have discovered that the down-regulation of calcium-dependent protein kinases (CDPKs or CDPL-like genes), exemplified by the *G. max* cDNA designated as 49806575, confers resistance to plant parasitic nematodes. This down-regulation can be accomplished using RNAi that targets such CDPK-like genes.
The invention provides the embodiments characterized in any one of claims 1 to 10.

The present invention may be understood more readily by reference to the following detailed description of the preferred embodiments of the invention and the examples included herein. Unless otherwise noted, the terms used herein are to be understood according to conventional usage by those of ordinary skill in the relevant art. In addition to the definitions of terms provided below, definitions of common terms in molecular biology may also be found in Rieger et al., 1991 Glossary of genetics: classical and molecular, 5th Ed., Berlin: Springer-Verlag; and in Current Protocols in Molecular Biology, F.M. Ausubel et al., Eds., Current Protocols, a joint venture between Greene Publishing Associates, Inc. and John Wiley & Sons, Inc., (1998 Supplement). It is to be understood that as used in the specification and in the claims, "a" or "an" can mean one or more, depending upon the context in which it is used. Thus, for example, reference to "a cell" can mean that at least one cell can be utilized. It is to be understood that the terminology used herein is for the purpose of describing specific embodiments only and is not intended to be limiting.

Throughout this application, various patent and literature publications are referenced. The disclosures of all of these publications and those references cited within those publications in their entireties are hereby incorporated by reference into this application in order to more fully describe the state of the art to which this invention pertains.

A plant "CDPK-like gene" is defined herein as a gene having at least 70% sequence identity to the 49806575 polynucleotide having the sequence set forth in SEQ ID NO:1, which is the *G. max* CDPK-like gene. In accordance with the invention, CDPK-like genes include genes having sequences such as those set forth in SEQ ID NO: 2 which are homologs of the *G. max* CDPK-like gene. The CDPK-like genes defined herein encode polypeptides having at least 70% sequence identity to the *G. max* CDKP-like partial polypeptide having the sequence as set forth in SEQ ID NO:3. Such polypeptides include CDPK-like polypeptides having the sequences as set forth in SEQ ID NOs:7, 9, 11, 13, 15, 17, 19, 21, 23, 25 and 27.

Additional CDPK-like genes (CDPK-like gene homologs) may be isolated from plants other than soybean using the information provided herein and techniques known to those of skill in the art of biotechnology. For example, a nucleic acid molecule from a plant that hybridizes under stringent conditions to the nucleic acid of SEQ ID NO:1 can be isolated from plant tissue cDNA libraries. Alternatively, mRNA can be isolated from plant cells (e.g., by the guanidinium-thiocyanate extraction procedure of Chirgwin et al., 1979, Biochemistry 18:5294-5299), and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon the nucleotide sequence shown in SEQ ID NO:1. Additional oligonucleotide primers may be designed that are based on the sequences of the CDPK-like genes having the sequences as set forth in SEQ ID NO: 2. Nucleic acid molecules corresponding to the CDPK-like target genes defined herein can be amplified using cDNA or, alternatively, genomic DNA, as a template and appropriate oligonucleotide primers according to standard PCR amplification techniques. The nucleic acid molecules so amplified can be cloned into appropriate vectors and characterized by DNA sequence analysis.

As used herein, "RNAi" or "RNA interference" refers to the process of sequence-specific post-transcriptional gene silencing in plants, mediated by double-stranded RNA (dsRNA). As used herein, "dsRNA" refers to RNA that is partially or completely double stranded. Double stranded RNA is also referred to as small or short interfering RNA (siRNA), short interfering nucleic acid (siNA), short interfering RNA, micro-RNA (miRNA), and the like. In the RNAi process, dsRNA comprising a first strand that is substantially identical to a portion of a target gene e.g. a CDPK-like gene and a second strand that is complementary to the first strand is introduced into a plant. After introduction into the plant, the target gene-specific dsRNA is processed into relatively small fragments (siRNAs) and can subsequently become distributed throughout the plant, leading to a loss-of-function mutation having a phenotype that, over the period of a generation, may come to closely resemble the phenotype arising from a complete or partial deletion of the target gene. Alternatively, the target gene-specific dsRNA is operably associated with a regulatory element or promoter that results in expression of the dsRNA in a tissue, temporal, spatial or inducible manner and may further be processed into relatively small fragments by a plant cell containing the RNAi processing machinery, and the loss-of-function phenotype is obtained. Also, the regulatory element or promoter may direct expression preferentially to the roots or syncytia or giant cell where the dsRNA may be expressed either constitutively in those tissues or upon induction by the feeding of the nematode or juvenile nematode, such as J2 nematodes.

As used herein, taking into consideration the substitution of uracil for thymine when comparing RNA and DNA sequences, the term "substantially identical" as applied to dsRNA means that the nucleotide sequence of one strand of the dsRNA is at least about 80%-90% identical to 20 or more contiguous nucleotides of the target gene, more preferably, at least about 90-95% identical to 20 or more contiguous nucleotides of the target gene, and most preferably at least about 95%, 96%, 97%, 98% or 99% identical or absolutely identical to 20 or more contiguous nucleotides of the target gene. 20 or more nucleotides means a portion, being at least about 20, 21, 22, 23, 24, 25, 50, 100, 200, 300, 400, 500, 1000, 1500, consecutive bases or up to the full length of the target gene.

As used herein, "complementary" polynucleotides are those that are capable of base pairing according to the standard Watson-Crick complementarity rules. Specifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. As used herein, the term "substantially complementary" means that two nucleic acid sequences are complementary over at least at 80% of their nucleotides. Preferably, the two nucleic acid sequences are complementary over at least at 85%, 90%, 95%, 96%, 97%, 98%, 99% or more or all of their nucleotides. Alternatively, "substantially complementary" means that two nucleic acid sequences can hybridize under high stringency conditions. As used herein, the term "substantially identical" or "corresponding to" means that two nucleic acid sequences have at least 80% sequence identity. Preferably, the two nucleic acid sequences have at least 85%, 90%, 95%, 96%, 97%, 98%, 99% or 100% of sequence identity.

Also as used herein, the terms "nucleic acid" and "polynucleotide" refer to RNA or DNA that is linear or branched, single or double stranded, or a hybrid thereof. The term also encompasses RNA/DNA hybrids. When dsRNA is produced synthetically, less common bases, such as inosine, 5-methylcytosine, 6-methyladenine, hypoxanthine and others can also be used for antisense, dsRNA, and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made.

As used herein, the term "control," when used in the context of an infection, refers to the reduction or prevention of an infection. Reducing or preventing an infection by a nematode will cause a plant to have increased resistance to the nematode, however, such increased resistance does not imply that the plant necessarily has 100% resistance to infection. In preferred embodiments, the resistance to infection by a nematode in a resistant plant is greater than 10%, 20%, 30%, 40%, 50%, 60%, 70%, 80%, 90%, or 95% in comparison to a wild type plant that is not resistant to nematodes. Preferably the wild type plant is a plant of a similar, more preferably identical genotype as the plant having increased resistance to the nematode, but does not comprise a dsRNA directed to the target gene. The plant's resistance to infection by the nematode may be due to the death, sterility, arrest in development, or impaired mobility of the nematode upon exposure to the plant comprising dsRNA specific to a gene essential for development or maintenance of a functional feeding site, syncytia, or giant cell. The term "resistant to nematode infection" or "a plant having nematode resistance" as used herein refers to the ability of a plant, as compared to a wild type plant, to avoid infection by nematodes, to kill nematodes or to hamper, reduce or stop the development, growth or multiplication of nematodes. This might be achieved by an active process, e.g. by producing a substance detrimental to the nematode, or by a passive process, like having a reduced nutritional value for the nematode or not developing structures induced by the nematode feeding site like syncytia or giant cells. The level of nematode resistance of a plant can be determined in various ways, e.g. by counting the nematodes being able to establish parasitism on that plant, or measuring development times of nematodes, proportion of male and female nematodes or, for cyst nematodes, counting the number of cysts or nematode eggs produced on roots of an infected plant or plant assay system.

The term "plant" is intended to encompass plants at any stage of maturity or development, as well as any tissues or organs (plant parts) taken or derived from any such plant unless otherwise clearly indicated by context. Plant parts include, but are not limited to, stems, roots, flowers, ovules, stamens, seeds, leaves, embryos, meristematic regions, callus tissue, anther cultures, gametophytes, sporophytes, pollen, microspores, protoplasts, hairy root cultures, rooted explant cultures and the like. The present invention also includes seeds produced by the plants of the present invention. In one embodiment, the seeds are true breeding for an increased resistance to nematode infection as compared to a wild-type variety of the plant seed. As used herein, a "plant cell" includes, but is not limited to, a protoplast, gamete producing cell, and a cell that regenerates into a whole plant. Tissue culture of various tissues of plants and regeneration of plants therefrom is well known in the art and is widely published.

As used herein, the term "transgenic" refers to any plant, plant cell, callus, plant tissue, or plant part that contains all or part of at least one recombinant polynucleotide. In many cases, all or part of the recombinant polynucleotide is stably integrated into a chromosome or stable extra-chromosomal element, so that it is passed on to successive generations. For the purposes of the invention, the term "recombinant polynucleotide" refers to a polynucleotide that has been altered, rearranged, or modified by genetic engineering. Examples include any cloned polynucleotide, or polynucleotides, that are linked or joined to heterologous sequences. The term "recombinant" does not refer to alterations of polynucleotides that result from naturally occurring events, such as spontaneous mutations, or from non-spontaneous mutagenesis followed by selective breeding.

As used herein, the term "amount sufficient to inhibit expression" refers to a concentration or amount of the dsRNA that is sufficient to reduce levels or stability of mRNA or protein produced from a target gene in a plant. As used herein, "inhibiting expression" refers to the absence or observable decrease in the level of protein and/or mRNA product from a target gene. Inhibition of target gene expression may be lethal to the parasitic nematode either directly or indirectly through modification or eradication of the feeding site, syncytia, or giant cell, or such inhibition may delay or prevent entry into a particular developmental step (e.g., metamorphosis), if access to a fully functional feeding site, syncytia, or giant cell is associated with a particular stage of the parasitic nematode's life cycle. The consequences of inhibition can be confirmed by examination of the plant root for reduction or elimination of cysts or other properties of the nematode or nematode infestation (as presented below in Example 2).

In accordance with the invention, a plant is transformed with a nucleic acid or a dsRNA, which specifically inhibits expression of a target gene (CDPK-like gene) in the plant that is essential for the development or maintenance of a feeding site, syncytia, or giant cell; ultimately affecting the survival, metamorphosis, or reproduction of the nematode. In one embodiment, the dsRNA is encoded by a vector that has been transformed into an ancestor of the infected plant. Preferably, the nucleic acid sequence expressing said dsRNA is under the transcriptional control of a root specific promoter or a parasitic nematode feeding cell-specific promoter or a nematode inducible promoter.

Accordingly, the dsRNA referred to in accordance with the invention comprises a first strand is substantially identical to a portion of a CDPK-like gene such as the soybean 49806575 cDNA, and a second strand that is substantially complementary to the first strand. The target gene is selected from the group consisting of: (a) a polynucleotide having the sequence set forth in SEQ ID NO: 1, or 2; (b) a polynucleotide having at least 70% sequence identity to SEQ ID NO: 1, or 2. The length of the substantially identical double-stranded nucleotide sequences may be at least about 19, 20, 21, 22, 23, 24, 25, 50, 100, 200, 300, 400, 500, 1000, 1500, consecutive bases or up to the whole length of the CDPK-like gene. In a preferred embodiment, the length of the double-stranded nucleotide sequence is from about 19 to about 200-500 consecutive nucleotides in length. In another preferred embodiment, the dsRNA is substantially identical or is identical to bases 1 to 320 of SEQ ID NO: 2.

As discussed above, fragments of dsRNA larger than about 19-24 nucleotides in length are cleaved intracellularly by nematodes and plants to siRNAs of about 19-24 nucleotides in length, and these siRNAs are the actual mediators of the RNAi phenomenon. The table in Figures 51-5g sets forth exemplary 21-mers of the CDPK-like genes defined herein. This table can also be used to calculate the 19, 20, 22, 23 or 24-mers by adding or subtracting the appropriate number of nucleotides from each 21-mer. Thus the dsRNA may range in length from about 19 nucleotides to about 320 nucleotides. Preferably, the dsRNA of the invention has a length from about 21 nucleotides to about 600 nucleotides. More preferably, the dsRNA has a length from about 21 nucleotides to about 500 nucleotides, or from about 21 nucleotides to about 400 nucleotides.

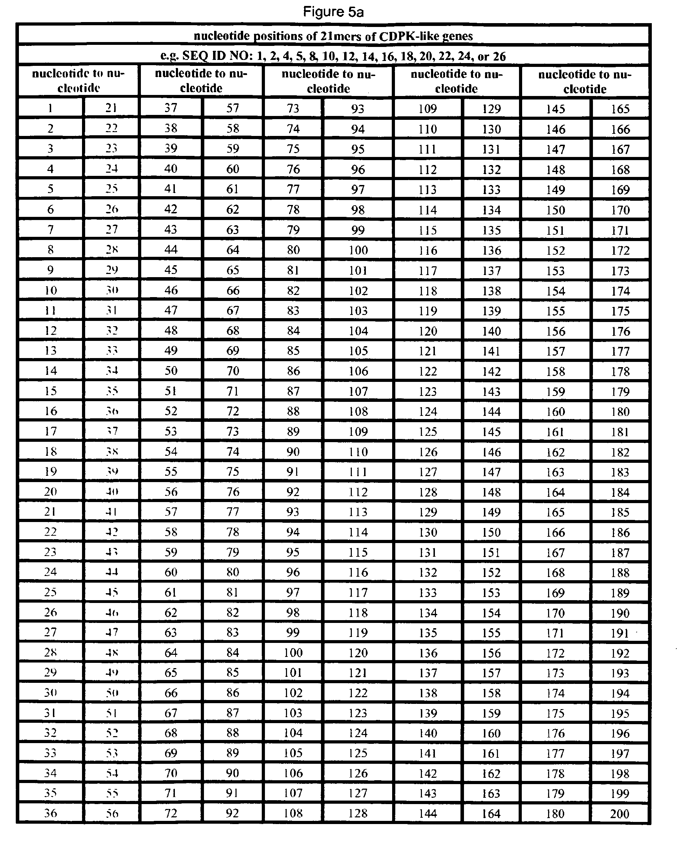

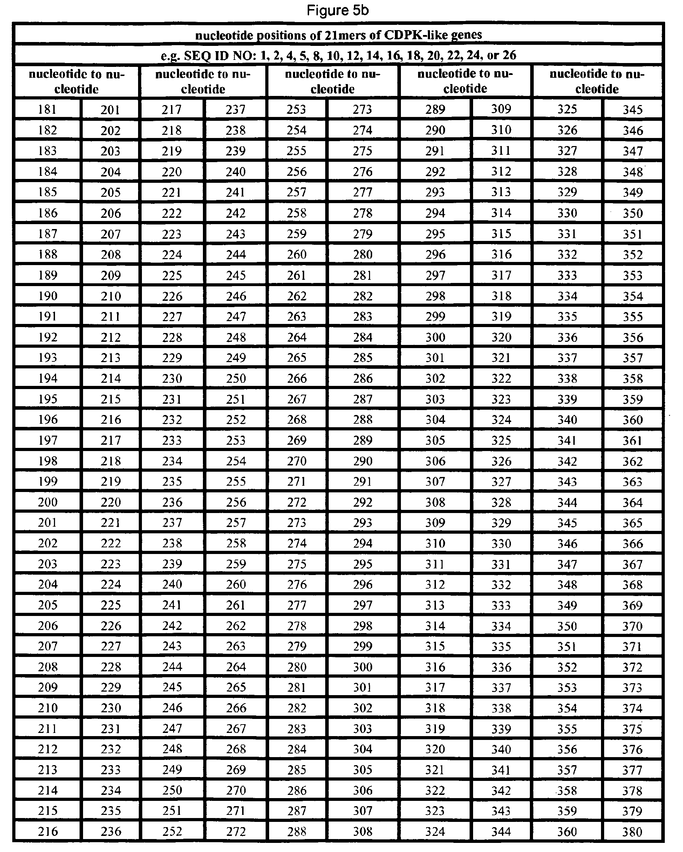

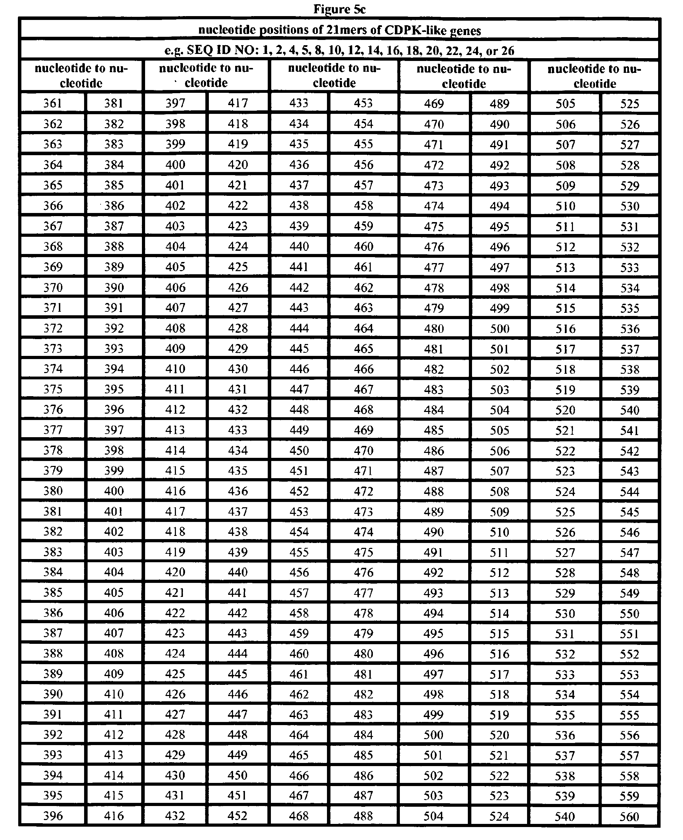

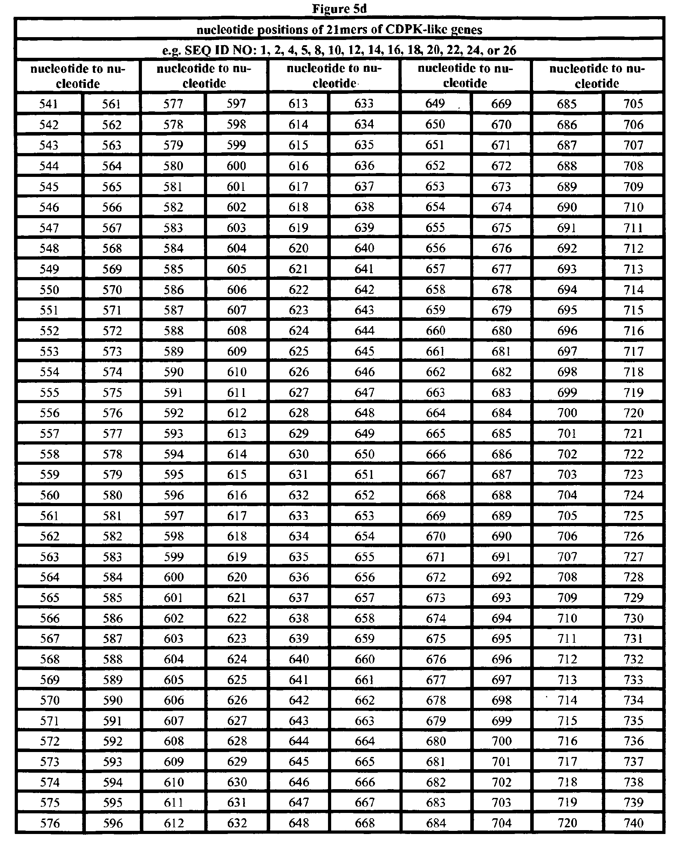

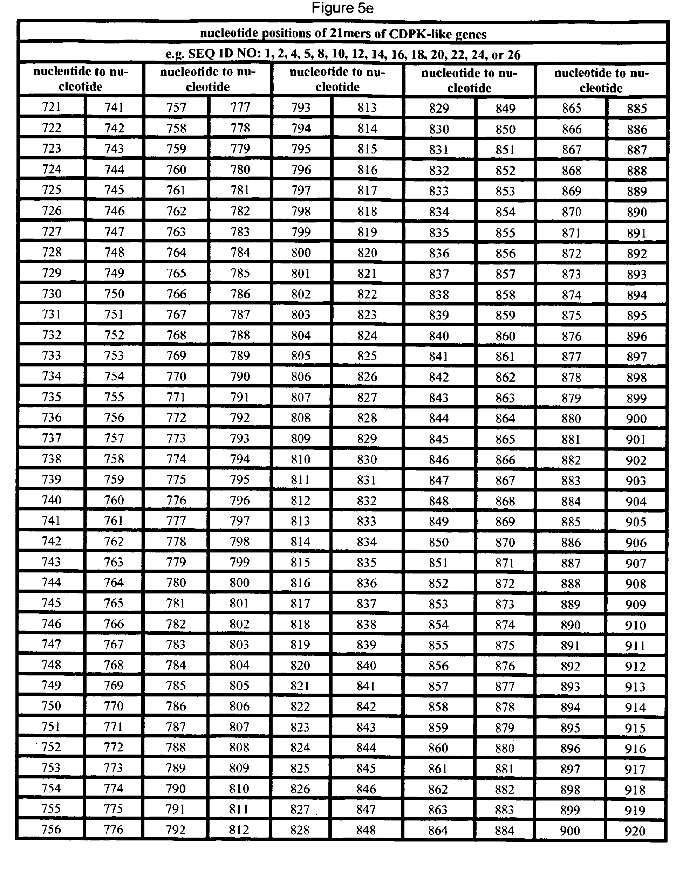

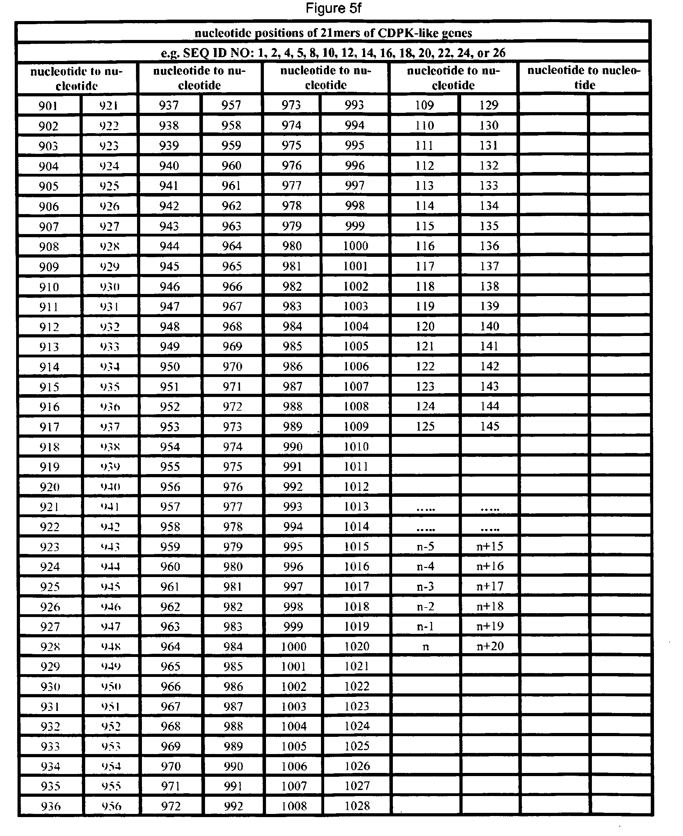

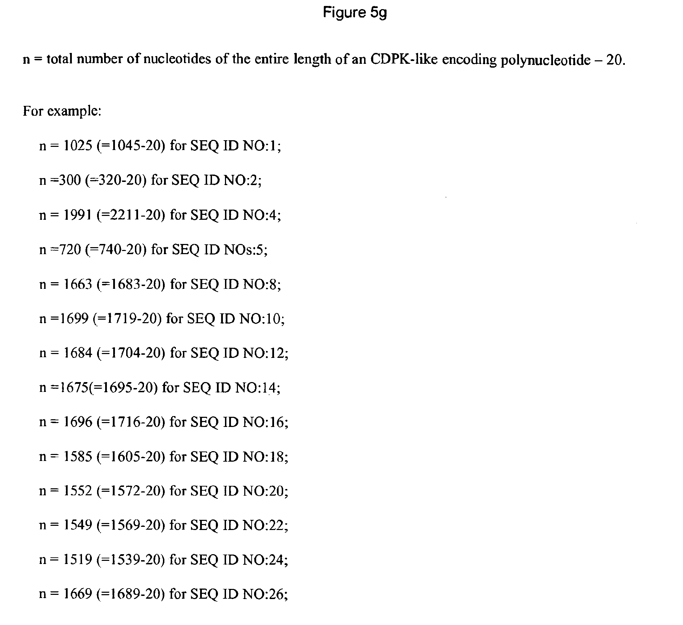

As disclosed herein, 100% sequence identity between the dsRNA and the target gene is not required to practice the present invention. While a dsRNA comprising a nucleotide sequence identical to a portion of the CDPK-like gene is preferred for inhibition, the invention can tolerate sequence variations that might be expected due to gene manipulation or synthesis, genetic mutation, strain polymorphism, or evolutionary divergence. Thus the dsRNAs of the invention also encompass dsRNAs comprising a mismatch with the target gene of at least 1, 2, or more nucleotides. For example, it is contemplated in the present invention that the 21 mer dsRNA sequences exemplified in Figures 5a-5g may contain an addition, deletion or substitution of 1, 2, or more nucleotides, so long as the resulting sequence still interferes with the CDPK-like gene function.

Sequence identity between the dsRNAs of the invention and the CDPK-like target genes may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 80 % sequence identity, 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript under stringent conditions (e.g., 400 mM NaCl, 40 mM PIPES pH 6.4, 1 mM EDTA, 60°C hybridization for 12-16 hours; followed by washing at 65°C with 0.1%SDS and 0.1 % SSC for about 15-60 minutes).

When dsRNA has a length longer than about 21 nucleotides, for example from about 50 nucleotides to about 1000 nucleotides, it will be cleaved randomly to dsRNAs of about 21 nucleotides within the plant or parasitic nematode cell, the siRNAs. The cleavage of a longer dsRNA of the invention will yield a pool of about 21mer dsRNAs (ranging from about 19mers to about 24mers), derived from the longer dsRNA. This pool of about 21 mer dsRNAs is also encompassed within the scope of the present invention, whether generated intracellularly within the plant or nematode or synthetically using known methods of oligonucleotide synthesis.

The siRNAs applied according to the invention have sequences corresponding to fragments of about 19-24 contiguous nucleotides across the entire sequence of the CDPK-like target gene. For example, a pool of such siRNA derived from the CDPK-like gene as set forth in SEQ ID NO:1, or 2, may comprise a multiplicity of RNA molecules which are selected from the group consisting of oligonucleotides substantially identical to the 21mer nucleotides of SEQ ID NO:1, or 2, found in Figures 5a-5g. A pool of siRNA derived from the CDPK-like target gene of SEQ ID NO:1 may also comprise any combination of the specific RNA molecules having any of the 21 contiguous nucleotide sequences derived from SEQ ID NO:1 set forth in Figures 5a-5g. Further, as noted above, multiple specialized Dicers in plants generate siRNAs typically ranging in size from 19nt to 24nt (See Henderson et al., 2006. Nature Genetics 38:721-725.). The siRNAs can range from about 19 contiguous nucleotide sequences to about 24 contiguous nucleotide sequences. Similarly, a pool of siRNA may comprise a multiplicity of RNA molecules having any of about 19, 20, 21, 22, 23, or 24 contiguous nucleotide sequences derived from SEQ ID NO: 1, or 2. Alternatively, the pool of siRNA may comprise a multiplicity of RNA molecules having a combination of any of about 19, 20, 21, 22, 23, and/or 24 contiguous nucleotide sequences derived from SEQ ID NO: 1.

The dsRNA referred to according to this invention may optionally comprise a single stranded overhang at either or both ends. The double-stranded structure may be formed by a single self-complementary RNA strand (i.e. forming a hairpin loop) or two complementary RNA strands. RNA duplex formation may be initiated either inside or outside the cell. When the dsRNA forms a hairpin loop, it may optionally comprise an intron, as set forth in US 2003/0180945A1 or a nucleotide spacer, which is a stretch of sequence between the complementary RNA strands to stabilize the hairpin transgene in cells. Methods for making various dsRNA molecules are set forth, for example, in WO 99/53050 and in U.S. Pat. No. 6,506,559. The RNA may be introduced in an amount that allows delivery of at least one copy per cell. Higher doses of double-stranded material may yield more effective inhibition.

Disclosed herein is also an isolated recombinant expression vector comprising a nucleic acid encoding a dsRNA molecule as described above, wherein expression of the vector in a host plant cell results in increased resistance to a parasitic nematode as compared to a wild-type variety of the host plant cell. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host plant cell into which they are introduced. Other vectors are integrated into the genome of a host plant cell upon introduction into the host cell, and thereby are replicated along with the host genome. Moreover, certain vectors are capable of directing the expression of genes to which they are operatively linked. Such vectors are referred to herein as "expression vectors." In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., potato virus X, tobacco rattle virus, and Geminivirus), which serve equivalent functions.

The recombinant expression vectors comprise a nucleic acid of the invention in a form suitable for expression of the nucleic acid in a host plant cell, which means that the recombinant expression vector includes one or more regulatory sequences, e.g. promoters, selected on the basis of the host plant cells to be used for expression, which is operatively linked to the nucleic acid sequence to be expressed. With respect to a recombinant expression vector, the terms "operatively linked" and "in operative association" are interchangeable and are intended to mean that the nucleotide sequence of interest is linked to the regulatory sequence(s) in a manner which allows for expression of the nucleotide sequence (e.g., in a host plant cell when the vector is introduced into the host plant cell). The term "regulatory sequence" is intended to include promoters, enhancers, and other expression control elements (e.g., polyadenylation signals). Such regulatory sequences are described, for example, in Goeddel, Gene Expression Technology: Methods in Enzymology 185, Academic Press, San Diego, CA (1990) and Gruber and Crosby, in: Methods in Plant Molecular Biology and Biotechnology, Eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the expression vector can depend on such factors as the choice of the host cell to be transformed, the level of expression of dsRNA desired, etc. The expression vectors can be introduced into plant host cells to thereby produce dsRNA molecules referred to in accordance with the invention encoded by nucleic acids as described herein.

The recombinant expression vector comprises a regulatory sequence operatively linked to a nucleotide sequence that is a template for one or both strands of the dsRNA molecules. The nucleic acid molecule further can comprise a promoter flanking either end of the nucleic acid molecule, wherein the promoters drive expression of each individual DNA strand, thereby generating two complementary RNAs that hybridize and form the dsRNA. The nucleic acid molecule may also comprise a nucleotide sequence that is transcribed into both strands of the dsRNA on one transcription unit, wherein the sense strand is transcribed from the 5' end of the transcription unit and the antisense strand is transcribed from the 3' end, wherein the two strands are separated by about 3 to about 500 base pairs or more, and wherein after transcription, the RNA transcript folds on itself to form a hairpin. The invention, the spacer region in the hairpin transcript may be any DNA fragment.

According to the present invention, the introduced polynucleotide may be maintained in the plant cell stably if it is incorporated into a non-chromosomal autonomous replicon or integrated into the plant chromosomes. Alternatively, the introduced polynucleotide may be present on an extra-chromosomal non-replicating vector and be transiently expressed or transiently active. Whether present in an extra-chromosomal non-replicating vector or a vector that is integrated into a chromosome, the polynucleotide preferably resides in a plant expression cassette. A plant expression cassette preferably contains regulatory sequences capable of driving gene expression in plant cells that are operatively linked so that each sequence can fulfill its function, for example, termination of transcription by polyadenylation signals. Preferred polyadenylation signals are those originating from *Agrobacterium tumefaciens* t-DNA such as the gene 3 known as octopine synthase of the Ti-plasmid pTiACH5 (Gielen et al., 1984, EMBO J. 3:835) or functional equivalents thereof, but also all other terminators functionally active in plants are suitable. As plant gene expression is very often not limited on transcriptional levels, a plant expression cassette preferably contains other operatively linked sequences like translational enhancers such as the overdrive-sequence containing the 5'-untranslated leader sequence from tobacco mosaic virus enhancing the polypeptide per RNA ratio (Gallie et al., 1987, Nucl. Acids Research 15:8693-8711). Examples of plant expression vectors include those detailed in: Becker, D. et al., 1992, New plant binary vectors with selectable markers located proximal to the left border, Plant Mol. Biol. 20:1195-1197; Bevan, M.W., 1984, Binary Agrobacterium vectors for plant transformation, Nucl. Acid. Res. 12:8711-8721; and Vectors for Gene Transfer in Higher Plants; in: Transgenic Plants, Vol. 1, Engineering and Utilization, eds.: Kung and R. Wu, Academic Press, 1993, S. 15-38.

Plant gene expression should be operatively linked to an appropriate promoter conferring gene expression in a temporal-preferred, spatial-preferred, cell type-preferred, and/or tissue-preferred manner. Promoters useful in the expression cassettes of the invention include any promoter that is capable of initiating transcription in a plant cell present in the plant's roots. Such promoters include, but are not limited to those that can be obtained from plants, plant viruses and bacteria that contain genes that are expressed in plants, such as *Agrobacterium* and *Rhizobium.* Preferably, the expression cassette of the invention comprises a root-specific promoter, a pathogen inducible promoter, or a nematode inducible promoter. More preferably the nematode inducible promoter is a parasitic nematode feeding cell-specific promoter. A parasitic nematode feeding site-specific promoter may be specific for syncytial cells or giant cells or specific for both kinds of cells. A promoter is inducible, if its activity, measured on the amount of RNA produced under control of the promoter, is at least 30%, 40%, 50% preferably at least 60%, 70%, 80%, 90% more preferred at least 100%, 200%, 300% higher in its induced state, than in its un-induced state. A promoter is cell-, tissue- or organ-specific, if its activity, measured on the amount of RNA produced under control of the promoter, is at least 30%, 40%, 50% preferably at least 60%, 70%, 80%, 90% more preferred at least 100%, 200%, 300% higher in a particular cell-type, tissue or organ, then in other cell-types or tissues of the same plant, preferably the other cell-types or tissues are cell types or tissues of the same plant organ, e.g. a root. In the case of organ specific promoters, the promoter activity has to be compared to the promoter activity in other plant organs, e.g. leafs, stems, flowers or seeds.

The promoter may be constitutive, inducible, developmental stage-preferred, cell type-preferred, tissue-preferred or organ-preferred. Constitutive promoters are active under most conditions. Non-limiting examples of constitutive promoters include the CaMV 19S and 35S promoters (Odell et al., 1985, Nature 313:810-812), the sX CaMV 35S promoter (Kay et al., 1987, Science 236:1299-1302), the Sep1 promoter, the rice actin promoter (McElroy et al., 1990, Plant Cell 2:163-171), the *Arabidopsis* actin promoter, the ubiquitin promoter (Christensen et al., 1989, Plant Molec. Biol. 18:675-689); pEmu (Last et al., 1991, Theor. Appl. Genet. 81:581-588), the figwort mosaic virus 35S promoter, the Smas promoter (Velten et al., 1984, EMBO J. 3:2723-2730), the GRP1-8 promoter, the cinnamyl alcohol dehydrogenase promoter (U.S. Patent No. 5,683,439), promoters from the T-DNA of *Agrobacterium,* such as mannopine synthase, nopaline synthase, and octopine synthase, the small subunit of ribulose biphosphate carboxylase (ssuRUBISCO) promoter, and the like. Promoters that express the dsRNA in a cell that is contacted by parasitic nematodes are preferred. Alternatively, the promoter may drive expression of the dsRNA in a plant tissue remote from the site of contact with the nematode, and the dsRNA may then be transported by the plant to a cell that is contacted by the parasitic nematode, in particular cells of, or close by nematode feeding sites, e.g. syncytial cells or giant cells.

Inducible promoters are active under certain environmental conditions, such as the presence or absence of a nutrient or metabolite, heat or cold, light, pathogen attack, anaerobic conditions, and the like. For example, the promoters TobRB7, AtRPE, AtPyk10, Gemini19, and AtHMG1 have been shown to be induced by nematodes (for a review of nematode-inducible promoters, see Ann. Rev. Phytopathol. (2002) 40:191-219; see also U.S. Pat. No. 6,593,513). Method for isolating additional promoters, which are inducible by nematodes are set forth in U.S. Pat. Nos. 5,589,622 and 5,824,876. Other inducible promoters include the hsp80 promoter from *Brassica,* being inducible by heat shock; the PPDK promoter is induced by light; the PR-1 promoter from tobacco, *Arabidopsis,* and maize are inducible by infection with a pathogen; and the Adh1 promoter is induced by hypoxia and cold stress. Plant gene expression can also be facilitated via an inducible promoter (For review, see Gatz, 1997, Annu. Rev. Plant Physiol. Plant Mol. Biol. 48:89-108). Chemically inducible promoters are especially suitable if time-specific gene expression is desired. Non-limiting examples of such promoters are a salicylic acid inducible promoter (PCT Application No. WO 95/19443), a tetracycline inducible promoter (Gatz et al., 1992, Plant J. 2:397-404) and an ethanol inducible promoter (PCT Application No. WO 93/21334).

Developmental stage-preferred promoters are preferentially expressed at certain stages of development. Tissue and organ preferred promoters include, but are not limited to, those that are preferentially expressed in certain tissues or organs, such as leaves, roots, seeds, or xylem. Examples of tissue preferred and organ preferred promoters include, but are not limited to fruit-preferred, ovule-preferred, male tissue-preferred, seed-preferred, integument-preferred, tuber-preferred, stalk-preferred, pericarp-preferred, and leaf-preferred, stigma-preferred, pollen-preferred, anther-preferred, a petal-preferred, sepal-preferred, pedicel-preferred, silique-preferred, stem-preferred, root-preferred promoters and the like. Seed preferred promoters are preferentially expressed during seed development and/or germination. For example, seed preferred promoters can be embryo-preferred, endosperm preferred and seed coat-preferred. See Thompson *et al.,* 1989, BioEssays 10:108. Examples of seed preferred promoters include, but are not limited to cellulose synthase (celA), Cim1, gamma-zein, globulin-1, maize 19 kD zein (cZ19B1) and the like.

Other suitable tissue-preferred or organ-preferred promoters include the napin-gene promoter from rapeseed (U.S. Patent No. 5,608,152), the USP-promoter from *Vicia faba* (Baeumlein et al., 1991, Mol Gen Genet. 225(3):459-67), the oleosin-promoter from *Arabidopsis* (PCT Application No. WO 98/45461), the phaseolin-promoter from *Phaseolus vulgaris* (U.S. Patent No. 5,504,200), the Bce4-promoter from Brassica (PCT Application No. WO 91/13980), or the legumin B4 promoter (LeB4; Baeumlein et al., 1992, Plant Journal, 2(2):233-9), as well as promoters conferring seed specific expression in monocot plants like maize, barley, wheat, rye, rice, etc. Suitable promoters to note are the Ipt2 or Ipt1-gene promoter from barley (PCT Application No. WO 95/15389 and PCT Application No. WO 95/23230) or those described in PCT Application No. WO 99/16890 (promoters from the barley hordein-gene, rice glutelin gene, rice oryzin gene, rice prolamin gene, wheat gliadin gene, wheat glutelin gene, oat glutelin gene, Sorghum kasirin-gene, and rye secalin gene).

Other promoters useful in the expression cassettes of the invention include, but are not limited to, the major chlorophyll a/b binding protein promoter, histone promoters, the Ap3 promoter, the β-conglycin promoter, the napin promoter, the soybean lectin promoter, the maize 15kD zein promoter, the 22kD zein promoter, the 27kD zein promoter, the g-zein promoter, the waxy, shrunken 1, shrunken 2, and bronze promoters, the Zm13 promoter (U.S. Patent No. 5,086,169), the maize polygalacturonase promoters (PG) (U.S. Patent Nos. 5,412,085 and 5,545,546), and the SGB6 promoter (U.S. Patent No. 5,470,359), as well as synthetic or other natural promoters.

In accordance with the present invention, the expression cassette comprises an expression control sequence operatively linked to a nucleotide sequence that is a template for one or both strands of the dsRNA. The dsRNA template comprises (a) a first stand having a sequence substantially identical to from about 19 to about 500, or up to the full length, consecutive nucleotides of SEQ ID NO: 1, or 2; and (b) a second strand having a sequence substantially complementary to the first strand. In further embodiments, a promoter flanks either end of the template nucleotide sequence, wherein the promoters drive expression of each individual DNA strand, thereby generating two complementary RNAs that hybridize and form the dsRNA. In alternative embodiments, the nucleotide sequence is transcribed into both strands of the dsRNA on one transcription unit, wherein the sense strand is transcribed from the 5' end of the transcription unit and the anti-sense strand is transcribed from the 3' end, wherein the two strands are separated by about 3 to about 500 base pairs, and wherein after transcription, the RNA transcript folds on itself to form a hairpin.

In another embodiment, the vector contains a bidirectional promoter, driving expression of two nucleic acid molecules, whereby one nucleic acid molecule codes for the sequence substantially identical to a portion of a CDPK-like gene and the other nucleic acid molecule codes for a second sequence being substantially complementary to the first strand and capable of forming a dsRNA, when both sequences are transcribed.. A bidirectional promoter is a promoter capable of mediating expression in two directions.

In another embodiment, the vector contains two promoters one mediating transcription of the sequence substantially identical to a portion of a CDPK-like gene and another promoter mediating transcription of a second sequence being substantially complementary to the first strand and capable of forming a dsRNA, when both sequences are transcribed. The second promoter might be a different promoter. A different promoter means a promoter having a different activity in regard to cell or tissue specificity, or showing expression on different inducers for example, pathogens, abiotic stress or chemicals. For example, one promoter might by constitutive or tissue specific and another might be tissue specific or inducible by pathogens. In one embodiment one promoter mediates the transcription of one nucleic acid molecule suitable for over-expression of a CDPK-like gene, while another promoter mediates tissue- or cell-specific transcription or pathogen inducible expression of the complementary nucleic acid

The invention is also embodied in a transgenic plant capable of expressing the dsRNA of the invention and thereby inhibiting the CDPK-like genes in the roots, feeding site, syncytia and/or giant cell. The plant or transgenic plant may be any plant, such like, but not limited to trees, cut flowers, ornamentals, vegetables or crop plants. The plant may be from a genus selected from the group consisting of *Medicago*, *Lycopersicon*, *Brassica*, *Cucumis*, *Solanum, Juglans, Gossypium, Malus, Vitis, Antirrhinum, Populus, Fragaria, Arabidopsis, Picea, Capsicum, Chenopodium, Dendranthema, Pharbitis, Pinus, Pisum, Oryza, Zea, Triticum, Triticale, Secale, Lolium, Hordeum, Glycine, Pseudotsuga, Kalanchoe, Beta, Helianthus, Nicotiana, Cucurbita, Rosa, Fragaria, Lotus, Medicago, Onobrychis, trifolium, Trigonella, Vigna, Citrus, Linum, Geranium, Manihot, Daucus, Raphanus, Sinapis, Atropa, Datura, Hyoscyamus, Nicotiana, Petunia, Digitalis, Majorana, Ciahorium, Lactuca, Bromus, Asparagus, Antirrhinum, Heterocallis, Nemesis, Pelargonium, Panieum, Pennisetum, Ranunculus, Senecio, Salpiglossis*, *Browaalia*, *Phaseolus*, *Avena*, and *Allium*, *or* the plant may be selected from a genus selected from the group consisting of Arabidopsis, *Medicago*, *Lycopersicon*, *Brassica*, *Cucumis*, *Solanum, Juglans, Gossypium, Malus, Vitis*, *Antirrhinum, Brachipodium, Populus, Fragaria, Arabidopsis, Picea, Capsicum, Chenopodium, Dendranthema, Pharbitis, Pinus, Pisum, Oryza, Zea, Triticum, Triticale, Secale, Lolium, Hordeum, Glycine, Pseudotsuga, Kalanchoe, Beta, Helianthus, Nicotiana, Cucurbita, Rosa, Fragaria, Lotus, Medicago, Onobrychis, trifolium, Trigonella, Vigna, Citrus, Linum, Geranium, Manihot, Daucus, Raphanus, Sinapis, Atropa, Datura, Hyoscyamus, Nicotiana, Petunia, Digitalis, Majorana, Ciahorium, Lactuca, Bromus, Asparagus, Antirrhinum, Heterocallis, Nemesis, Pelargonium, Panicum, Pennisetum, Ranunculus, Senecio*, *Salpiglossis*, *Browaalia*, *Phaseolus*, *Avena*, and *Allium.* In one embodiment the plant is a monocotyledonous plant or a dicotyledonous plant.

Preferably the plant is a crop plant. Crop plants are all plants, used in agriculture. Accordingly in one embodiment the plant is a monocotyledonous plant, preferably a plant of the family *Poaceae*, *Musaceae*, *Liliaceae* or *Bromeliaceae,* preferably of the family *Poaceae.* Accordingly, in yet another embodiment the plant is a *Poaceae* plant of the genus *Zea*, *Triticum, Oryza, Hordeum, Secale, A vena, Saccharum, Sorghum, Pennisetum, Setaria, Panicum, Eleusine*, *Miscanthus*, *Brachypodium*, *Festuca* or *Lolium.* When the plant is of the genus *Zea,* the preferred species is *Z. mays.* When the plant is of the genus *Triticum*, the preferred species is *T. aestivum, T. speltae* or *T*. *durum*. When the plant is of the genus *Oryza,* the preferred species is *O*. *sativa*. When the plant is of the genus *Hordeum,* the preferred species is *H*. *vulgare*. When the plant is of the genus *Secale*, the preferred species *S. cereale.* When the plant is of the genus *Avena,* the preferred species is *A. sativa.* When the plant is of the genus *Saccarum*, the preferred species is *S. officinarum.* When the plant is of the genus *Sorghum,* the preferred species is *S*. *vulgare*, *S*. *bicoloror S*. *sudanense*. When the plant is of the genus *Pennisetum*, the preferred species is *P*. *glaucum*. When the plant is of the genus *Setaria*, the preferred species is *S*. *italica*. When the plant is of the genus *Panicum,* the preferred species is *P*. *miliaceum* or *P*. *virgatum*. When the plant is of the genus *Eleusine*, the preferred species is *E. coracana.* When the plant is of the genus *Miscanthus*, the preferred species is *M*. *sinensis*. When the plant is a plant of the genus *Festuca,* the preferred species is *F*. *arundinaria*, *F*. *rubra* or *F*. *pratensis*. When the plant is of the genus *Lolium,* the preferred species is *L*. *perenne or L*. *multiflorum*. Alternatively, the plant may be *Triticosecale*. Alternatively, in one embodiment the plant is a dicotyledonous plant, preferably a plant of the family Fabaceae, Solanaceae, Brassicaceae, Chenopodiaceae, Asteraceae, Malvaceae, Linacea, Euphorbiaceae, Convolvulaceae Rosaceae, Cucurbitaceae, Theaceae, Rubiaceae, Sterculiaceae or Citrus. In one embodiment the plant is a plant of the family Fabaceae, Solanaceae or Brassicaceae. Accordingly, in one embodiment the plant is of the family Fabaceae, preferably of the genus Glycine, Pisum, Arachis, Cicer, Vicia, Phaseolus, Lupinus, Medicago or Lens. Preferred species of the family Fabaceae are *M*. *truncatula*, *M*, *sativa*, *G*. *max*, *P*. *sativum, A. hypogea, C. arietinum, V. faba, P*. *vulgaris*, *Lupinus albus, Lupinus luteus, Lupinus angustifolius or Lens culinaris.* More preferred are the species *G. max A. hypogea* and *M*. *sativa.* Most preferred is the species *G. max.* When the plant is of the family Solanaceae, the preferred genus is Solanum, Lycopersicon, Nicotiana or Capsicum. Preferred species of the family Solanaceae are *S*. *tuberosum*, *L*. *esculentum*, *N*. *tabaccum* or *C. chinense.* More preferred is *S. tuberosum.* Accordingly, in one embodiment the plant is of the family Brassicaceae, preferably of the genus Brassica or Raphanus. Preferred species of the family Brassicaceae are the species *B. napus*, *B. oleracea*, *B. juncea or B. rapa.* More preferred is the species *B. napus.* When the plant is of the family Chenopodiaceae, the preferred genus is Beta and the preferred species is the *B. vulgaris.* When the plant is of the family Asteraceae, the preferred genus is Helianthus and the preferred species is *H. annuus.* When the plant is of the family Malvaceae, the preferred genus is *Gossypium or Abelmoschus.* When the genus is Gossypium, the preferred species is *G. hirsutum* or *G. barbadense* and the most preferred species is *G*. *hirsutum.* A preferred species of the genus Abelmoschus is the species *A. esculentus.* When the plant is of the family Linacea, the preferred genus is Linum and the preferred species is *L*. *usitatissimum*. When the plant is of the family Euphorbiaceae, the preferred genus is Manihot, Jatropa or Rhizinus and the preferred species are *M*. *esculenta*, *J*. *curcas* or *R*. *comunis*. When the plant is of the family Convolvulaceae, the preferred genus is Ipomea and the preferred species is *I*. *batatas*. When the plant is of the family Rosaceae, the preferred genus is Rosa, Malus, Pyrus, Prunus, Rubus, Ribes, Vaccinium or Fragaria and the preferred species is the hybrid *Fragaria x ananassa.* When the plant is of the family Cucurbitaceae, the preferred genus is Cucumis, Citrullus or Cucurbita and the preferred species is *Cucumis sativus*, *Citrullus lanatus* or *Cucurbita pepo.* When the plant is of the family Theaceae, the preferred genus is Camellia and the preferred species is *C. sinensis.* When the plant is of the family Rubiaceae, the preferred genus is Coffea and the preferred species is *C. arabica* or *C. canephora.* When the plant is of the family Sterculiaceae, the preferred genus is Theobroma and the preferred species is *T. cacao.* When the plant is of the genus Citrus, the preferred species is *C*. *sinensis*, *C*. *limon*, *C*. *reticulata*, *C*. *maxima* and hybrids of Citrus species, or the like. In a preferred embodiment of the invention, the plant is a soybean, a potato or a corn plant

In one embodiment the plant is a Fabaceae plant and the target gene is substantially similar to SEQ ID NO: 1, or 2.

Suitable methods for transforming or transfecting host cells including plant cells are well known in the art of plant biotechnology. Any method may be used to transform the recombinant expression vector into plant cells to yield the transgenic plants of the invention. General methods for transforming dicotyledenous plants are disclosed, for example, in U.S. Pat. Nos. 4,940,838; 5,464,763, and the like. Methods for transforming specific dicotyledenous plants, for example, cotton, are set forth in U.S. Pat. Nos. 5,004,863; 5,159,135; and 5,846,797. Soybean transformation methods are set forth in U.S. Pat. Nos. 4,992,375; 5,416,011; 5,569,834; 5,824,877; 6,384,301 and in EP 0301749B1 may be used. Transformation methods may include direct and indirect methods of transformation. Suitable direct methods include polyethylene glycol induced DNA uptake, liposome-mediated transformation (US 4,536,475), biolistic methods using the gene gun (Fromm ME et al., Bio/Technology. 8(9):833-9, 1990; Gordon-Kamm et al. Plant Cell 2:603, 1990), electroporation, incubation of dry plant embryos in DNA-comprising solution, and microinjection. In the case of these direct transformation methods, the plasmids used need not meet any particular requirements. Simple plasmids, such as those of the pUC series, pBR322, M13mp series, pACYC184 and the like can be used. If intact plants are to be regenerated from the transformed cells, an additional selectable marker gene is preferably located on the plasmid. The direct transformation techniques are equally suitable for dicotyledonous and monocotyledonous plants.

Transformation can also be carried out by bacterial infection by means of Agrobacterium (for example EP 0 116 718), viral infection by means of viral vectors (EP 0 067 553; US 4,407,956; WO 95/34668; WO 93/03161) or by means of pollen (EP 0 270 356; WO 85/01856; US 4,684,611). *Agrobacterium* based transformation techniques (especially for dicotyledonous plants) are well known in the art. The Agrobacterium strain (*e.g., Agrobacterium tumefaciens* or *Agrobacterium rhizogenes*) comprises a plasmid (Ti or Ri plasmid) and a T-DNA element which is transferred to the plant following infection with *Agrobacterium.* The T-DNA (transferred DNA) is integrated into the genome of the plant cell. The T-DNA may be localized on the Ri- or Ti-plasmid or is separately comprised in a so-called binary vector. Methods for the *Agrobacterium*-mediated transformation are described, for example, in Horsch RB et al. (1985) Science 225:1229. The *Agrobacterium*-mediated transformation is best suited to dicotyledonous plants but has also been adapted to monocotyledonous plants. The transformation of plants by Agrobacteria is described in, for example, White FF, Vectors for Gene Transfer in Higher Plants, Transgenic Plants, Vol. 1, Engineering and Utilization, edited by S.D. Kung and R. Wu, Academic Press, 1993, pp. 15 - 38; Jenes B et al. Techniques for Gene Transfer, Transgenic Plants, Vol. 1, Engineering and Utilization, edited by S.D. Kung and R. Wu, Academic Press, 1993, pp. 128-143; Potrykus (1991) Annu Rev Plant Physiol Plant Molec Biol 42:205- 225. Transformation may result in transient or stable transformation and expression. Although a nucleotide sequence of the present invention can be inserted into any plant and plant cell falling within these broad classes, it is particularly useful in crop plant cells.

The transgenic plants of the invention may be crossed with similar transgenic plants or with transgenic plants lacking the nucleic acids of, the invention or with nontransgenic plants, using known methods of plant breeding, to prepare seeds. Further, the transgenic plant of the present invention may comprise, and/or be crossed to another transgenic plant that comprises one or more nucleic acids, thus creating a "stack" of transgenes in the plant and/or its progeny. The seed is then planted to obtain a crossed fertile transgenic plant comprising the nucleic acid of the invention. The crossed fertile transgenic plant may have the particular expression cassette inherited through a female parent or through a male parent. The second plant may be an inbred plant. The crossed fertile transgenic may be a hybrid. Also included within the present invention are seeds of any of these crossed fertile transgenic plants. The seeds of this invention can be harvested from fertile transgenic plants and be used to grow progeny generations of transformed plants of this invention including hybrid plant lines comprising the DNA construct.

"Gene stacking" can also be accomplished by transferring two or more genes into the cell nucleus by plant transformation. Multiple genes may be introduced into the cell nucleus during transformation either sequentially or in unison. Multiple genes in plants or target pathogen species can be down-regulated by gene silencing mechanisms, specifically RNAi, by using a single transgene targeting multiple linked partial sequences of interest. Stacked, multiple genes under the control of individual promoters can also be over-expressed to attain a desired single or multiple phenotype. Constructs containing gene stacks of both over-expressed genes and silenced targets can also be introduced into plants yielding single or multiple agronomically important phenotypes. In certain embodiments the nucleic acid sequences of the present invention can be stacked with any combination of polynucleotide sequences of interest to create desired phenotypes. The combinations can produce plants with a variety of trait combinations including but not limited to disease resistance, herbicide tolerance, yield enhancement, cold and drought tolerance. These stacked combinations can be created by any method including but not limited to cross breeding plants by conventional methods or by genetic transformation. If the traits are stacked by genetic transformation, the polynucleotide sequences of interest can be combined sequentially or simultaneously in any order. For example if two genes are to be introduced, the two sequences can be contained in separate transformation cassettes or on the same transformation cassette. The expression of the sequences can be driven by the same or different promoters.

In accordance with this embodiment, the transgenic plant of the invention is produced by a method comprising the steps of providing a CDPK-like target gene, preparing an expression cassette having a first region that is substantially identical to a portion of the selected CDPK-like gene and a second region which is complementary to the first region, transforming the expression cassette into a plant, and selecting progeny of the transformed plant which express the dsRNA construct of the invention.

Increased resistance to nematode infection is a general trait wished to be inherited into a wide variety of plants. Increased resistance to nematode infection is a general trait wished to be inherited into a wide variety of plants. The present invention may be used to reduce crop destruction by any plant parasitic nematode. Preferably, the parasitic nematodes belong to nematode families inducing giant or syncytial cells. Nematodes inducing giant or syncytial cells are found in the families Longidoridae, Trichodoridae, Heterodidae, Meloidogynidae, Pratylenchidae or Tylenchulidae. In particular in the families Heterodidae and Meloidogynidae.

Accordingly, parasitic nematodes targeted by the present invention belong to one or more genus selected from the group of Naccobus, Cactodera, Dolichodera, Globodera, Heterodera, Punctodera, Longidorus or Meloidogyne. In a preferred embodiment the parasitic nematodes belong to one or more genus selected from the group of Naccobus, Cactodera, Dolichodera, Globodera, Heterodera, Punctodera or Meloidogyne. In a more preferred embodiment the parasitic nematodes belong to one or more genus selected from the group of Globodera, Heterodera, or Meloidogyne. In an even more preferred embodiment the parasitic nematodes belong to one or both genus selected from the group of Globodera or Heterodera. In another embodiment the parasitic nematodes belong to the genus Meloidogyne.

When the parasitic nematodes are of the genus *Globodera,* the species are preferably from the group consisting of *G*. *achilleae*, *G*. *artemisiae*, *G*. *hypolysi*, *G*. *mexicana*, *G*. *millefolii*, *G*. *mali*, *G*. *pallida*, *G*. *rostochiensis*, *G*. *tabacum*, and *G*. *virginiae*. In another preferred embodiment the parasitic *Globodera* nematodes includes at least one of the species *G*. *pallida*, *G*. *tabacum*, or *G*. *rostochiensis*. When the parasitic nematodes are of the genus *Heterodera*, the species may be preferably from the group consisting of *H*. *avenae*, *H*. *carotae*, *H. ciceri, H cruciferae, H delvii, H. elachista, H. filipjevi, H. gambiensis, H*. *glycines*, *H*. *goettingiana, H graduni, H humuli, H. hordecalis, H. latipons, H major, H. medicaginis, H. oryzicola, H*. *pakistanensis*, *H*. *rosii*, *H*. *sacchari*, *H. schachtii*, *H*. *sorghi*, *H*. *trifolii*, *H. urticae*, *H. vigni* and *H. zeae.* In another preferred embodiment the parasitic *Heterodera* nematodes include at least one of the species *H*. *glycines*, *H*. *avenae*, *H*. *cajani*, *H*. *gottingiana*, *H*. *trifolii*, *H. zeae* or *H*. *schachtii*. In a more preferred embodiment the parasitic nematodes includes at least one of the species *H*. *glycines* or *H. schachtii.* In a most preferred embodiment the parasitic nematode is the species *H. glycines*.

When the parasitic nematodes are of the genus Meloidogyne, the parasitic nematode may be selected from the group consisting of M. acronea, M. arabica, M. arenaria, M. artiellia, M. brevicauda, M. camelliae, M. chitwoodi, M. cofeicola, M. esigua, M. graminicola, M. hapla, M. incognita, M. indica, M. inomata, M. javanica, M. lini, M. mali, M. microcephala, M. microtyla, M. naasi, M. salasi and M. thamesi. In a preferred embodiment the parasitic nematodes includes at least one of the species M. javanica, M. incognita, M. hapla, M. arenaria or M. chitwoodi.

The following examples are not intended to limit the scope of the claims to the invention, but are rather intended to be exemplary of certain embodiments. Any variations in the exemplified methods that occur to the skilled artisan are intended to fall within the scope of the present invention.

### BRIEF DESCRIPTION OF THE DRAWINGS

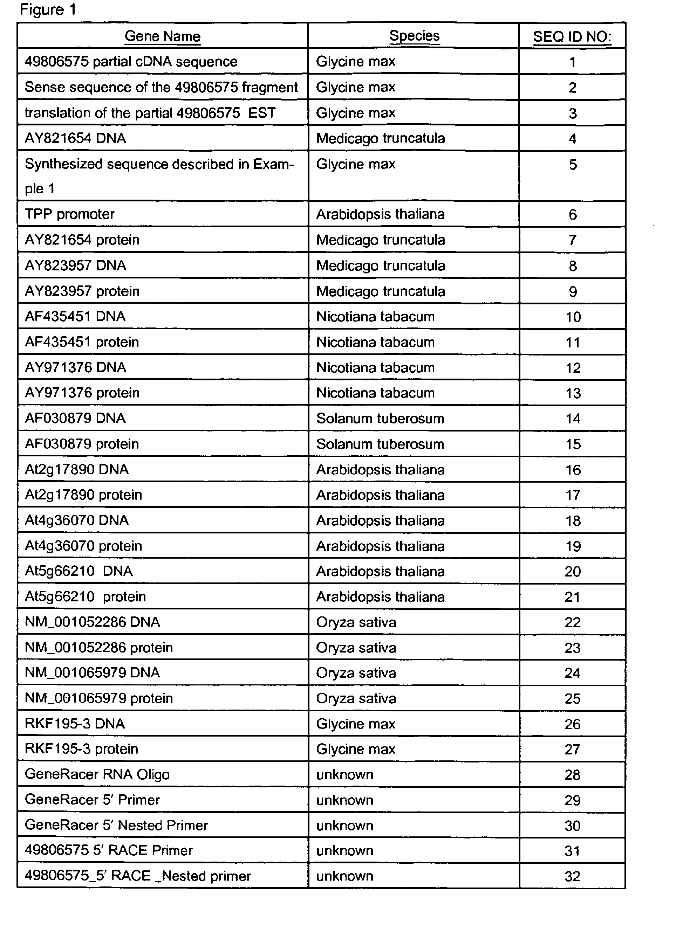

Figure 1 shows the table of SEQ ID NOs assigned to corresponding sequences. SEQ ID NO: 1 is the partial cDNA sequence from *Glycine max* Hygene clone 49806575, including the stop codon and 3' untranslated region. SEQ ID NO: 2 is the sense sequence of the fragment of 49806575 (SEQ ID NO: 1) used in the rooted explant assay of Example 2. SEQ ID NO; 3 is the amino acid sequence encoded by 49806575 (SEQ ID NO: 1). SEQ ID NO: 4 is the cDNA sequence of *Medicago* Gen-bank accession AY821654, including non-coding and coding sequences. The first base of the coding region corresponds to nucleotide 147, and the last base of the stop codon corresponds to nucleotide 1829. SEQ ID NO: 5 is the synthesized sequence described in Example 1., and SEQ ID NO: 6 is the sequence of the TPP-like promoter (SEQ ID NO:6) described in co-pending USSN 60/874,375 and hereby incorporated by reference

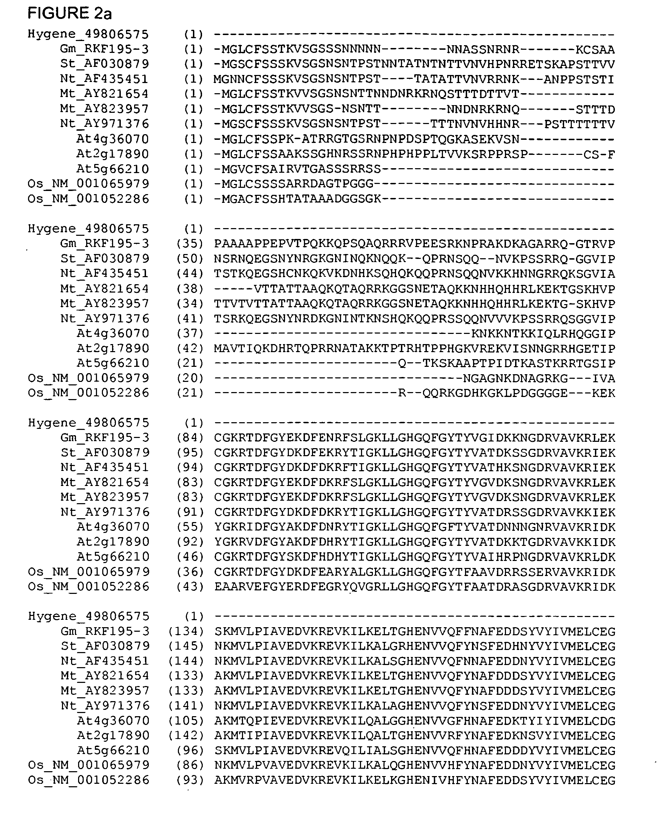

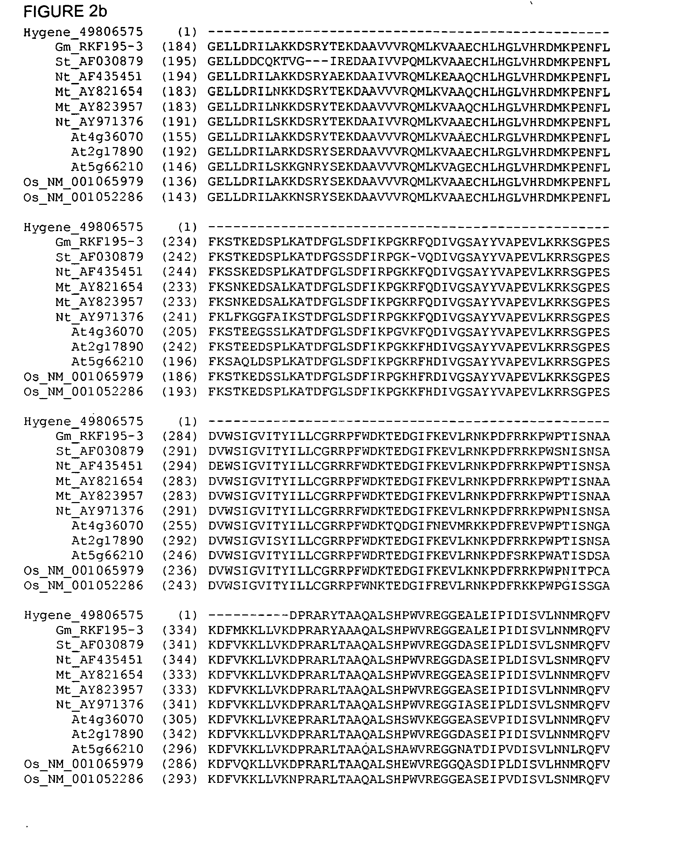

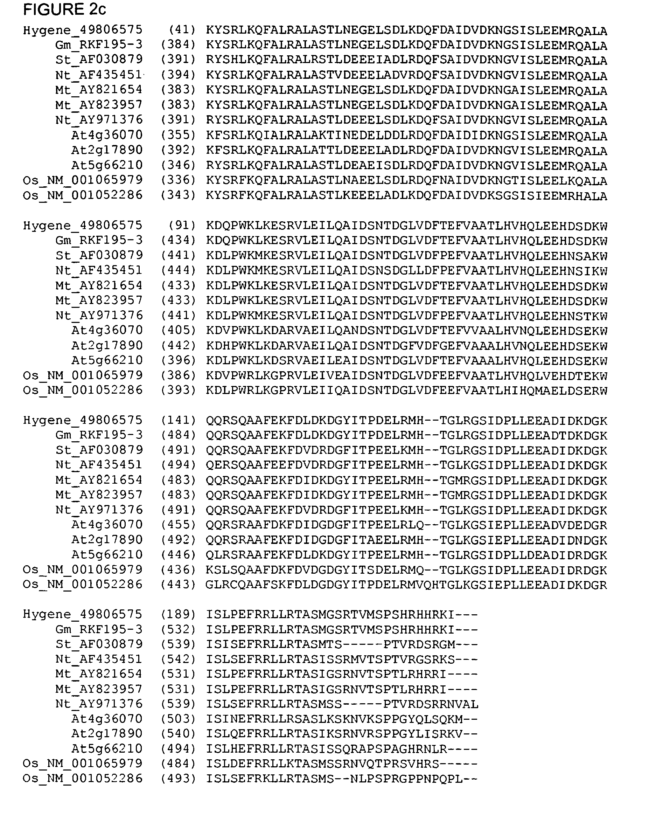

Figures 2a-2c show amino acid alignment of CDPK-like proteins: the partial *Glycine* cDNA clone 49806575 (SEQ ID NO:3), the protein encoded by AY821654 from *Medicago* (SEQ ID NO: 7), the protein encoded by AY823957 from *Medicago* (SEQ ID NO: 9), the protein encoded by AF435451 from *Nicotiana* (SEQ ID NO: 11), the protein encoded by AY971376 from *Nicotiana* (SEQ ID NO: 13), the protein encoded by AF030879 from *Solanum* (SEQ ID NO: 15), the protein encoded by At2g17890 from *Arabidopsis* (SEQ ID NO: 17), the protein encoded by At4g36070 from *Arabidopsis* (SEQ ID NO: 19), the protein encoded by At5g66210 from *Arabidopsis* (SEQ ID NO: 21), the protein encoded by NM_001052286 from *Oryza* (SEQ ID NO: 23), the protein encoded by NM_001065979 from *Oryza* (SEQ ID NO: 25) and the amplified product from CDPK 5' RACE PCR, RKF195-3, from *Glycine* (SEQ ID NO:27). The alignment is performed in Vector NTI software suite (gap opening penalty = 10, gap extension penalty = 0.05, gap separation penalty = 8).

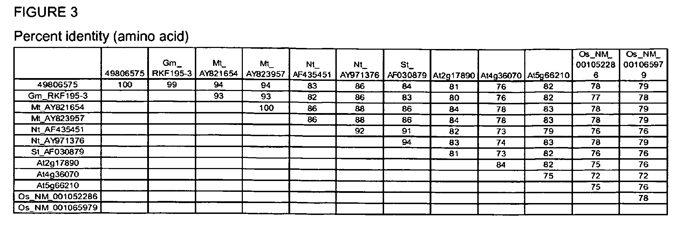

Figure 3 shows the global amino acid percent identity of exemplary CDPK-like genes: the protein encoded by the partial *Glycine* clone 49806575 (SEQ ID NO:3), the protein encoded by RKF195-3, from *Glycine* (SEQ ID NO: 27), the protein encoded by AY821654 from *Medicago* (SEQ ID NO: 7), the protein encoded by AY823957 from *Medicago* (SEQ ID NO: 9), the protein encoded by AF435451 from *Nicotiana* (SEQ ID NO:11), the protein encoded by AY971376 from *Nicotiana* (SEQ ID NO:13), the protein encoded by AF030879 from *Solanum* (SEQ ID NO: 15), the protein encoded by At2g17890 from *Arabidopsis* (SEQ ID NO:17), the protein encoded by At4g36070 from *Arabidopsis* (SEQ ID NO: 19), the protein encoded by At5g66210 from *Arabidopsis* (SEQ ID NO: 21), the protein encoded by NM_001052286 from *Oryza* (SEQ ID NO: 23) and the protein encoded by NM_001065979 from *Oryza* (SEQ ID NO: 25). Only the region overlapping with the partial cDNA clone 49806575 was included in the analysis. Pairwise alignments and percent identities were calculated using Needle of EMBOSS-4.0.0 (Needleman, S. B. and Wunsch, C. D. (1970) J. Mol. Biol. 48, 443-453).

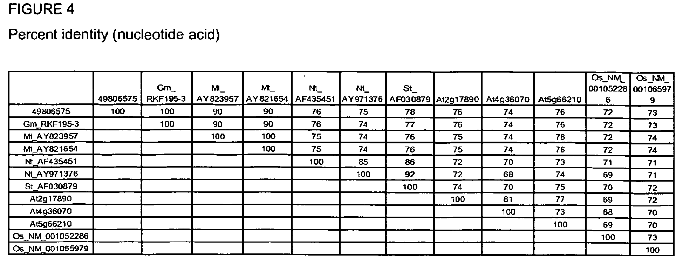

Figure 4 shows the global nucleotide percent identity of exemplary CDPK-like genes: the partial *Glycine* clone 49806575 (SEQ ID NO: 1), RKF195-3, from *Glycine* (SEQ ID NO:26), AY821654 from *Medicago* (SEQ ID NO:4), AY823957 from *Medicago* (SEQ ID NO: 8), AF435451 from *Nicotiana* (SEQ ID NO: 10), AY971376 from *Nicotiana* (SEQ ID NO: 12), AF030879 from *Solanum* (SEQ ID NO: 14), .At2g17890 from *Arabidopsis* (SEQ ID NO: 16), At4g36070 from *Arabidopsis* (SEQ ID NO: 18), At5g66210 from *Arabidopsis* (SEQ ID NO: 20), NM_001052286 from *Oryza* (SEQ ID NO: 22), and NM_001065979 from *Oryza* (SEQ ID NO: 24). Only the region overlapping with the partial cDNA clone 49806575 was included in the analysis. Pairwise alignments and percent identities were calculated using Needle of EMBOSS-4.0.0 (Needleman, S. B. and Wunsch, C. D. (1970) J. Mol. Biol. 48, 443-453).

Figures 5a-5g show various 21 mers possible for exemplary CDPK-like genes of SEQ ID NO: 1, 2, 4, 5, 8, 10, 12, 14, 16, 18, 20, 22, 24, or 26 or a polynucleotide sequence encoding a CDPK-like homolog by nucleotide position.

### EXAMPLE 1: Binary Vector Construction for Soybean Transformation

This exemplified method employs a binary vector containing the target gene corresponding to soybean cDNA clone 49806575. Clone 49806575 was identified by searching a proprietary database of cDNA sequences using the public *Medicago truncatula* sequence AY821654. The expression vector consists of the synthesized fragment (SEQ ID NO :5), which in turn is comprised of a 320 bp antisense portion of 49806575 gene, a spacer, a sense fragment of the target gene (SEQ ID NO:2) corresponding to nucleotides 50-372 of SEQ ID NO:1, and a vector backbone. In this vector, RCB562, dsRNA for the 49806575 target gene was expressed under a syncytia or root preferred promoter, TPP-like promoter (SEQ ID NO: 6, see co-pending application U.S. patent application 60/874,375, hereby incorporated by reference). The TPP-like promoter drives transgene expression preferentially in roots and/or syncytia or giant cells. The selection marker for transformation in this vector was a mutated AHAS gene from *Arabidopsis thaliana* that conferred resistance to the herbicide Arsenal (Imazapyr, BASF Corporation, Mount Olive, NJ). The expression of mutated AHAS was driven by the parsley ubiquitin promoter (See Plesch, G. and Ebneth, M., "Method for the stable expression of nucleic acids in transgenic plants, controlled by a parsley ubiquitin promoter", WO 03/102198, hereby incorporated by reference.).

### EXAMPLE 2: Use of Soybean Plant Assay System to Detect Resistance to SCN Infection

The rooted explant assay was employed to demonstrate dsRNA expression and resulting nematode resistance. This assay can be found in co-pending application USSN 12/001,234, the contents of which are hereby incorporated by reference.

Clean soybean seeds from soybean cultivar were surface sterilized and germinated. Three days before inoculation, an overnight liquid culture of the disarmed *Agrobacterium* culture, for example, the disarmed *A. rhizogenes* strain K599 containing the binary vector RCB562, was initiated. The next day the culture was spread onto an LB agar plate containing kanamycin as a selection agent. The plates were incubated at 28°C for two days. One plate was prepared for every 50 explants to be inoculated. Cotyledons containing the proximal end from its connection with the seedlings were used as the explant for transformation. After removing the cotyledons the surface was scraped with a scalpel around the cut site. The cut and scraped cotyledon was the target for *Agrobacterium* inoculation. The prepared explants were dipped onto the disarmed thick *A. rhizogenes* colonies prepared above so that the colonies were visible on the cut and scraped surface. The explants were then placed onto 1 % agar in Petri dishes for co-cultivation under light for 6-8 days.

After the transformation and co-cultivation soybean explants were transferred to rooting induction medium with a selection agent, for example S-B5-708 for the mutated aceto-hydroxy acid synthase (AHAS) gene (Sathasivan et al., Plant Phys. 97:1044-50, 1991). Cultures were maintained in the same condition as in the co-cultivation step. The S-B5-708 medium comprises: 0.5X B5 salts, 3mM MES, 2% sucrose, 1X B5 vitamins, 400µg/ml Timentin, 0.8% Noble agar, and 1 µM Imazapyr (selection agent for AHAS gene) (BASF Corporation, Florham Park, NJ) at pH5.8.
Two to three weeks after the selection and root induction, transformed roots were formed on the cut ends of the explants. Explants were transferred to the same selection medium (S-B5-708 medium) for further selection. Transgenic roots proliferated well within one week in the medium and were ready to be subcultured.

Strong and white soybean roots were excised from the rooted explants and cultured in root growth medium supplemented with 200 mg/l Timentin (S-MS-606 medium) in six-well plates. Cultures were maintained at room temperature under the dark condition. The S-MS-606 medium comprises: 0.2X MS salts and B5 vitamins, 2% sucrose, and 200mg/l Timentin at pH5.8.
One to five days after sub-culturing, the roots were inoculated with surface sterilized nematode juveniles in multi-well plates for either gene of interest or promoter construct assay. As a control, soybean cultivar Williams 82 control vector and Jack control vector roots were used. The root cultures of each line that occupied at least half of the well were inoculated with surface-decontaminated race 3 of soybean cyst nematode (SCN) second stage juveniles (J2) at the level of 500 J2/well. The plates were then sealed and put back into the incubator at 25°C in darkness. Several independent root lines were generated from each binary vector transformation and the lines were used for bioassay. Four weeks after nematode inoculation, the cysts in each well were counted.

For each transformed line, the average number of cysts per line, the percent female index and the standard error values were determined across several replicated wells (Female index = average number of SCN cysts developing on the transgenic roots expressed as percentage of the average number of cysts developing on the W82 wild type susceptible control roots). Multiple independent, biologically replicated experiments were run to compare cyst numbers between RCB562 transformants and susceptible Williams82 lines. The results show that RCB562 transformed roots had statistically significant reductions (p-value ≤ 0.05) in cyst count over multiple transgenic lines and a general trend of reduced cyst count in the majority of transgenic lines assayed.

### Example 3 RACE To Determine Full Transcribed Sequence

A full length transcript sequence with high homology to the partial cDNA clone 49806575 (SEQ ID NO: 1) was isolated using the GeneRacer Kit (L1502-01) from Invitrogen by following the manufacturers instructions. Total RNA from soybean roots harvested 6 days after infection with SCN was prepared according to the Invitrogen GeneRacer Kit protocol to generate dephosphorylated and decapped RNA ligated to the GeneRacer RNA Oligo described by SEQ ID NO:28. The prepared RNA was reverse transcribed according to the GeneRacer Kit protocol and used as the RACE library template for PCR to isolate 5' cDNA ends using primary and secondary (nested) PCR reactions according to the GeneRacer Kit protocol. The primers used for the primary PCR reaction are described by SEQ ID NOs 29 and 31. The secondary nested PCR reaction primers are described by SEQ ID NOs 30 and 32.

Products from secondary PCR reaction were separated by gel electrophoresis. Specific products were purified from agarose gel and cloned into pCR4-TOPO vectors (Invitrogen) following manufacturers instructions, Resulting colonies were miniprepped and sequenced. One of the full length fragments described as SEQ ID NO:26 (RKF195-3_2) had high percent identity with SEQ ID NO:1 (49806575 cDNA sequence). The alignment between proteins encoded by the partial *Glycine max* 49806575 sequence, the full length *Glycine max* RKF195-3_2 and CDPK-like genes from other plant species is shown in Figures 2a-2d.

Those skilled in the art will recognize, or will be able to ascertain using no more than routine experimentation, many equivalents to the specific embodiments of the invention described herein. Such equivalents are intended to be encompassed by the following claims.

## Claims

1. A method of conferring nematode resistance to a plant, said method comprising the steps of:
a) preparing a nucleic acid encoding a dsRNA molecule having a region that is absolutely identical to a portion of a CDPK-like gene, wherein the nucleic acid is able to form a double-stranded transcript of a portion of a CDPK-like gene once expressed in the plant;
b) transforming a recipient plant with said nucleic acid;
c) producing one or more transgenic offspring of said recipient plant; and
d) selecting the offspring for nematode resistance;
wherein the portion of the CDPK-like gene is from a polynucleotide selected from the group consisting of:
a) a polynucleotide comprising a sequence as set forth in SEQ ID NO:1, or 2;
b) a polynucleotide encoding a polypeptide having a sequence as set forth in SEQ ID NO:3;
c) a polynucleotide having 70% sequence identity to a polynucleotide having a sequence as set forth in SEQ ID NO: 1, or 2;
d) a polynucleotide encoding a polypeptide having 70% sequence identity to a polypeptide having a sequence as set forth in SEQ ID NO: 3,
e) a polynucleotide comprising a fragment of at least 200 consecutive nucleotides of a polynucleotide having a sequence as set forth in SEQ ID NO: 1, or 2.

2. Use of a dsRNA molecule comprising i) a first strand comprising a sequence absolutely identical to a portion of a CDPK-like gene, and ii) a second strand comprising a sequence complementary over all their nucleotides to the first strand, wherein the portion of the CDPK-like gene is from a polynucleotide selected from the group consisting of:
a) a polynucleotide comprising a sequence as set forth in SEQ ID NO:1, or 2;
b) a polynucleotide encoding a polypeptide having a sequence as set forth in SEQ ID NO:3;
c) a polynucleotide having 70% sequence identity to a polynucleotide having a sequence as set forth in SEQ ID NO: 1, or 2;
d) a polynucleotide encoding a polypeptide having 70% sequence identity to a polypeptide having a sequence as set forth in SEQ ID NO: 3;
e) a polynucleotide comprising a fragment of at least 200 consecutive nucleotides of a polynucleotide having a sequence as set forth in SEQ ID NO: 1, or 2
for conferring resistance to plant parasitic nematodes.

3. Use of a pool of dsRNA molecules comprising a multiplicity of RNA molecules each comprising a double stranded region having a length of 19 to 24 nucleotides, wherein said dsRNA molecules are derived from a polynucleotide selected from the group consisting of:
a) a polynucleotide comprising a sequence as set forth in SEQ ID NO: 1, or 2;
b) a polynucleotide encoding a polypeptide having a sequence as set forth in SEQ ID NO: 3;
c) a polynucleotide having 90% sequence identity to a polynucleotide having a sequence as set forth in SEQ ID NO: 1, or 2; and
d) a polynucleotide encoding a polypeptide having 90% sequence identity to a polypeptide having a sequence as set forth in SEQ ID NO: 3;
for conferring resistance to plant parasitic nematodes.

4. A parasitic nematode resistant transgenic plant capable of expressing a dsRNA that is absolutely identical to a portion of a CDPK-like gene, wherein the portion of the CDPK-like gene is from a polynucleotide selected from the group consisting of:
a) a polynucleotide comprising a sequence as set forth in SEQ ID NO: 1, or 2;
b) a polynucleotide having 95% sequence identity to a polynucleotide having a sequence as set forth in SEQ ID NO: 1, or 2;
c) a polynucleotide comprising a fragment of at least 200 consecutive nucleotides of a polynucleotide having a sequence as set forth in SEQ ID NO: 1, or 2.

5. The plant of claim 4, wherein the dsRNA comprises a multiplicity of RNA molecules each comprising a double stranded region having a length of 19 to 24 nucleotides, wherein said RNA molecules are derived from a portion of a polynucleotide selected from the group consisting of:
a) a polynucleotide comprising a sequence as set forth in SEQ ID NO: 1, or 2;
b) a polynucleotide having 95% sequence identity to a polynucleotide having a sequence as set forth in SEQ ID NO: 1, or 2.

6. A method for making a nematode-resistant transgenic plant capable of expressing a dsRNA that inhibits expression of an CDPK-like gene in the plant, said method comprises the steps of i) preparing a nucleic acid having a region that is absolutely identical to a portion of the CDPK-like gene, wherein the nucleic acid is able to form a double-stranded transcript once expressed in the plant; ii) transforming a recipient plant with said nucleic acid; iii) producing one or more transgenic offspring of said recipient plant; and iv) selecting the offspring for expression of said transcript, wherein the portion of the CDPK-like gene is from a polynucleotide selected from the group consisting of:
a) a polynucleotide comprising a sequence as set forth in SEQ ID NO: 1, 2;
b) a polynucleotide encoding a polypeptide having a sequence as set forth in SEQ ID NO: 3;
c) a polynucleotide having 70% sequence identity to a polynucleotide having a sequence as set forth in SEQ ID NO: 1, or 2;
d) a polynucleotide encoding a polypeptide having 70% sequence identity to a polypeptide having a sequence as set forth in SEQ ID NO: 3;
e) a polynucleotide comprising a fragment of at least 200 consecutive nucleotides of a polynucleotide having a sequence as set forth in SEQ ID NO: 1, or 2.

7. The method of claim 6, wherein the dsRNA comprises a multiplicity of RNA molecules each comprising a double stranded region having a length of 19 to 24 nucleotides, wherein said RNA molecules are derived from a polynucleotide selected from the group consisting of:
a) a polynucleotide comprising a sequence as set forth in SEQ ID NO: 1, or 2;
b) a polynucleotide encoding a polypeptide having a sequence as set forth in SEQ ID NO: 3;
c) a polynucleotide having 90% sequence identity to a polynucleotide having a sequence as set forth in SEQ ID NO: 1, or 2; and
d) a polynucleotide encoding a polypeptide having 90% sequence identity to a polypeptide having a sequence as set forth in SEQ ID NO: 3.

8. The plant of any one of claims 4 or 5, or the method of any one of claims 6 or 7, wherein the plant is selected from the group consisting of soybean, potato, tomato, peanuts, cotton, cassava, coffee, coconut, pineapple, citrus trees, banana, corn, rape, beet, sunflower, sorghum, wheat, oats, rye, barley, rice, green bean, lima bean, pea, and tobacco.

9. The method of any one of claims 6 or 7, wherein the dsRNA is expressed in plant roots or syncytia.

10. A method for controlling the infection of a plant by a parasitic nematode, comprising the steps of transforming the plant with a nucleic acid encoding a dsRNA molecule operably linked to a root-preferred, nematode inducible or nematode feeding site-preferred promoter, whereby the dsRNA comprising one strand that is absolutely identical to a portion of a target nucleic acid essential to the formation, development or support of the feeding site, in particular the formation, development or support of a syncytia or giant cell, thereby controlling the infection of the plant by the nematode by removing or functionally incapacitating the feeding site, syncytia or giant cell,
wherein the target nucleic acid is a CDPK-like gene, wherein the portion of the CDPK-like gene is from a polynucleotide selected from the group consisting of:
a) a polynucleotide comprising a sequence as set forth in SEQ ID NO:1, or 2;
b) a polynucleotide encoding a polypeptide having a sequence as set forth in SEQ ID NO:3;
c) a polynucleotide having 70% sequence identity to a polynucleotide having a sequence as set forth in SEQ ID NO: 1, or 2;
d) a polynucleotide encoding a polypeptide having 70% sequence identity to a polypeptide having a sequence as set forth in SEQ ID NO: 3.

## Patentansprüche

1. Verfahren zum Vermitteln von Nematodenresistenz an eine Pflanze, wobei das Verfahren die folgenden Schritte umfasst:
a) Herstellen einer Nukleinsäure, die für ein dsRNA-Molekül mit einer Region, die zu einem Abschnitt eines CDPK-like-Gens völlig identisch ist, codiert, wobei die Nukleinsäure, sobald sie in der Pflanze exprimiert wird, fähig ist, ein doppelsträngiges Transkript eines Abschnitts eines CDPK-like-Gens zu bilden;
b) Transformieren einer Empfängerpflanze mit der Nukleinsäure;
c) Herstellen von einem oder mehreren transgenen Nachkommen der Empfängerpflanze; und
d) Selektieren der Nachkommen auf Nematodenresistenz;
wobei der Abschnitt des CDPK-like-Gens von einem Polynukleotid, ausgewählt aus der Gruppe bestehend aus:
a) einem Polynukleotid umfassend eine Sequenz gemäß SEQ ID NO: 1 oder 2;
b) einem Polynukleotid, das für ein Polypeptid mit einer Sequenz gemäß SEQ ID NO: 3 codiert;
c) einem Polynukleotid mit 70% Sequenzidentität zu einem Polynukleotid mit einer Sequenz gemäß SEQ ID NO: 1 oder 2;
d) einem Polynukleotid, das für ein Polypeptid mit 70% Sequenzidentität zu einem Polypeptid mit einer Sequenz gemäß SEQ ID NO: 3 codiert,
e) einem Polynukleotid, umfassend ein Fragment von mindestens 200 aufeinanderfolgenden Nukleotiden eines Polynukleotids mit einer Sequenz gemäß SEQ ID NO: 1 oder 2
stammt.

2. Verwendung eines dsRNA-Moleküls, umfassend i) einen ersten Strang, umfassend eine Sequenz, die zu einem Abschnitt eines CDPK-like-Gens völlig identisch ist, und ii) einen zweiten Strang, umfassend eine Sequenz, die zu dem ersten Strang über alle Nukleotide komplementär ist, wobei der Abschnitt des CDPK-like-Gens von einem Polynukleotid, ausgewählt aus der Gruppe bestehend aus:
a) einem Polynukleotid umfassend eine Sequenz gemäß SEQ ID NO: 1 oder 2;
b) einem Polynukleotid, das für ein Polypeptid mit einer Sequenz gemäß SEQ ID NO: 3 codiert;
c) einem Polynukleotid mit 70% Sequenzidentität zu einem Polynukleotid mit einer Sequenz gemäß SEQ ID NO: 1 oder 2;
d) einem Polynukleotid, das für ein Polypeptid mit 70% Sequenzidentität zu einem Polypeptid mit einer Sequenz gemäß SEQ ID NO: 3 codiert,
e) einem Polynukleotid, umfassend ein Fragment von mindestens 200 aufeinanderfolgenden Nukleotiden eines Polynukleotids mit einer Sequenz gemäß SEQ ID NO: 1 oder 2
stammt, für die Vermittlung von Resistenz gegen pflanzenparasitäre Nematoden.

3. Verwendung eines Pools von dsRNA-Molekülen, umfassend eine Vielzahl von RNA-Molekülen, die jeweils eine doppelsträngige Region mit einer Länge von 19 bis 24 Nukleotiden umfassen, wobei die dsRNA-Moleküle von einem Polynukleotid, ausgewählt aus der Gruppe bestehend aus
a) einem Polynukleotid umfassend eine Sequenz gemäß SEQ ID NO: 1 oder 2;
b) einem Polynukleotid, das für ein Polypeptid mit einer Sequenz gemäß SEQ ID NO: 3 codiert;
c) einem Polynukleotid mit 90% Sequenzidentität zu einem Polynukleotid mit einer Sequenz gemäß SEQ ID NO: 1 oder 2; und
d) einem Polynukleotid, das für ein Polypeptid mit 90% Sequenzidentität zu einem Polypeptid mit einer Sequenz gemäß SEQ ID NO: 3 codiert,
stammen, für die Vermittlung von Resistenz gegen pflanzenparasitäre Nematoden.

4. Transgene Pflanze, die gegen parasitäre Nematoden resistent ist und die fähig ist, eine dsRNA zu exprimieren, die zu einem Abschnitt eines CDPK-like-Gens völlig identisch ist, wobei der Abschnitt des CDPK-like-Gens von einem Polynukleotid, ausgewählt aus der Gruppe bestehend aus
a) einem Polynukleotid umfassend eine Sequenz gemäß SEQ ID NO: 1 oder 2;
b) einem Polynukleotid mit 95% Sequenzidentität zu einem Polynukleotid mit einer Sequenz gemäß SEQ ID NO: 1 oder 2;
c) einem Polynukleotid, umfassend ein Fragment von mindestens 200 aufeinanderfolgenden Nukleotiden eines Polynukleotids mit einer Sequenz gemäß SEQ ID NO: 1 oder 2
stammt.

5. Pflanze nach Anspruch 4, wobei die dsRNA eine Vielzahl von RNA-Molekülen umfasst, die jeweils eine doppelsträngige Region mit einer Länge von 19 bis 24 Nukleotiden umfassen, wobei die RNA-Moleküle von einem Abschnitt eines Polynukleotids, ausgewählt aus der Gruppe bestehend aus
a) einem Polynukleotid, umfassend eine Sequenz gemäß SEQ ID NO: 1 oder 2;
b) einem Polynukleotid mit 95% Sequenzidentität zu einem Polynukleotid mit einer Sequenz gemäß SEQ ID NO: 1 oder 2
abstammen.

6. Verfahren zur Herstellung einer nematodenresistenten transgenen Pflanze, die fähig ist, eine dsRNA, die die Expression eines CDPK-like-Gens in der Pflanze hemmt, zu exprimieren, wobei das Verfahren die folgenden Schritte umfasst: i) Herstellen einer Nukleinsäure, mit einer Region, die zu einem Abschnitt des CDPK-like-Gens völlig identisch ist, wobei die Nukleinsäure, sobald sie in der Pflanze exprimiert wird, fähig ist, ein doppelsträngiges Transkript zu bilden; ii) Transformieren einer Empfängerpflanze mit der Nukleinsäure; iii) Herstellen von einem oder mehreren transgenen Nachkommen der Empfängerpflanze; und iv) Selektieren der Nachkommen auf die Expression des Transkripts, wobei der Abschnitt des CDPK-like-Gens von einem Polynukleotid, ausgewählt aus der Gruppe bestehend aus:
a) einem Polynukleotid umfassend eine Sequenz gemäß SEQ ID NO: 1 oder 2;
b) einem Polynukleotid, das für ein Polypeptid mit einer Sequenz gemäß SEQ ID NO: 3 codiert;
c) einem Polynukleotid mit 70% Sequenzidentität zu einem Polynukleotid mit einer Sequenz gemäß SEQ ID NO: 1 oder 2;
d) einem Polynukleotid, das für ein Polypeptid mit 70% Sequenzidentität zu einem Polypeptid mit einer Sequenz gemäß SEQ ID NO: 3 codiert,
e) einem Polynukleotid, umfassend ein Fragment von mindestens 200 aufeinanderfolgenden Nukleotiden eines Polynukleotids mit einer Sequenz gemäß SEQ ID NO: 1 oder 2
stammt.

7. Verfahren nach Anspruch 6, wobei die dsRNA eine Vielzahl von RNA-Molekülen umfasst, die jeweils eine doppelsträngige Region mit einer Länge von 19 bis 24 Nukleotiden umfassen, wobei die RNA-Moleküle von einem Polynukleotid, ausgewählt aus der Gruppe bestehend aus
a) einem Polynukleotid, umfassend eine Sequenz gemäß SEQ ID NO: 1 oder 2;
b) einem Polynukleotid, das für ein Polypeptid mit einer Sequenz gemäß SEQ ID NO: 3 codiert;
c) einem Polynukleotid mit 90% Sequenzidentität zu einem Polynukleotid mit einer Sequenz gemäß SEQ ID NO: 1 oder 2; und
d) einem Polynukleotid, das für ein Polypeptid mit 90% Sequenzidentität zu einem Polypeptid mit einer Sequenz gemäß SEQ ID NO: 3 codiert
abstammen.

8. Pflanze nach einem der Ansprüche 4 oder 5, oder Verfahren nach einem der Ansprüche 6 oder 7, wobei die Pflanze aus der Gruppe bestehend aus Sojabohne, Kartoffel, Tomate, Erdnüssen, Baumwolle, Cassava, Kaffee, Kokosnuss, Ananas, Zitrus-Bäumen, Banane, Mais, Raps, Rübe, Sonnenblume, Sorghumhirse, Weizen, Hafer, Roggen, Gerste, Reis, grüner Bohne, Limabohne, Erbse und Tabak ausgewählt ist.

9. Verfahren nach einem der Ansprüche 6 oder 7, wobei die dsRNA in Pflanzenwurzeln oder -syncytien exprimiert wird.

10. Verfahren zum Bekämpfen der Infektion einer Pflanze durch einen parasitären Nematoden, umfassend die Schritte des Transformierens der Pflanze mit einer Nukleinsäure, die für ein dsRNA-Molekül in operativer Verknüpfung mit einem Promoter mit Bevorzugung für die Wurzeln, einem nematodeninduzierbaren Promoter oder einem Promoter mit Bevorzugung für die Fraßstellen der Nematoden codiert, wobei die dsRNA einen Strang umfasst, der zu einem Abschnitt einer Zielnukleinsäure, die für die Ausbildung, die Entwicklung oder den Unterhalt der Fraßstelle, insbesondere die Ausbildung, die Entwicklung oder den Unterhalt eines Syncytiums oder einer Riesenzelle zwingend erforderlich ist, völlig identisch ist, wodurch die Infektion der Pflanze durch den Nematoden durch Entfernen oder funktionelles Ausschalten der Fraßstelle, des Syncytiums oder der Riesenzelle bekämpft wird,
wobei es sich bei der Zielnukleinsäure um ein CDPK-like-Gen handelt, wobei der Abschnitt des CDPK-like-Gens von einem Polynukleotid, ausgewählt aus der Gruppe bestehend aus:
a) einem Polynukleotid umfassend eine Sequenz gemäß SEQ ID NO: 1 oder 2;
b) einem Polynukleotid, das für ein Polypeptid mit einer Sequenz gemäß SEQ ID NO: 3 codiert;
c) einem Polynukleotid mit 70% Sequenzidentität zu einem Polynukleotid mit einer Sequenz gemäß SEQ ID NO: 1 oder 2;
d) einem Polynukleotid, das für ein Polypeptid mit 70% Sequenzidentität zu einem Polypeptid mit einer Sequenz gemäß SEQ ID NO: 3 codiert,
stammt.

## Revendications

1. Méthode pour conférer la résistance aux nématodes à une plante, ladite méthode comprenant les étapes consistant à :
a) préparer un acide nucléique codant pour une molécule d'ARNdb ayant une région qui est absolument identique à une portion d'un gène de type CDPK, l'acide nucléique étant capable de former un transcrit double brin d'une portion d'un gène de type CDPK une fois exprimé chez la plante ;
b) transformer une plante réceptrice avec ledit acide nucléique ;
c) produire une ou plusieurs progénitures transgéniques de ladite plante réceptrice ; et
d) sélectionner la progéniture pour la résistance aux nématodes ;
la portion du gène de type CDPK étant issue d'un polynucléotide choisi dans le groupe constitué de :
a) un polynucléotide comprenant une séquence telle qu'énoncée dans SEQ ID NO : 1 ou 2 ;
b) un polynucléotide codant pour un polypeptide ayant une séquence telle qu'énoncée dans SEQ ID NO : 3 ;
c) un polynucléotide ayant une identité de séquence de 70% avec un polynucléotide ayant une séquence telle qu'énoncée dans SEQ ID NO : 1 ou 2 ;
d) un polynucléotide codant pour un polypeptide ayant une identité de séquence de 70% avec un polypeptide ayant une séquence telle qu'énoncée dans SEQ ID NO : 3 ;
e) un polynucléotide comprenant un fragment d'au moins 200 nucléotides consécutifs d'un polynucléotide ayant une séquence telle qu'énoncée dans SEQ ID NO : 1 ou 2.

2. Utilisation d'une molécule d'ARNdb comprenant i) un premier brin comprenant une séquence absolument identique à une portion d'un gène de type CDPK, et ii) un deuxième brin comprenant une séquence complémentaire, sur tous leurs nucléotides, au premier brin, la portion du gène de type CDPK étant issue d'un polynucléotide choisi dans le groupe constitué de :
a) un polynucléotide comprenant une séquence telle qu'énoncée dans SEQ ID NO : 1 ou 2 ;
b) un polynucléotide codant pour un polypeptide ayant une séquence telle qu'énoncée dans SEQ ID NO : 3 ;
c) un polynucléotide ayant une identité de séquence de 70% avec un polynucléotide ayant une séquence telle qu'énoncée dans SEQ ID NO : 1 ou 2 ;
d) un polynucléotide codant pour un polypeptide ayant une identité de séquence de 70% avec un polypeptide ayant une séquence telle qu'énoncée dans SEQ ID NO : 3 ;
e) un polynucléotide comprenant un fragment d'au moins 200 nucléotides consécutifs d'un polynucléotide ayant une séquence telle qu'énoncée dans SEQ ID NO : 1 ou 2 ;
pour conférer la résistance aux nématodes parasites des plantes.

3. Utilisation d'un pool de molécules d'ARNdb comprenant une multiplicité de molécules d'ARN comprenant chacune une région double brin ayant une longueur de 19 à 24 nucléotides, lesdites molécules d'ARNdb étant issues d'un polynucléotide choisi dans le groupe constitué de :
a) un polynucléotide comprenant une séquence telle qu'énoncée dans SEQ ID NO : 1 ou 2 ;
b) un polynucléotide codant pour un polypeptide ayant une séquence telle qu'énoncée dans SEQ ID NO : 3 ;
c) un polynucléotide ayant une identité de séquence de 90% avec un polynucléotide ayant une séquence telle qu'énoncée dans SEQ ID NO : 1 ou 2 ;
d) un polynucléotide codant pour un polypeptide ayant une identité de séquence de 90% avec un polypeptide ayant une séquence telle qu'énoncée dans SEQ ID NO : 3 ;
pour conférer la résistance aux nématodes parasites des plantes.

4. Plante transgénique résistante aux nématodes parasites capable d'exprimer un ARNdb qui est absolument identique à une portion d'un gène de type CDPK, la portion du gène de type CDPK étant issue d'un polynucléotide choisi dans le groupe constitué de :
a) un polynucléotide comprenant une séquence telle qu'énoncée dans SEQ ID NO : 1 ou 2 ;
b) un polynucléotide ayant une identité de séquence de 95% avec un polynucléotide ayant une séquence telle qu'énoncée dans SEQ ID NO : 1 ou 2 ;
c) un polynucléotide comprenant un fragment d'au moins 200 nucléotides consécutifs d'un polynucléotide ayant une séquence telle qu'énoncée dans SEQ ID NO : 1 ou 2.

5. Plante selon la revendication 4, **caractérisée en ce que** l'ARNdb comprend une multiplicité de molécules d'ARN comprenant chacune une région double brin ayant une longueur de 19 à 24 nucléotides, lesdites molécules d'ARN étant issues d'une portion d'un polynucléotide choisi dans le groupe constitué de :
a) un polynucléotide comprenant une séquence telle qu'énoncée dans SEQ ID NO : 1 ou 2 ;
b) un polynucléotide ayant une identité de séquence de 95% avec un polynucléotide ayant une séquence telle qu'énoncée dans SEQ ID NO : 1 ou 2.

6. Méthode pour fabriquer une plante transgénique résistante aux nématodes capable d'exprimer un ARNdb qui inhibe l'expression d'un gène de type CDPK chez la plante, ladite méthode comprenant les étapes consistant à i) préparer un acide nucléique ayant une région qui est absolument identique à une portion du gène de type CDPK, l'acide nucléique étant capable de former un transcrit double brin une fois exprimé chez la plante ; ii) transformer une plante réceptrice avec ledit acide nucléique ; iii) produire une ou plusieurs progénitures transgéniques de ladite plante réceptrice ; et iv) sélectionner la progéniture pour l'expression dudit transcrit, la portion du gène de type CDPK étant issue d'un polynucléotide choisi dans le groupe constitué de :
a) un polynucléotide comprenant une séquence telle qu'énoncée dans SEQ ID NO : 1 ou 2 ;
b) un polynucléotide codant pour un polypeptide ayant une séquence telle qu'énoncée dans SEQ ID NO : 3 ;
c) un polynucléotide ayant une identité de séquence de 70% avec un polynucléotide ayant une séquence telle qu'énoncée dans SEQ ID NO : 1 ou 2 ;
d) un polynucléotide codant pour un polypeptide ayant une identité de séquence de 70% avec un polypeptide ayant une séquence telle qu'énoncée dans SEQ ID NO : 3 ;
e) un polynucléotide comprenant un fragment d'au moins 200 nucléotides consécutifs d'un polynucléotide ayant une séquence telle qu'énoncée dans SEQ ID NO : 1 ou 2.

7. Méthode selon la revendication 6, **caractérisée en ce que** l'ARNdb comprend une multiplicité de molécules d'ARN comprenant chacune une région double brin ayant une longueur de 19 à 24 nucléotides, lesdites molécules d'ARN étant issues d'un polynucléotide choisi dans le groupe constitué de :
a) un polynucléotide comprenant une séquence telle qu'énoncée dans SEQ ID NO : 1 ou 2 ;
b) un polynucléotide codant pour un polypeptide ayant une séquence telle qu'énoncée dans SEQ ID NO : 3 ;
c) un polynucléotide ayant une identité de séquence de 90% avec un polynucléotide ayant une séquence telle qu'énoncée dans SEQ ID NO : 1 ou 2 ;
d) un polynucléotide codant pour un polypeptide ayant une identité de séquence de 90% avec un polypeptide ayant une séquence telle qu'énoncée dans SEQ ID NO : 3.

8. Plante selon l'une ou l'autre des revendications 4 et 5, ou méthode selon l'une ou l'autre des revendications 6 et 7, **caractérisée en ce que** la plante est choisie dans le groupe constitué du soja, de la pomme de terre, de la tomate, de l'arachide, du coton, du manioc, du café, de la noix de coco, de l'ananas, des agrumes, de la banane, du maïs, du colza, de la betterave, du tournesol, du sorgho, du blé, de l'avoine, du seigle, de l'orge, du riz, de l'haricot vert, de l'haricot de Lima, du pois et du tabac.

9. Méthode selon l'une ou l'autre des revendications 6 et 7, **caractérisée en ce que** l'ARNdb est exprimé dans les syncytiums ou les racines des plantes.

10. Méthode pour contrôler l'infection d'une plante par un nématode parasite, comprenant les étapes consistant à transformer la plante avec un acide nucléique codant pour une molécule d'ARNdb liée de façon opérationnelle à un promoteur de préférence racinaire, inductible par les nématodes ou de préférence du site d'alimentation des nématodes, l'ARNdb comprenant ainsi un brin qui est absolument identique à une portion d'un acide nucléique cible essentiel pour la formation, le développement ou le support du site d'alimentation, en particulier la formation, le développement ou le support d'un syncytium ou d'une cellule géante, contrôlant ainsi l'infection de la plante par les nématodes en éliminant ou en rendant fonctionnellement inactif le site d'alimentation, le syncytium ou la cellule géante, l'acide nucléique cible étant un gène de type CDPK, la portion du gène de type CDPK étant issue d'un polynucléotide choisi dans le groupe constitué de :
a) un polynucléotide comprenant une séquence telle qu'énoncée dans SEQ ID NO : 1 ou 2 ;
b) un polynucléotide codant pour un polypeptide ayant une séquence telle qu'énoncée dans SEQ ID NO : 3 ;
c) un polynucléotide ayant une identité de séquence de 70% avec un polynucléotide ayant une séquence telle qu'énoncée dans SEQ ID NO : 1 ou 2 ;
d) un polynucléotide codant pour un polypeptide ayant une identité de séquence de 70% avec un polypeptide ayant une séquence telle qu'énoncée dans SEQ ID NO : 3.
